# EUROPEAN PATENT SPECIFICATION

(11) **EP 2 200 636 B1**
(45) Date of publication and mention of the grant of the patent: **07.08.2013**
(21) Application number: 08806718.6
(22) Date of filing: 03.10.2008
(51) Int. Cl.: A61K 39/00, C12N 15/11

(54) **PROTEIN**
PROTEIN
PROTÉINE

(30) Priority: 03.10.2007 GB 0719231; 03.10.2007 US 997444 P
(43) Date of publication of application: 30.06.2010
(73) Proprietor: Oxford Biotherapeutics Ltd., Abingdon Oxfordshire OX14 4RY (GB)
(72) Inventor: ROHLFF, Christian, Oxford, Oxfordshire OX14 4RY (GB)
(74) Representative: Webber, Philip Michael
(86) International application number: PCT/GB2008/050902
(87) International publication number: WO 2009/044208

(56) References cited:
- WO-A-01/30854
- WO-A-02/055659
- US-A- 5 320 942
- US-A1- 2003 224 993
- DAEMI NOUCHA ET AL: "Anti-beta4 integrin antibodies enhance migratory and invasive abilities of human colon adenocarcinoma cells and their MMP-2 expression" INTERNATIONAL JOURNAL OF CANCER, vol. 85, no. 6, 15 March 2000 (2000-03-15), pages 850-856, XP002507477 ISSN: 0020-7136
- GIANCOTTI ET AL: "Targeting integrin beta 4 for cancer and anti-angiogenic therapy" TRENDS IN PHARMACOLOGICAL SCIENCES, ELSEVIER, HAYWARTH, GB, vol. 28, no. 10, 2 October 2007 (2007-10-02), pages 506-511, XP022282053 ISSN: 0165-6147
- HOGERVORST F ET AL: "CLONING AND SEQUENCE ANALYSIS OF BETA-4 COMPLEMENTARY DNA AN INTEGRIN SUBUNIT THAT CONTAINS A UNIQUE 118-KD CYTOPLASMIC DOMAIN" EMBO (EUROPEAN MOLECULAR BIOLOGY ORGANIZATION) JOURNAL, vol. 9, no. 3, 1990, pages 765-770, XP002507478 ISSN: 0261-4189

## Description

### INTRODUCTION

The present invention relates to the identification of a membrane protein associated with breast cancer, colorectal cancer, gastric cancer, hepatocellular carcinoma, lung cancer and pancreatic cancer, which has utility as a marker for breast cancer, colorectal cancer, gastric cancer, hepatocellular carcinoma, lung cancer and pancreatic cancer and breast cancer, colorectal cancer, gastric cancer, hepatocellular carcinoma, lung cancer and pancreatic cancer metastases and which also forms a biological target against which therapeutic antibodies can be made.

### BACKGROUND OF THE INVENTION

### Breast Cancer diagnosis:

Finding a breast cancer as early as possible improves the likelihood that treatment will be successful. Screening methods such as mammograms, clinical breast examinations and breast self-examinations are useful in detecting breast cancer. Current diagnostic methods include breast ultrasound, ductogram, full-field digital mammography (FFDM), scintimammography and MRI. A biopsy (fine needle aspiration biopsy, core biopsy or surgical biopsy) is then performed to confirm the presence of breast cancer. Imaging tests such as a chest x-ray, bone scan, CT, MRI and PET are used to detect if the breast cancer has spread.

### Breast Cancer staging:

Breast cancer is staged using the American Joint Committee on Cancer (AJCC) TNM system - Stage 0 - Stage IV. Ductal carcinoma in situ (DCIS), a non-invasive cancer which accounts for 20% of new breast cancer cases is Stage 0. Nearly all women diagnosed at this early stage of breast cancer can be cured. Infiltrating (invasive) ductal carcinoma (IDC), which accounts for 80% of invasive breast cancer and infiltrating (invasive) lobular carcinoma (ILC), which accounts for 5% of invasive breast cancers are more severe Stage I-IV cancers and can metastasize.

Neoadjuvant chemotherapy can be given before surgery to shrink large cancers. Adjuvant chemotherapy after surgery reduces the risk of breast cancer recurrence. Chemotherapy can also be used as the main treatment for women whose cancer has spread outside the breast and underarm area. Chemotherapeutic agents used include anthracyclines (e.g. methotrexate, fluorouracil, doxorubicin, epirubicin), taxanes (e.g. paclitaxel, docetaxel, vinorelbine) and alkylating agents (e.g. cyclophosphamide).

Radiation therapy (usually external beam radiation but sometimes brachytherapy) is given once chemotherapy is complete.

Hormone therapy with selective estrogen receptor modulators (e.g. tamoxifen) can be given to women with estrogen receptor positive breast cancers. Taking tamoxifen after surgery for 5 years can reduce recurrence by about 50% in women with early breast cancer. Aromatase inhibitors such as exemestane, letrozole or anastrozole can also be used.

Women with HER2 positive cancers (about 1/3 of breast cancers) can be given biological response modifiers such as trastuzumab (Herceptin). Clinical trials have shown that adding trastuzumab to chemotherapy lowers the recurrence rate and death rate over chemotherapy alone after surgery in women with HER2 positive early breast cancers.

### Colorectal Cancer diagnosis:

Today, the faecal occult blood test and colonoscopy, a highly invasive procedure, are the most frequently used screening and diagnostic methods for colorectal cancer.
Other diagnostic tools include Flexible Sigmoidoscopy (allowing the observation of only about half of the colon) and Double Contrast Barium Enema (DCBE, to obtain X-ray images).

### Colorectal Cancer staging:

CRC has four distinct stages: patients with stage I disease have a five-year survival rate of >90%, while those with metastatic stage IV disease have a <5% survival rate according to the US National Institutes of Health (NIH).

### Gastric Cancer diagnosis:

Early stage gastric cancer rarely causes symptoms so only about 10-20% of gastric cancers in the USA are found in the early stages, before they have spread to other areas of the body. Studies in the USA have not found mass screening for gastric cancer to be useful because the disease is not that common. Endoscopy followed by a biopsy is the main procedure used to diagnose gastric cancer. Other diagnostic methods include barium upper gastrointestinal radiographs, endoscopic ultrasound, CT scan, PET scan, MRI scan, chest x-ray, laparoscopy, complete blood count (CBC) test and faecal occult blood test.

### Gastric Cancer staging:

Gastric cancer is staged using the American Joint Commission on Cancer (AJCC) TNM system - Stage 0 - Stage IV. Patients with stage 0 disease have a 5-year survival rate of >90%, while there is usually no cure for patients with stage IV disease and the 5-year survival rate is only 7%. The overall 5-year relative survival rate of people with gastric cancer in the USA is about 23%. The 5-year survival rate for cancers of the proximal stomach is lower than for cancers in the distal stomach.

### Gastric Cancer treatment:

Chemotherapy may be given as the primary treatment for gastric cancer that has spread to distant organs. Chemotherapy together with external beam radiation therapy may delay cancer recurrence and extend the life span of people with less advanced gastric cancer, especially when the cancer could not be removed completely by surgery. Chemotherapeutic agents used include fluorouracil, doxorubicin, methotrexate, etoposide and cisplatin.

### Hepatocellular carcinoma diagnosis:

Since symptoms of liver cancer often do not appear until the disease is advanced, only a small number of liver cancers are found in the early stages and can be removed with surgery. Many signs and symptoms of liver cancer are relatively nonspecific - that is, they can be caused by other cancers or by non-cancerous diseases. Imaging tests such as ultrasound, computed tomography (CT), magnetic resonance imaging (MRI) and angiography are commonly used to diagnose HCC. Other diagnostic tools include laparoscopy, biopsy, alpha-fetoprotein (AFP) blood test, liver function tests (LFTs), a prothrombin time (PT) and tests for hepatitis B and C.

### Hepatocellular carcinoma staging:

HCC has four stages, stage I to stage IV according to the American Joint Committee on Cancer (AJCC) TNM system. HCC can be classified as localized resectable, localized unresectable or advanced. The overall 5-year relative survival rate from liver cancer is about 9%. One reason for this low survival rate is that most patients with liver cancer also have cirrhosis of the liver, which itself can be fatal (people with liver cancer and class C cirrhosis are generally too sick for any treatment and usually die in a few months). The 5 year survival for localized resectable HCC following surgery is between 40% and 70%. For advanced HCC there is no standard treatment and the 5 year survival rate is less than 5%. Survival continues to drop after diagnosis and treatment so that by 10 years it is half of what it was at 5 years.

### Hepatocellular carcinoma treatment:

Treatments include radiofrequency ablation (RFA), ethanol ablation, cryosurgery, hepatic artery embolization, chemoembolization or three-dimensional conformal radiation therapy (3DCRT). Chemotherapy can also be used but shrinks fewer than 1 in 5 tumours. This may be improved by hepatic artery infusion (HAI). Chemotherapeutic agents used include Adriamycin, VP-16, Cisplatinum, Mitomycin, 5-FU and Leucovorin.

### Lung Cancer diagnosis:

Lung cancer is a life-threatening disease because it often metastasizes even before it can be detected on a chest x-ray. Usually symptoms of lung cancer do not appear until the disease is in an advanced stage. So far, there is no screening test that has been shown to improve a person's chance for a cure. Imaging tests such as a chest x-ray, CT scan, MRI scan or PET scan may be used to detect lung cancer. Tests to confirm the diagnosis are then performed and include sputum cytology, needle biopsy, bronchoscopy, endobronchial ultrasound and complete blood count (CBC).

### Lung Cancer staging:

Nearly 60% of people diagnosed with lung cancer die within one year of diagnosis; 75% die within 2 years. The 5-year survival rate for people diagnosed with NSCLC is about 15%; for SCLC the 5-year survival rate is about 6%.

NSCLC is staged using the American Joint Committee on Cancer (AJCC) TNM system - Stage 0 - Stage IV. The 5-year survival rates by stage are as follows: stage I: 47%; stage II; 26%; stage III: 8% and stage IV: 2%.

SCLC has a 2-stage system - limited stage and extensive stage. About two thirds of SCLC patients have extensive disease at diagnosis. If SCLC is found very early and is localised to the lung alone, the 5-year survival rate is around 21 %, but only 6% of patients fall into this category. Where the cancer has spread, the 5-year survival is around 11 %. For patients with extensive disease, the 5-year survival is just 2%.

### Lung Cancer treatment:

Chemotherapy may be given as the primary treatment or as an adjuvant to surgery. Targeted therapy using epidermal growth factor receptor (EGFR) antagonists such as gefitinib or erlotinib can also be given after other treatments have failed. Antiangiogenesis drugs, such as bevacizumab, have been found to prolong survival of patients with advanced lung cancer. Photodynamic therapy is also being researched as a treatment for lung cancer.

The main treatment for SCLC is chemotherapy, either alone or in combination with external beam radiation therapy and very rarely, surgery.

Chemotherapeutic agents used for NSCLC and SCLC include cisplatin, carboplatin, mitomycin C, ifosfamide, vinblastine, gemcitabine, etoposide, vinorelbine, paclitaxel, docetaxel and irinotecan.

### Pancreatic Cancer diagnosis:

Pancreatic cancer is very difficult to detect and very few pancreatic cancers are found early. Patients usually have no symptoms until the cancer has spread to other organs. There are currently no blood tests or easily available screening tests that can accurately detect early cancers of the pancreas. An endoscopic ultrasound followed by a biopsy is the best way to diagnose pancreatic cancer. Other detection methods include CT, CT-guided needle biopsy, PET, ultrasonography and MRI. Blood levels of CA 19-9 and carcinoembryonic antigen (CEA) may be elevated but by the time blood levels are high enough to be detected, the cancer is no longer in its early stages.

### Pancreatic Cancer staging:

Pancreatic cancer has four stages, stage I to stage IV according to the American Joint Committee on Cancer (AJCC) TNM system. Pancreatic cancer is also divided into resectable, locally advanced (unresectable) and metastatic cancer. For patients with advanced cancers, the overall survival rate is <1% at 5 years with most patients dying within 1 year.

### Pancreatic Cancer treatment:

External beam radiation therapy combined with chemotherapy can be given before or after surgery and can also be given to patients whose tumours are too widespread to be removed by surgery. The main chemotherapeutic agents which are used are gemcitabine and 5-fluorouracil.

Targeted therapy using drugs such as erlotinib and cetuximab may be of benefit to patients with advanced pancreatic cancer.
US 2003/224993 discloses compositions and methods for reducing the proliferation of cancer cells through targeted interactions with integrins.
WO 02/055659 discloses antisense compounds, compositions and methods are provided for modulating the expression of Integrin beta 4 binding protein.
US 5320942 discloses a novel cell surface marker and antigenic portions thereof; antibodies reactive with said marker; polynucleotides encoding said marker and antigenic portions thereof; methods of diagnosis and treatment using said polynucleotides and antibodies.
WO 01/30854 discloses an antibody which is displayed in, and on the cell surface of, human gastrointestinal epithelial tumour cells and in a subpopulation of normal human gastrointestinal epithelial cells.
Daemi Noucha et al. (International Journal of Cancer, vol. 85, no. 6, 15 March 2000, pages 850-856) discloses anti-beta 4 integrin antibodies which enhance migratory and invasive abilities of human colon adenocarcinoma cells and their MMP-2 expression.

### Therapeutic Challenges

The major challenges in treatment of the above mentioned cancers are to improve early detection rates, to find new non-invasive markers that can be used to follow disease progression and identify relapse, and to find improved and less toxic therapies, especially for more advanced disease where 5 year survival is still poor. There is a great need to identify targets which are more specific to the cancer cells, e.g. ones which are expressed on the surface of the tumour cells so that they can be attacked by promising new approaches like immunotherapeutics and targeted toxins.

### SUMMARY OF THE INVENTION

The present invention provides a composition comprising an antibody capable of specific binding to Integrin beta 4 or a fragment thereof, and a pharmaceutically acceptable diluent or carrier, for use in treating or preventing breast cancer, colorectal cancer, gastric cancer, hepatocellular carcinoma, lung cancer or pancreatic cancer, wherein Integrin beta 4 is overexpressed in said cancers.

We have used mass spectrometry to identify peptides generated by 1D gel electrophoresis and tryptic digest of membrane proteins extracted from breast, colorectal, gastric epithelium, liver, lung and pancreatic cancer tissue samples. Peptide sequences were compared to existing protein and cDNA databases and the corresponding gene sequences identified. The protein has not been previously reported to originate from breast, colorectal, gastric epithelium, liver, lung or pancreatic cancer cell membranes and represents a protein of new diagnostic and therapeutic value.

In some embodiments, the antibody contains or is conjugated to a detectable label or contains or is conjugated to a therapeutic moiety such as a cytotoxic moiety.

In some embodiments, the antibody of the present invention is selected from the group consisting of: a whole antibody, an antibody fragment, a humanized antibody, a single chain antibody, an immunoconjugate, a defucosylated antibody, and a bispecific antibody. The antibody fragment may be selected from the group consisting of: aUniBody, a domain antibody, and a Nanobody. In some embodiments, the immunoconjugates of the invention comprise a therapeutic agent. In another aspect of the invention, the therapeutic agent is a cytotoxin or a radioactive isotope.

In some embodiments, the antibody of the present invention is selected from the group consisting of: an Affibody, a DARPin, an Anticalin, an Avimer, a Versabody, and a Duocalin.

Also disclosed are methods of treating breast cancer, colorectal cancer, gastric cancer, hepatocellular carcinoma, lung cancer or pancreatic cancer, comprising administering to a patient a therapeutically effective amount of a compound that modulates (e.g., upregulates or downregulates) or complements the expression or the biological activity (or both) of the protein of the invention in patients having breast cancer, colorectal cancer, gastric cancer, hepatocellular carcinoma, lung cancer or pancreatic cancer, in order to (a) prevent the onset or development of breast cancer, colorectal cancer, gastric cancer, hepatocellular carcinoma, lung cancer or pancreatic cancer; (b) prevent the progression of breast cancer, colorectal cancer, gastric cancer, hepatocellular carcinoma, lung cancer or pancreatic cancer; or (c) ameliorate the symptoms of breast cancer, colorectal cancer, gastric cancer, hepatocellular carcinoma, lung cancer or pancreatic cancer.

Integrin beta-4 is a laminin-5 receptor which is phosphorylated on multiple tyrosines in the beta-4 cytoplasmic domain by a Src Family Kinase (SFK) after ligand binding. The phosphorylated complex activates the Ras to ERK cascade via the signaling adaptor protein She (Dans et al., 2001 J Biol. Chem. 276:1494-1502; Gagnoux-Palacios et al., 2003 J Cell Biol.162:1189-96; Mainiero et al.,1995 EMBO J. 14:4470-4481), as well as PI-3 kinase and Rac (Shaw, 2001; Shaw et al., 1997).
*In vitro* studies support a role for Integrin beta-4 in promoting tumor invasion, and a number of invasive carcinomas display elevated levels of Integrin beta-4 (Mercurio and Rabinovitz, 2001). Introduction of Integrin beta-4 in breast and colon carcinoma cells activates PI-3K - Rac and confers a more invasive phenotype (Shaw et al. 1997 Cell 91:949-960). The activation of this pathway may also confer resistance to apoptosis.

Integrin beta-4 can also amplify signaling from certain receptor tyrosine kinases that have known involvement in tumour progression, such as c-met (Trusolino et al. 2001 Cell 107:643-54), erb-b2 and EGFR (Guo et al, 2006, Cell 126: 489-502).

Transfection of a dominant negative form of Integrin beta-4 impairs the survival of breast carcinoma cells phenotype (Weaver et al., 2002 Cancer Cell 2:205-16). Antibodies that inhibit the phosphorylation of Integrin beta-4 also blocked anchorage independent growth of MDA-MB-231 breast cancer cells, independently of the action of the EGFR-inhibitory antibody, erbitux (Gabarra et al., 2008 AACR Meeting Abstracts 2008: 5252). The same antibodies also blocked apoptosis induced by serum starvation of these and other Integrin beta-4-expressing cell lines (SW620, MCF7, SU159).

These results suggest that Integrin beta-4 promotes cell migration and invasion and confers resistance to apoptosis in carcinoma cells, functions that can be tested in *vitro* and used as an assay for inhibitors of Integrin beta-4 that could act as anti-cancer agents.

In one embodiment the cancer to be treated is breast cancer.

In another embodiment the cancer to be treated is colorectal cancer.

In another embodiment the cancer to be treated is gastric cancer.

In another embodiment the cancer to be treated is hepatocellular carcinoma.

In another embodiment the cancer to be treated is lung cancer.

In another embodiment the cancer to be treated is pancreatic cancer.

### BRIEF DESCRIPTION OF THE FIGURES

Figure 1 shows the amino acid sequences of the five splice variants of the Integrin beta 4 protein. The tryptics detected experimentally by mass spectrometry are highlighted - mass match peptides are shown in bold, tandem peptides are underlined. Recombinant protein is shown in italics.
Figure 2 shows the Protein Index for the Integrin beta 4 protein.

### DETAILED DESCRIPTION OF THE INVENTION

In one aspect, the present invention provides a composition comprising an antibody capable of specific binding to Integrin beta 4 or a fragment thereof, and a pharmaceutically acceptable diluent or carrier, for use in treating or preventing breast cancer, colorectal cancer, gastric cancer, hepatocellular carcinoma, lung cancer or pancreatic cancer, wherein Integrin beta 4 is overexpressed in said cancers.

In some embodiments, the antibody contains or is conjugated to a detectable label or contains or is conjugated to a therapeutic moiety such as a cytotoxic moiety.

The composition may comprise a therapeutically effective amount of the antibody which is capable of specific binding to Integrin beta 4 or a fragment thereof.

The mammalian subject may be a non-human mammal, but is preferably human, more preferably a human adult, i.e. a human subject at least 21 (more preferably at least 35, at least 50, at least 60, at least 70, or at least 80) years old.

### Integrin beta 4

One-dimensional electrophoresis or another appropriate method may be used to analyze breast cancer, colorectal cancer, gastric cancer, hepatocellular carcinoma, lung cancer or pancreatic cancer tissue samples from a subject, preferably a living subject, in order to measure the expression of the Integrin beta 4 protein for screening or diagnosis of breast cancer, colorectal cancer, gastric cancer, hepatocellular carcinoma, lung cancer or pancreatic cancer, to determine the prognosis of a breast cancer, colorectal cancer, gastric cancer, hepatocellular carcinoma, lung cancer or pancreatic cancer patient, to monitor the effectiveness of breast cancer, colorectal cancer, gastric cancer, hepatocellular carcinoma, lung cancer or pancreatic cancer therapy, or for drug development.

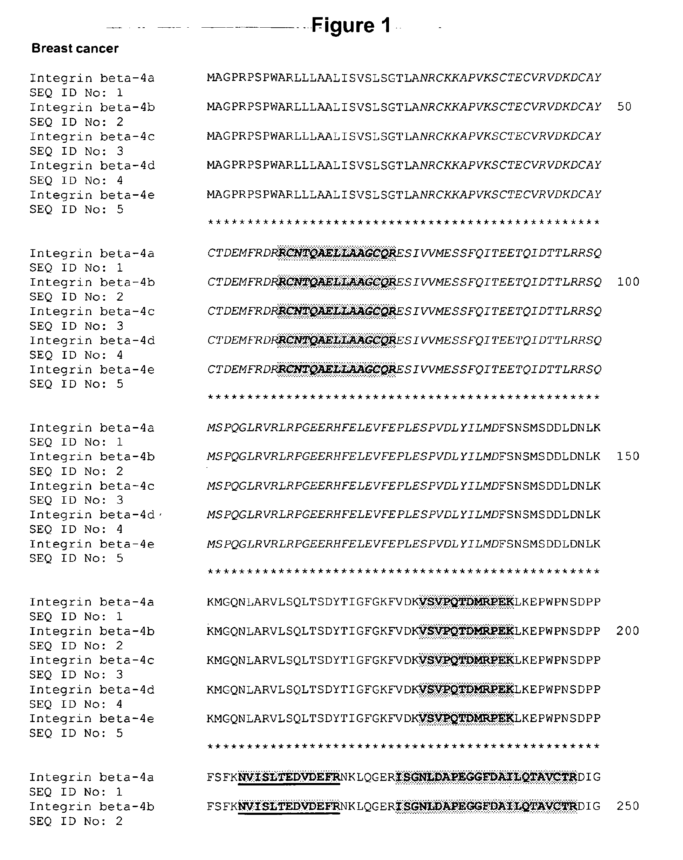

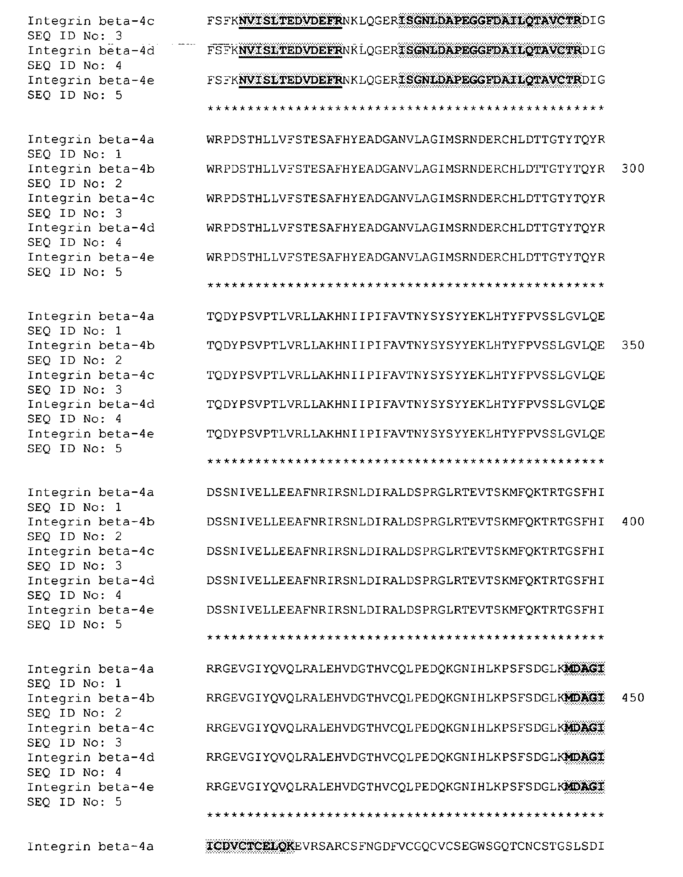

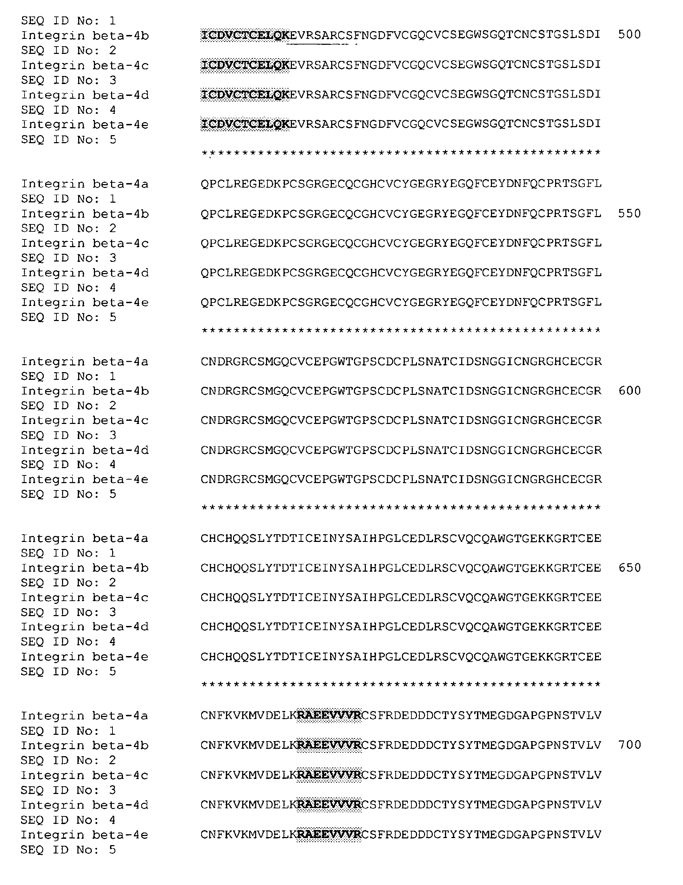

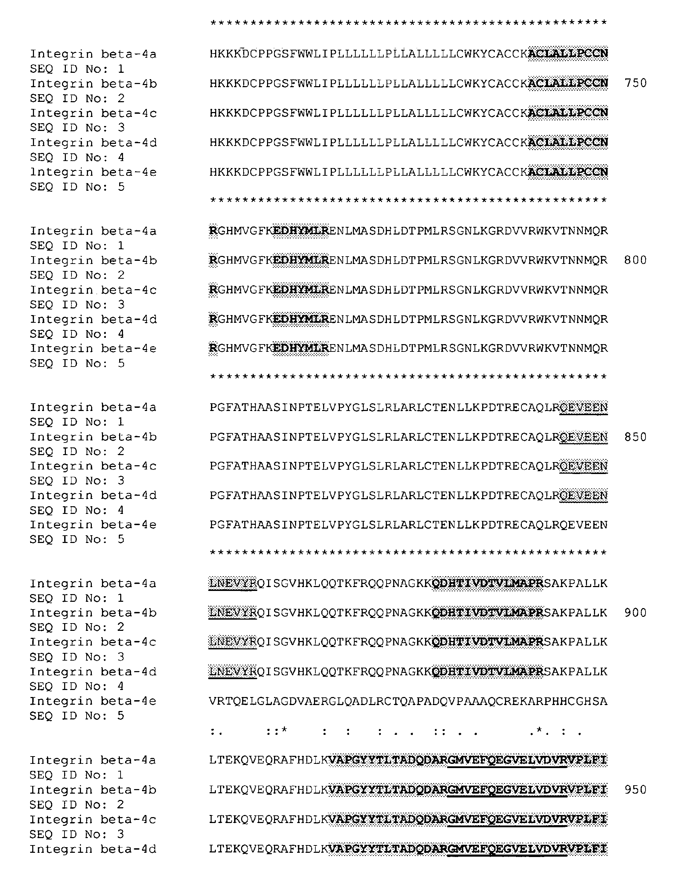

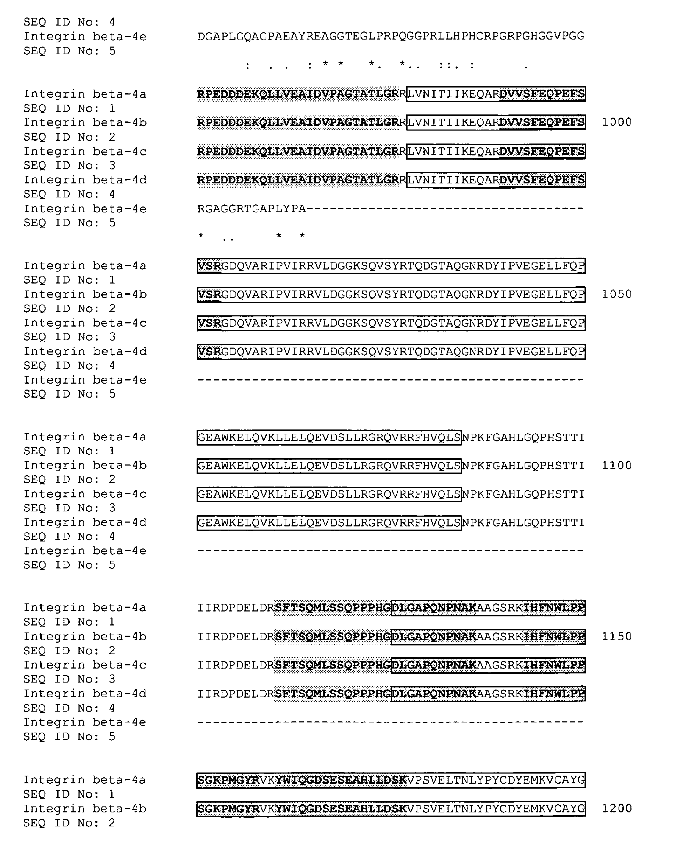

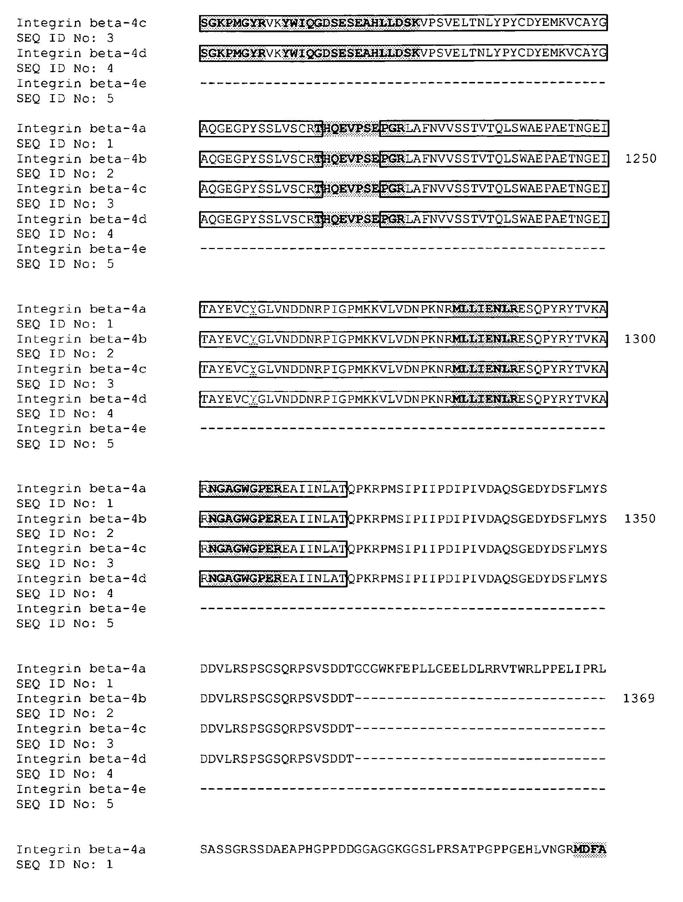

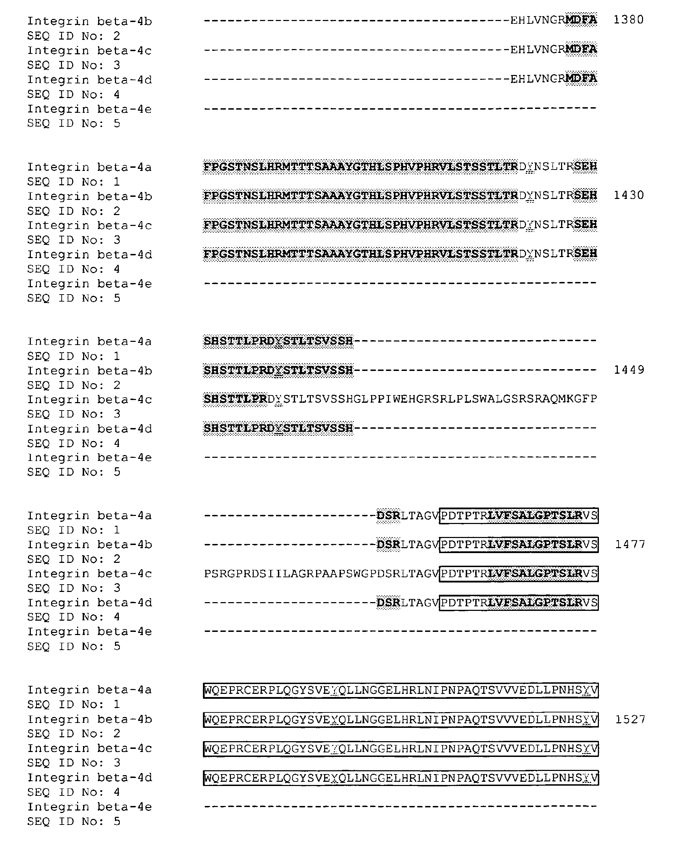

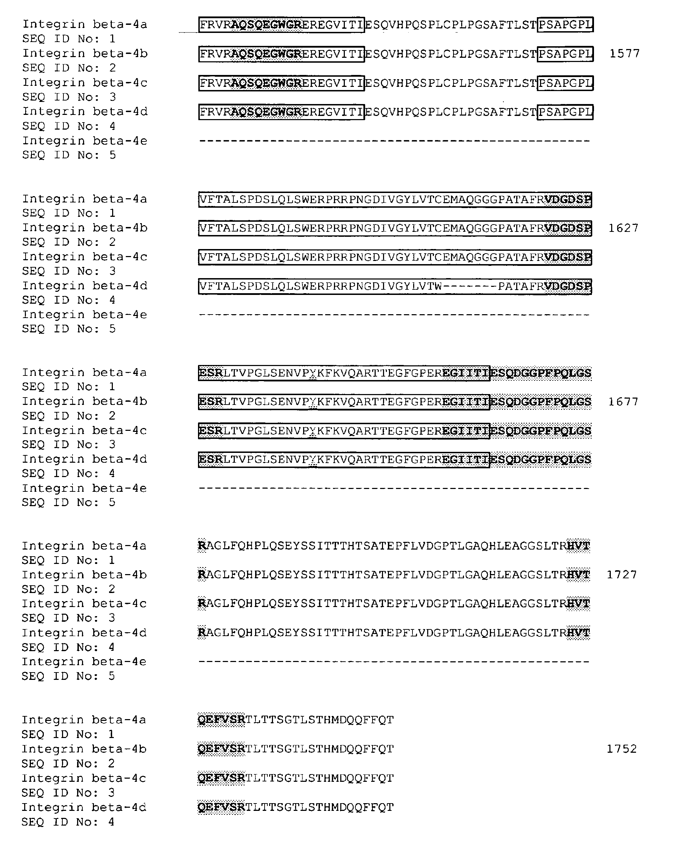

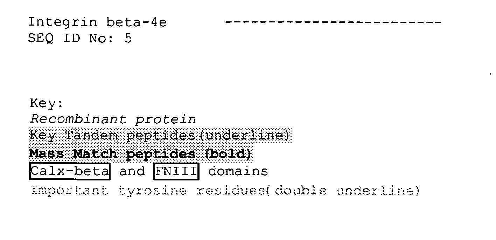

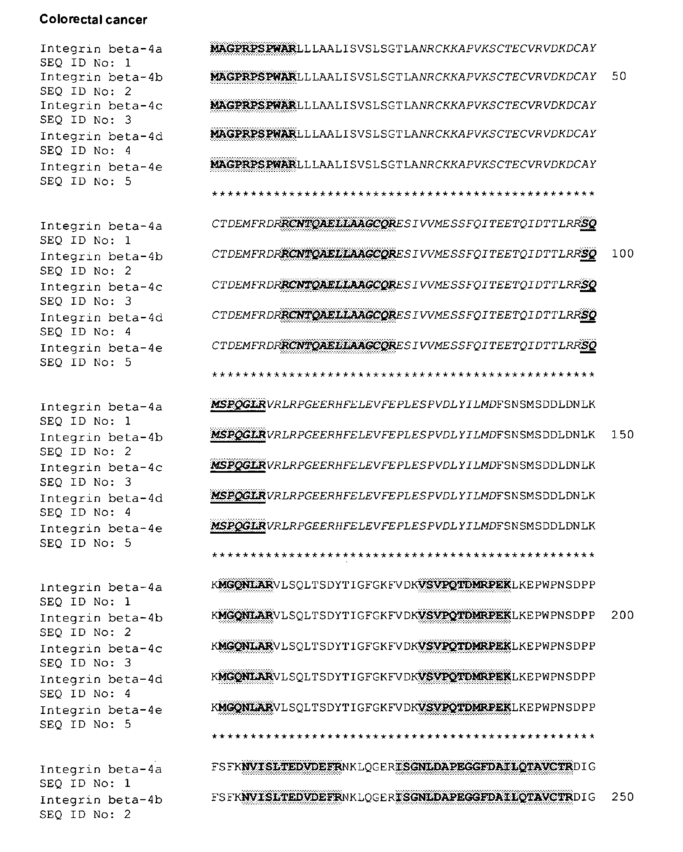

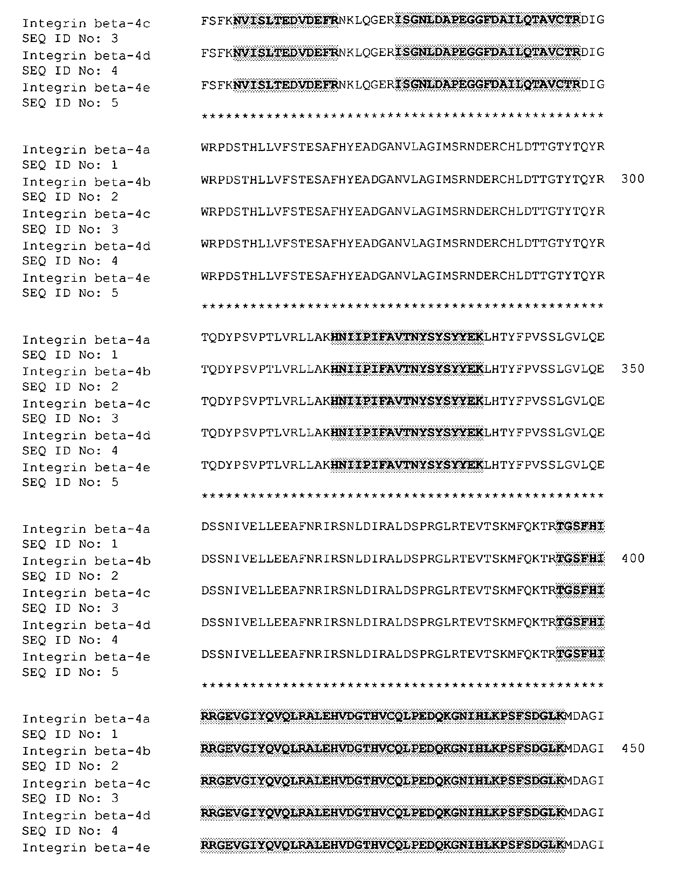

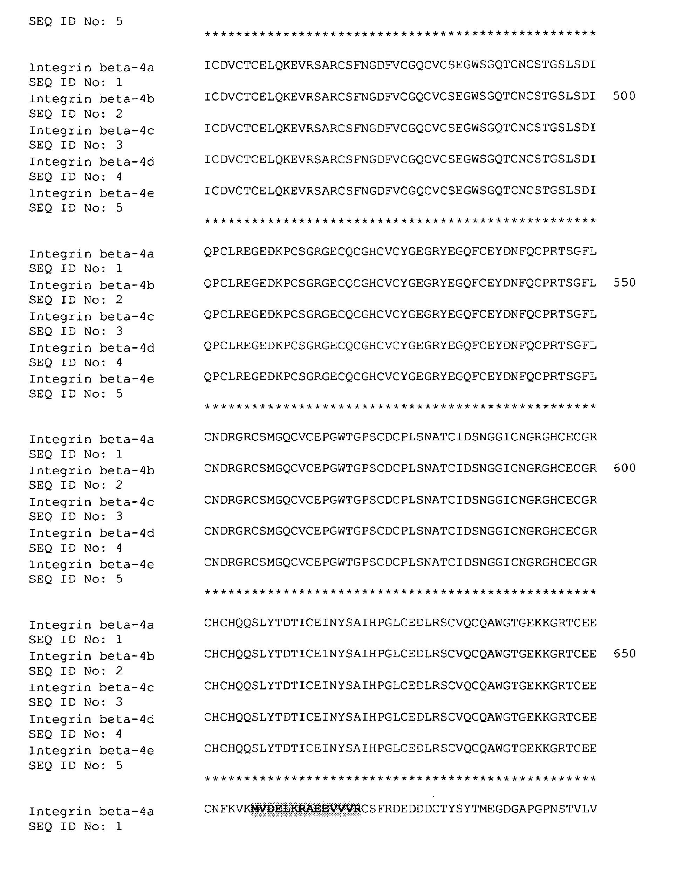

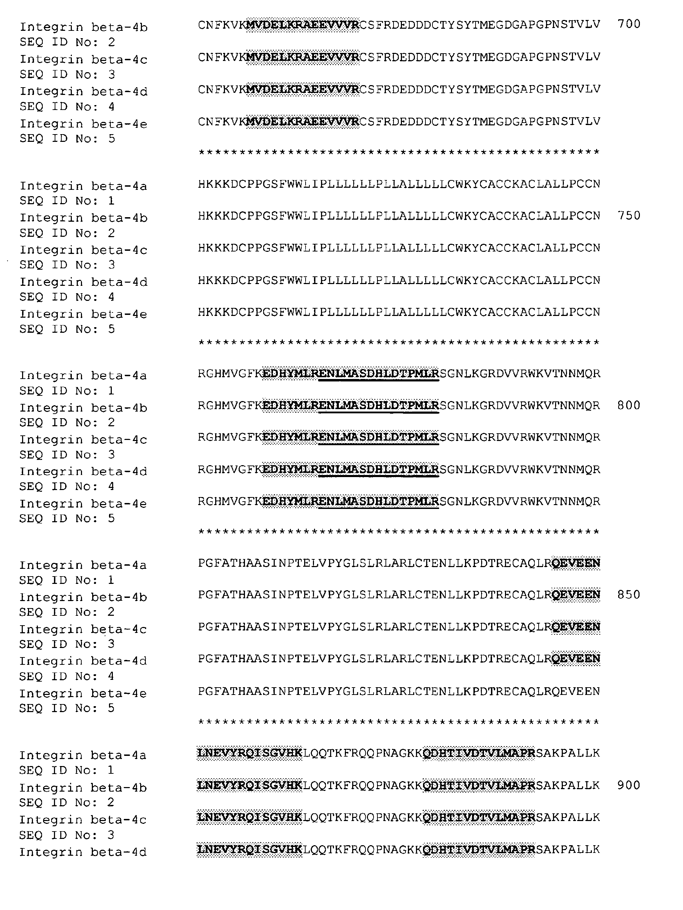

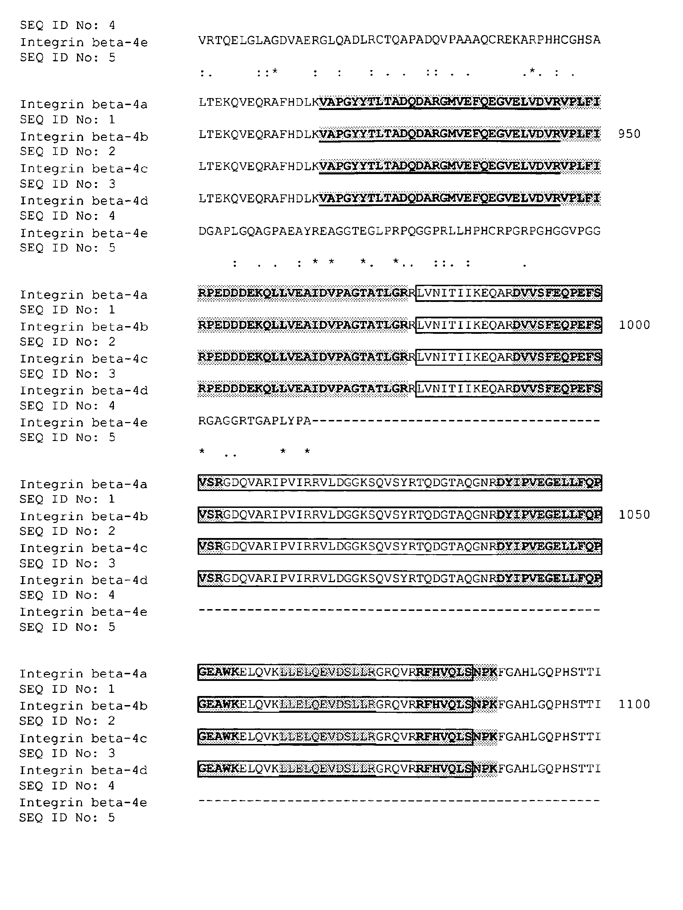

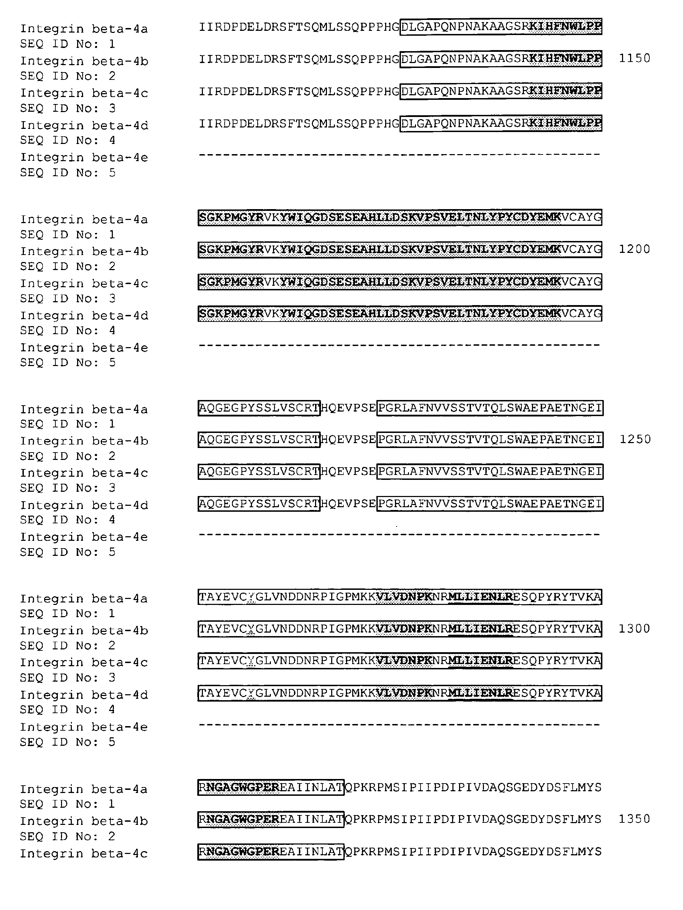

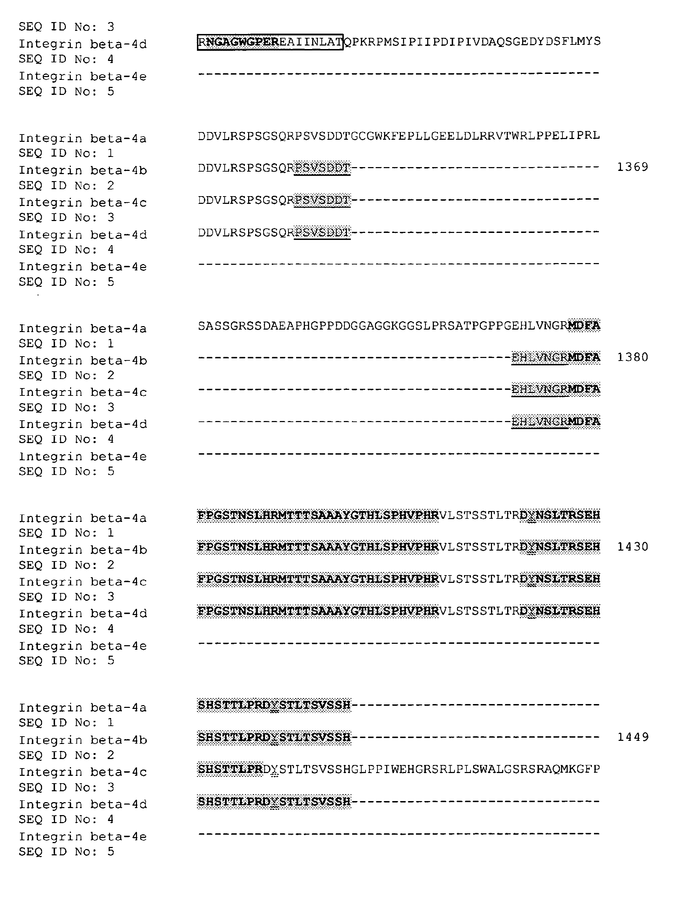

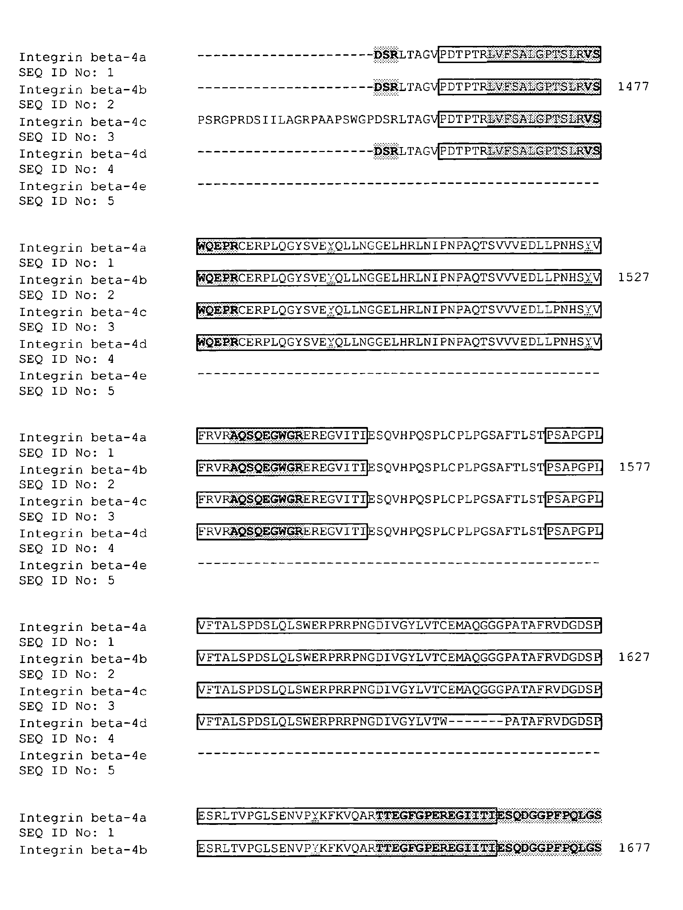

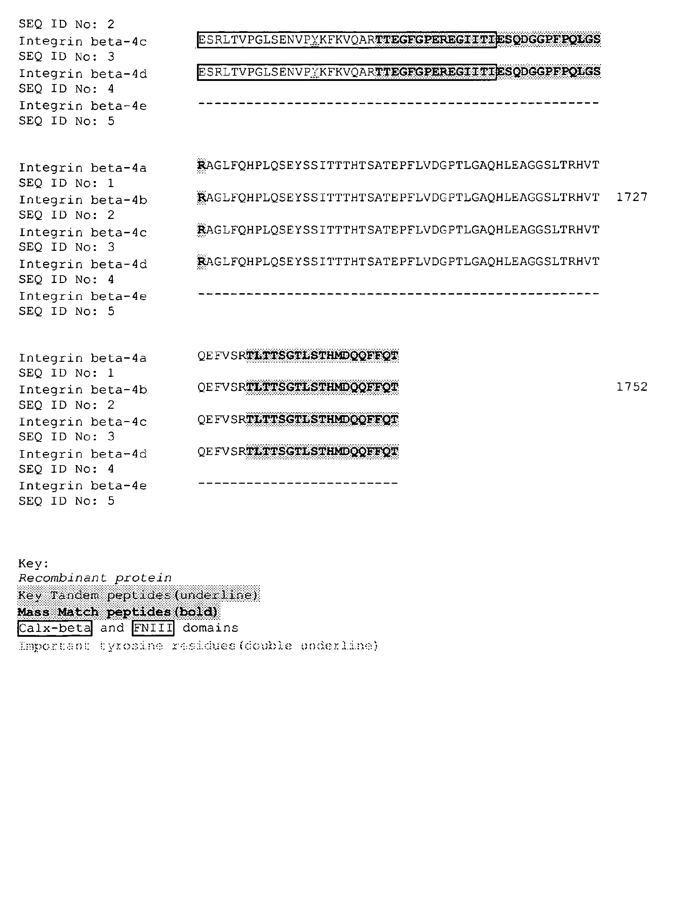

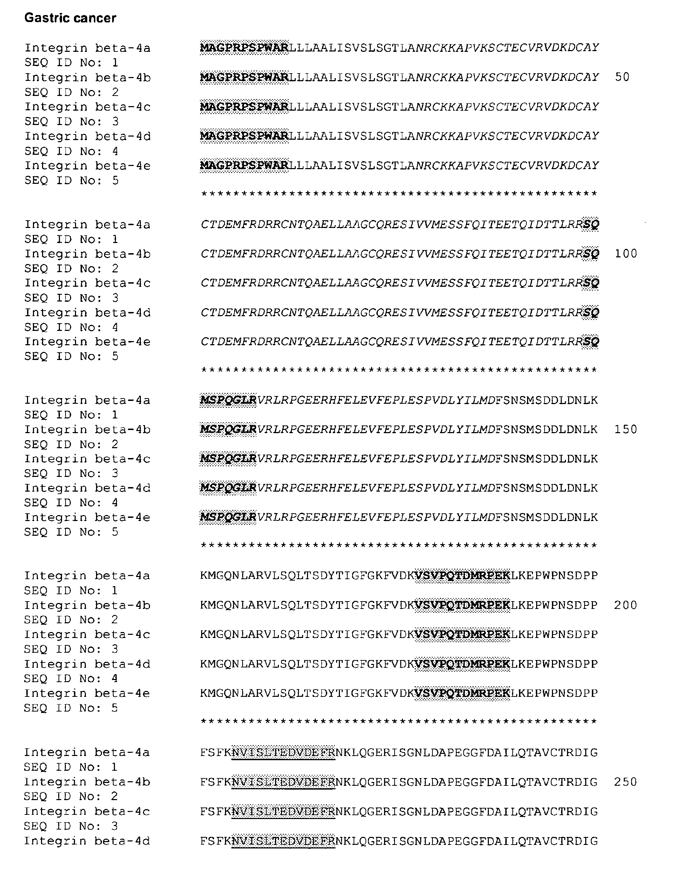

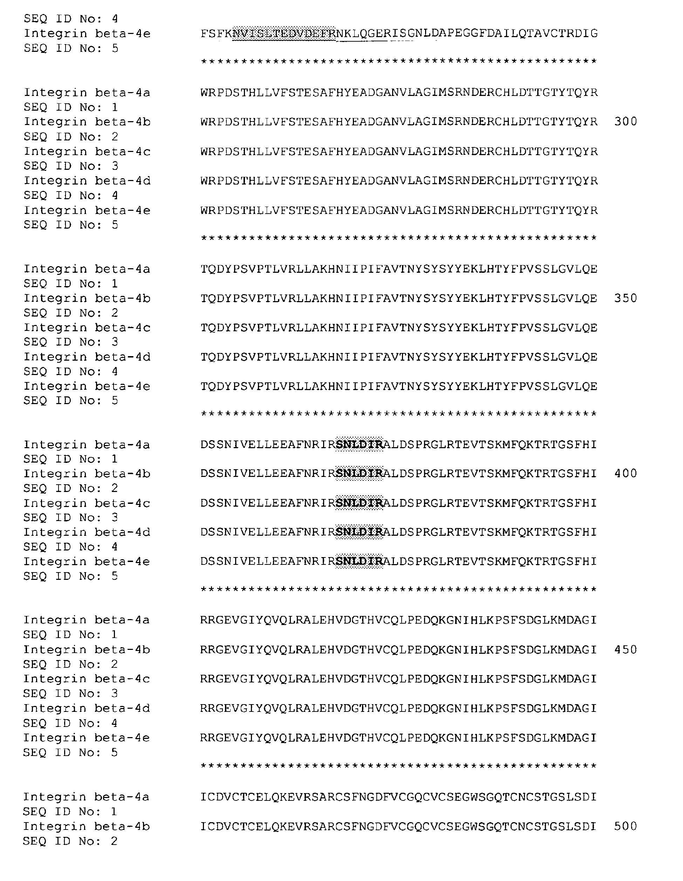

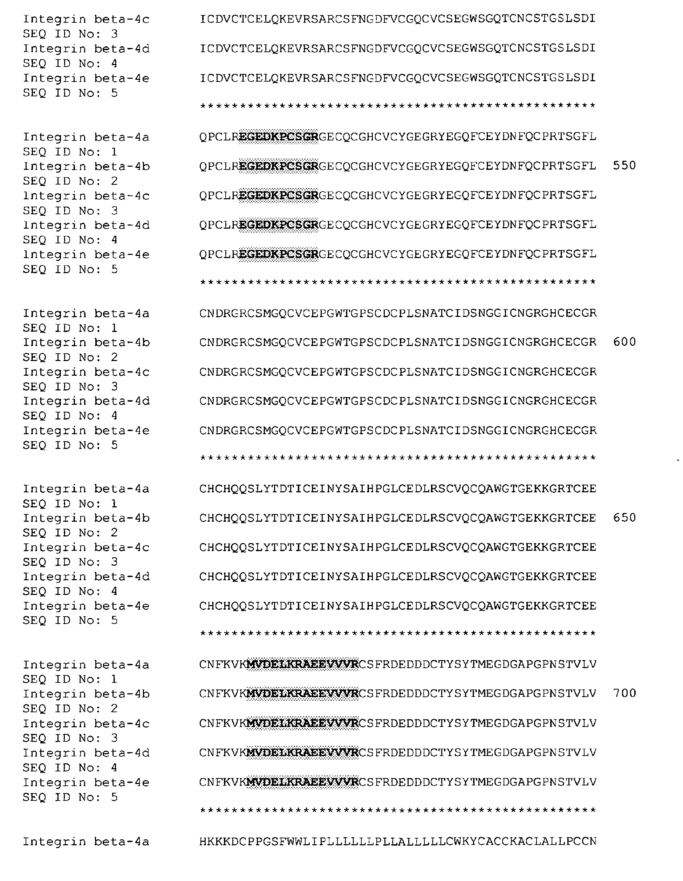

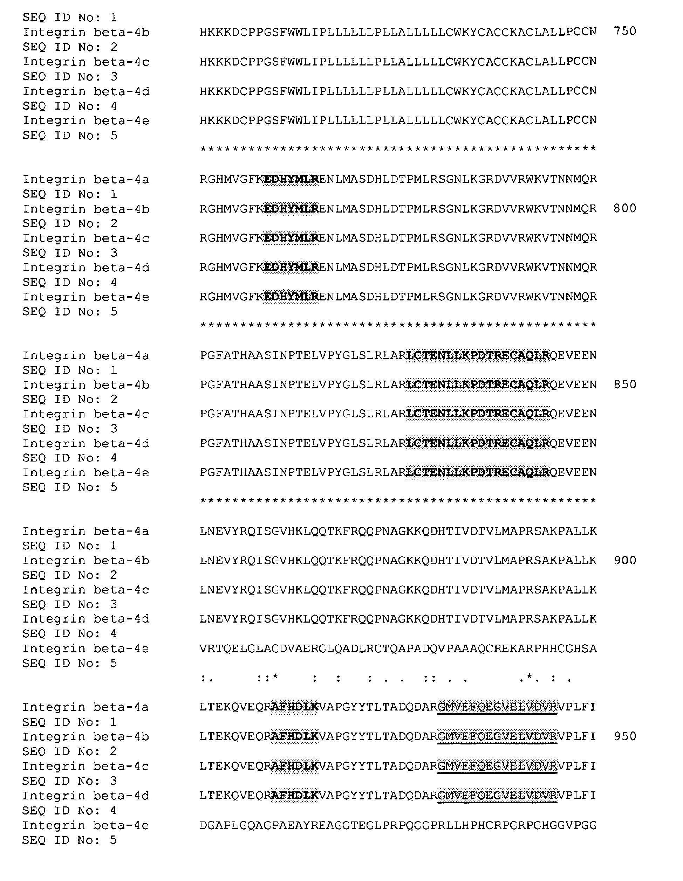

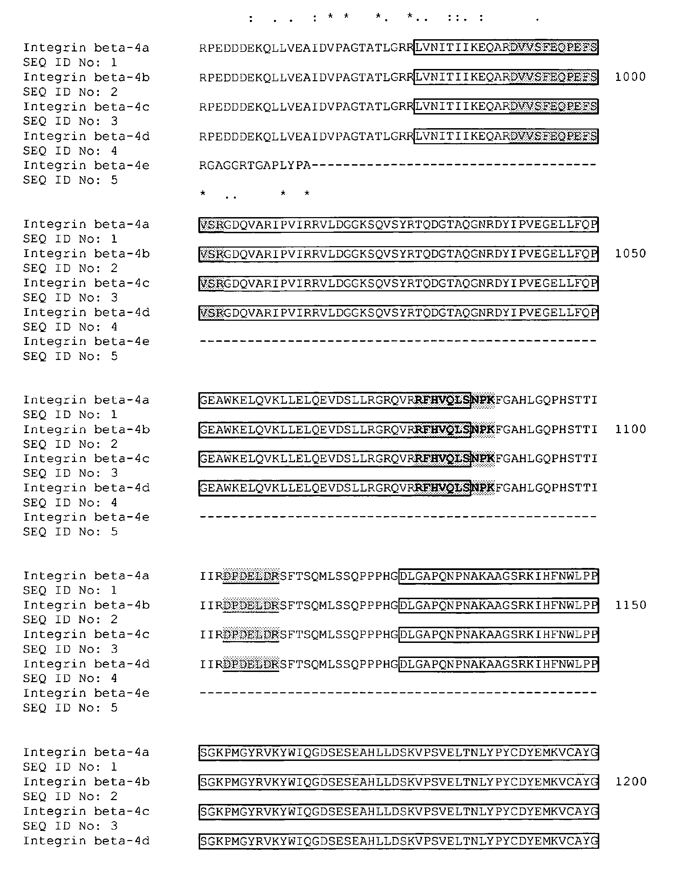

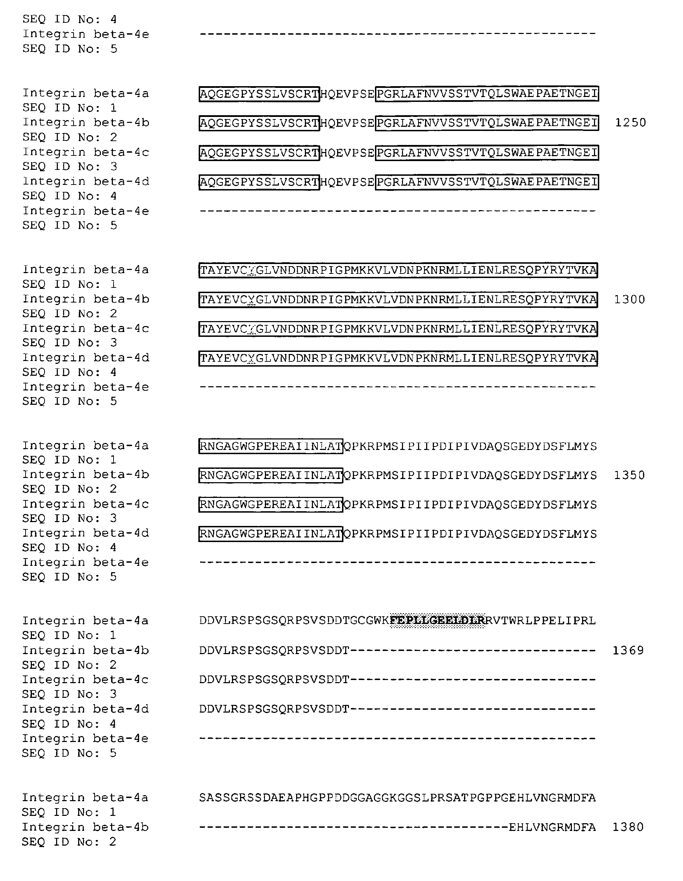

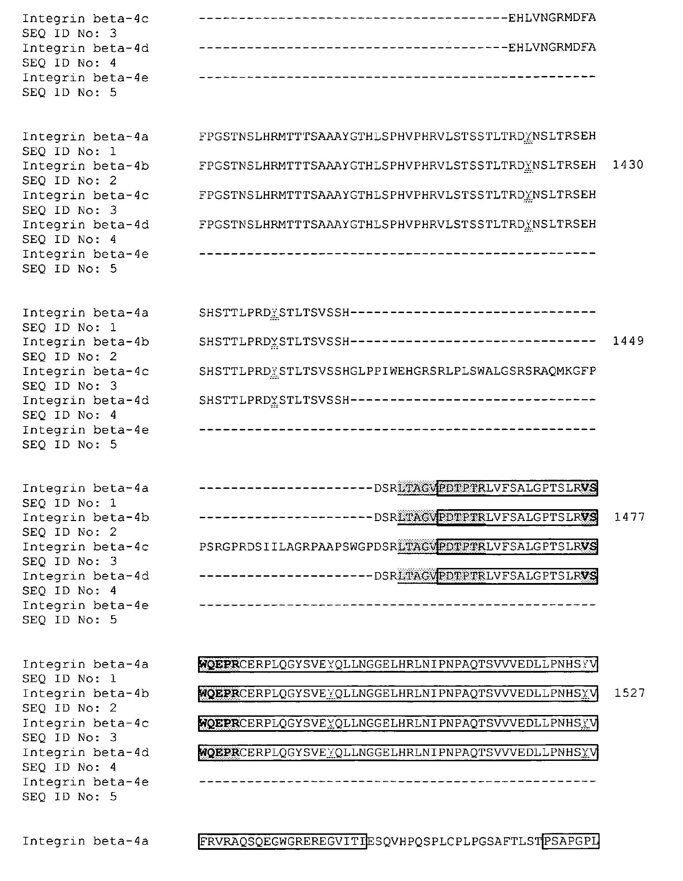

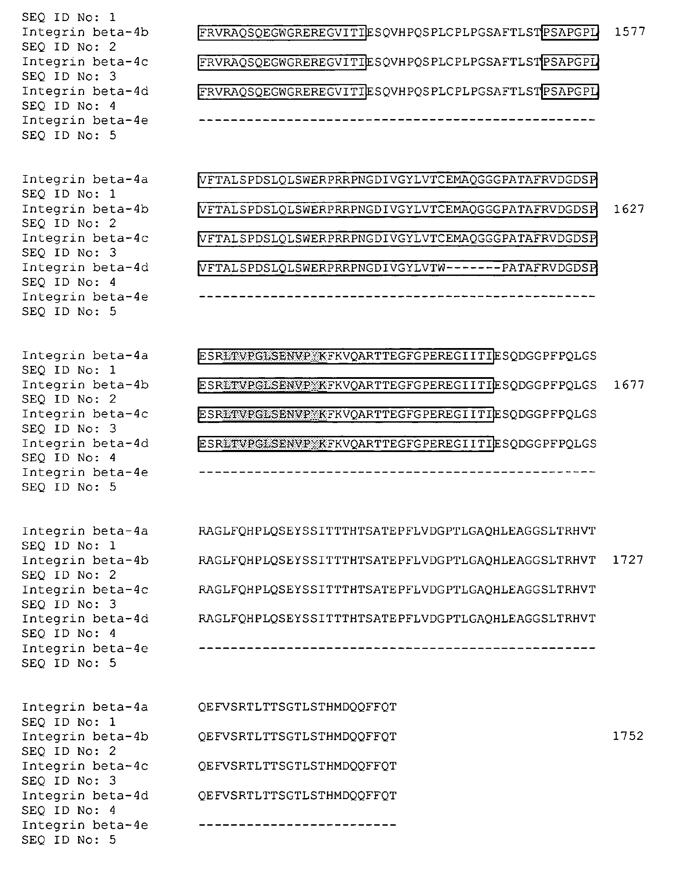

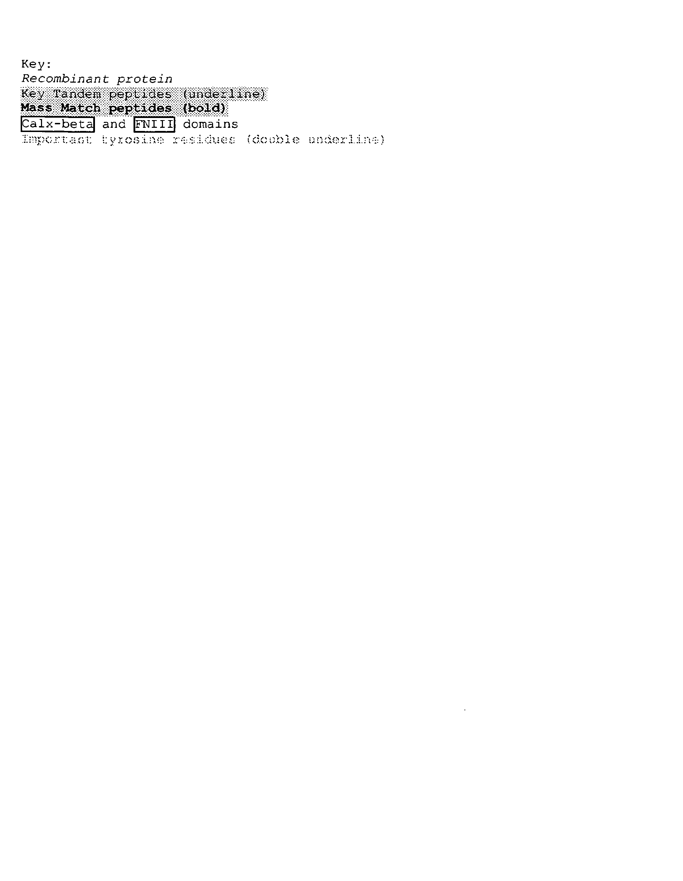

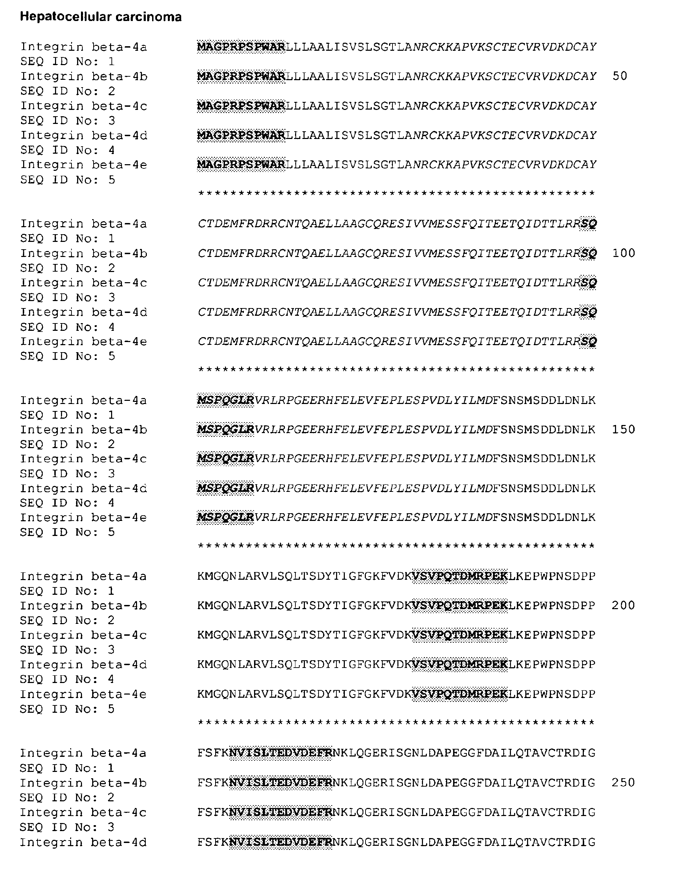

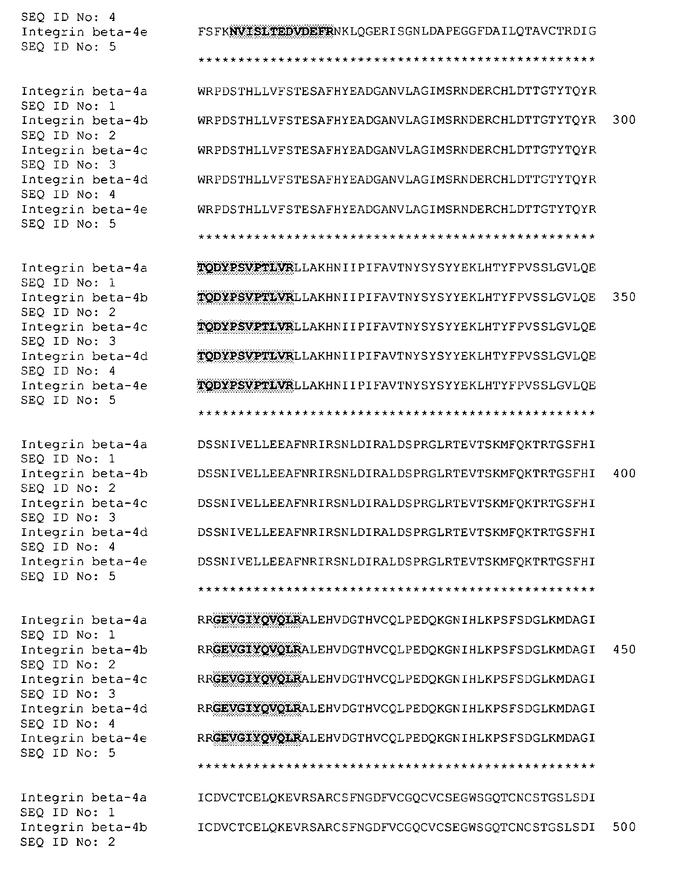

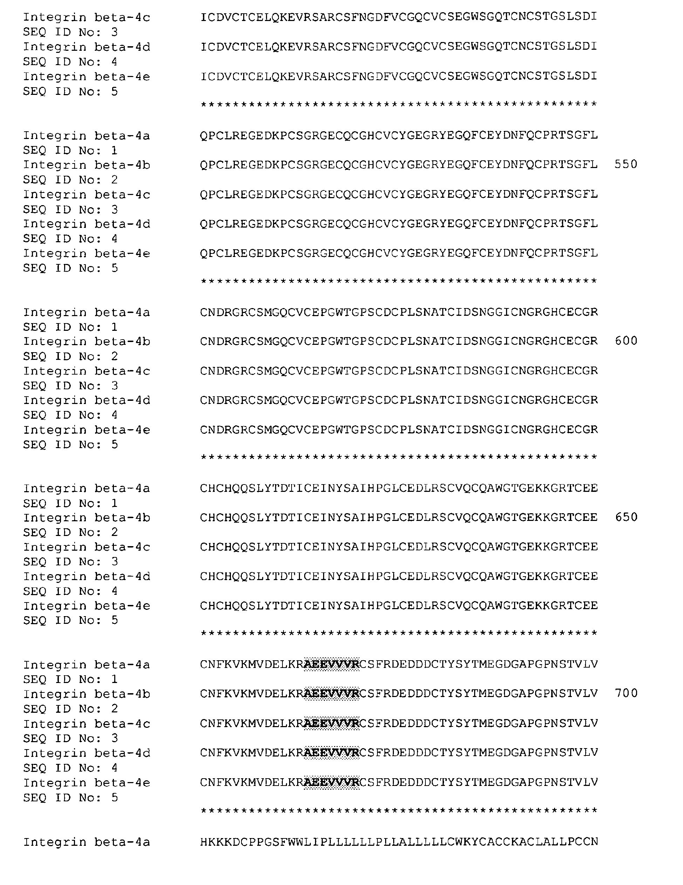

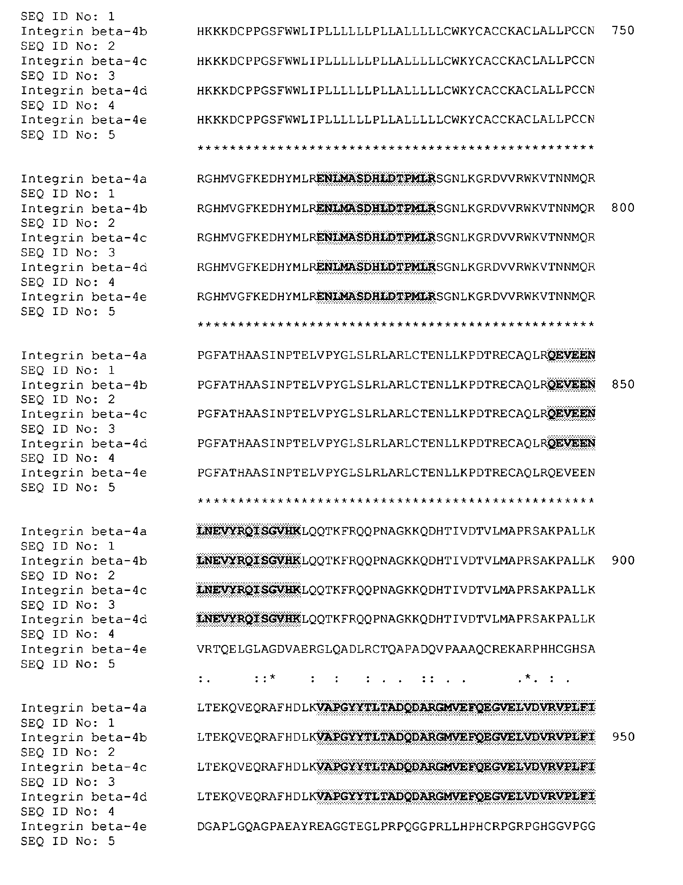

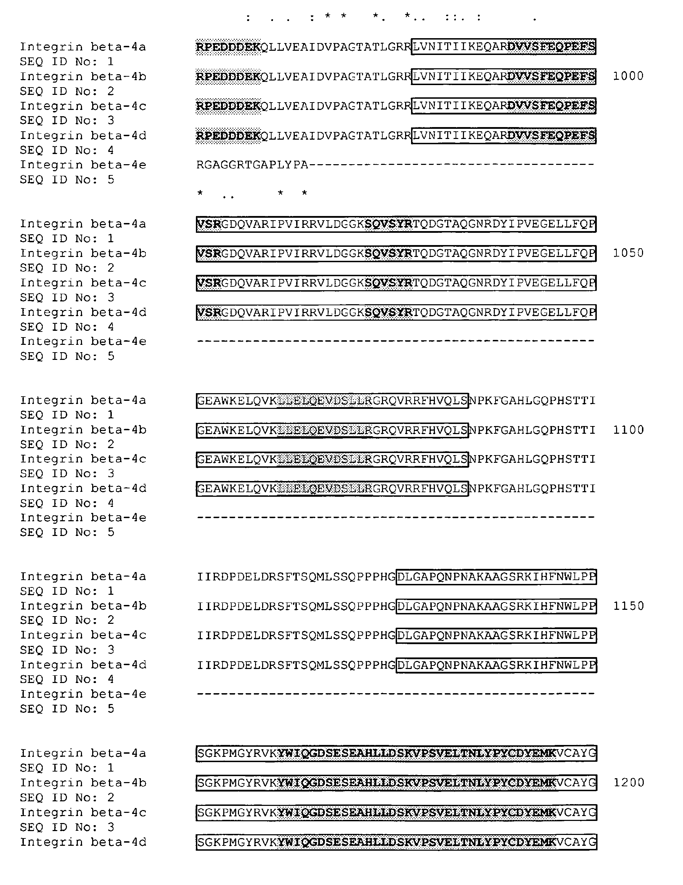

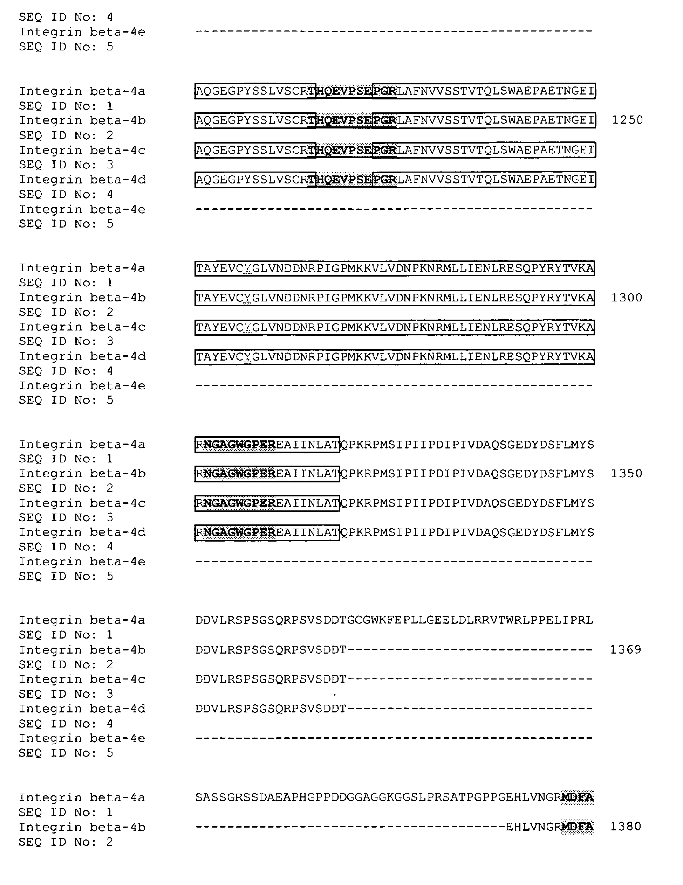

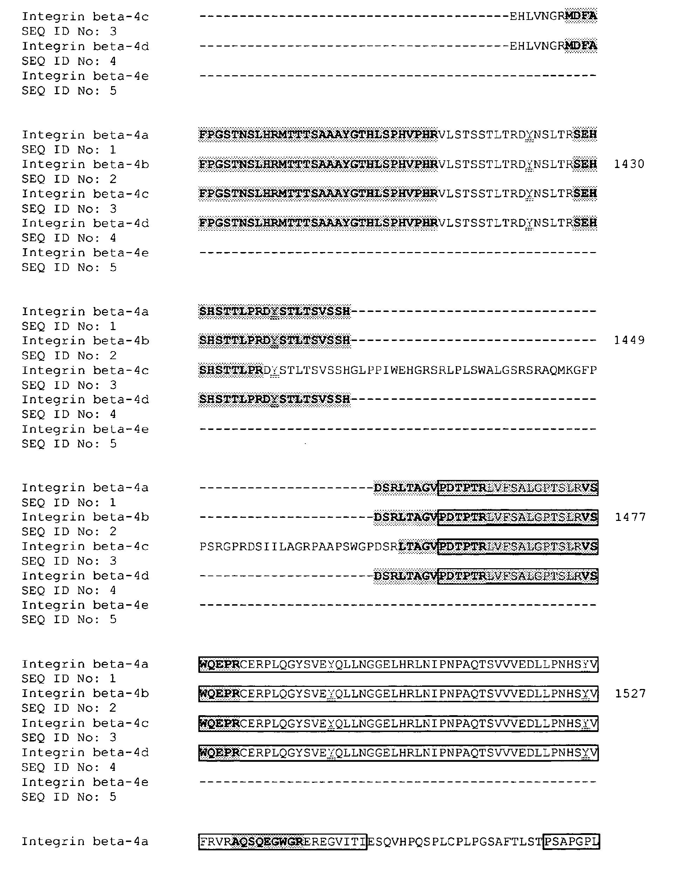

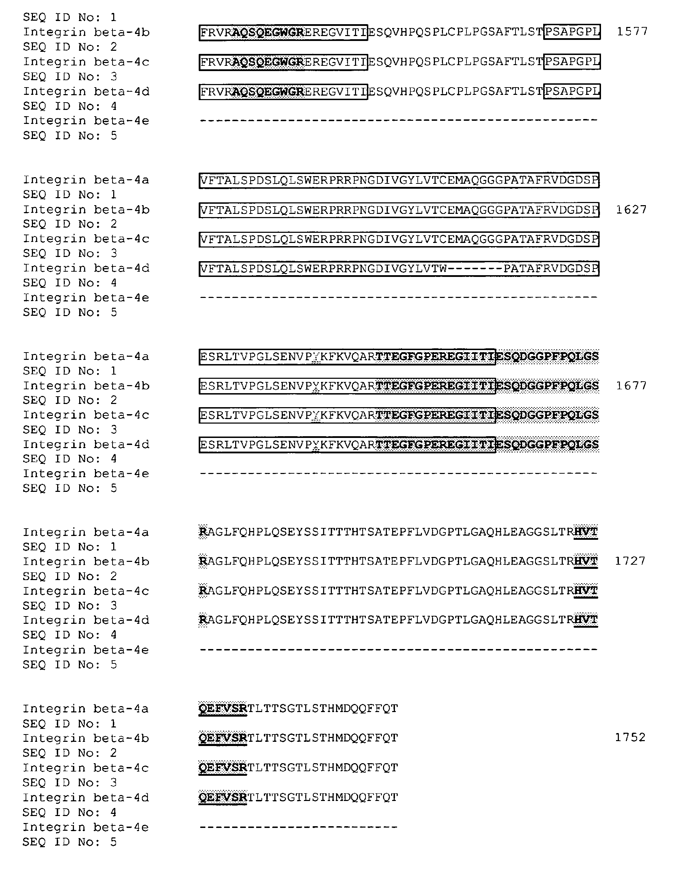

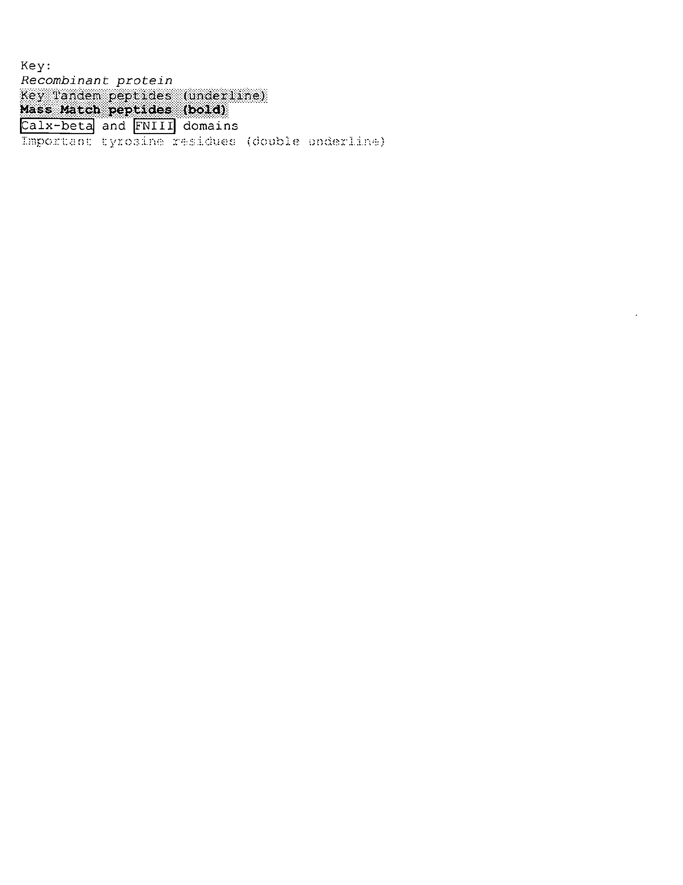

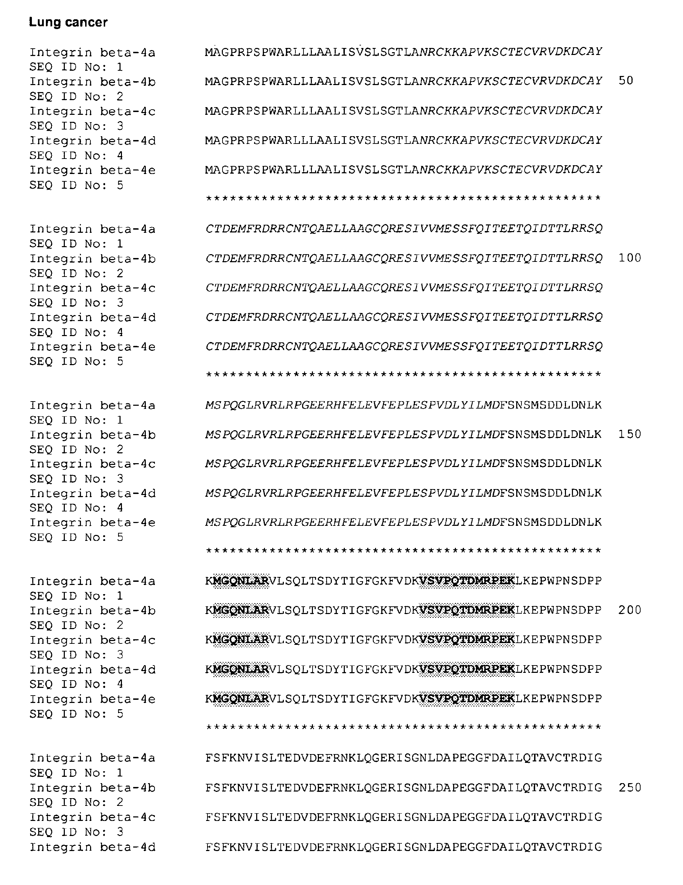

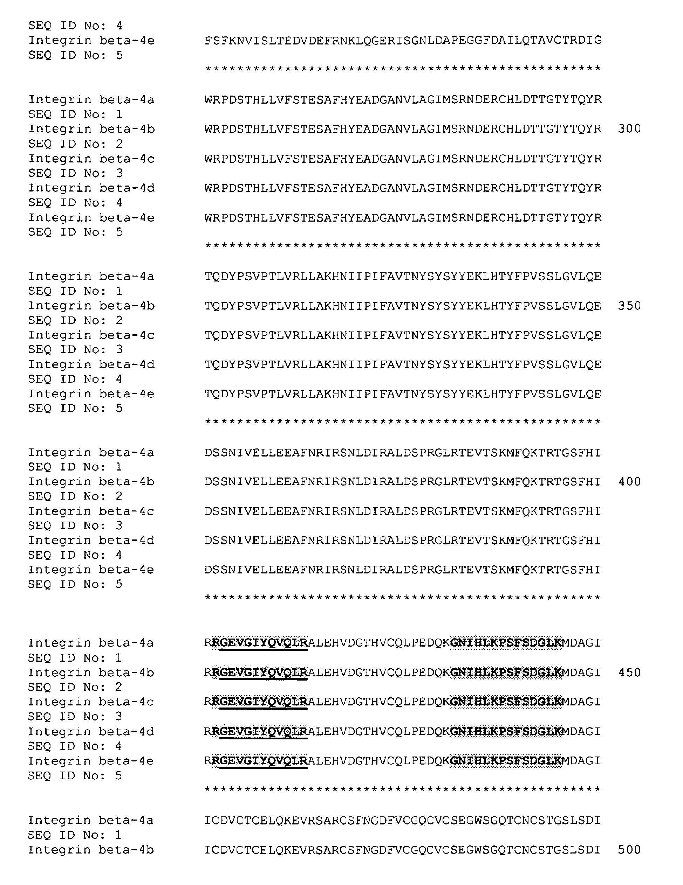

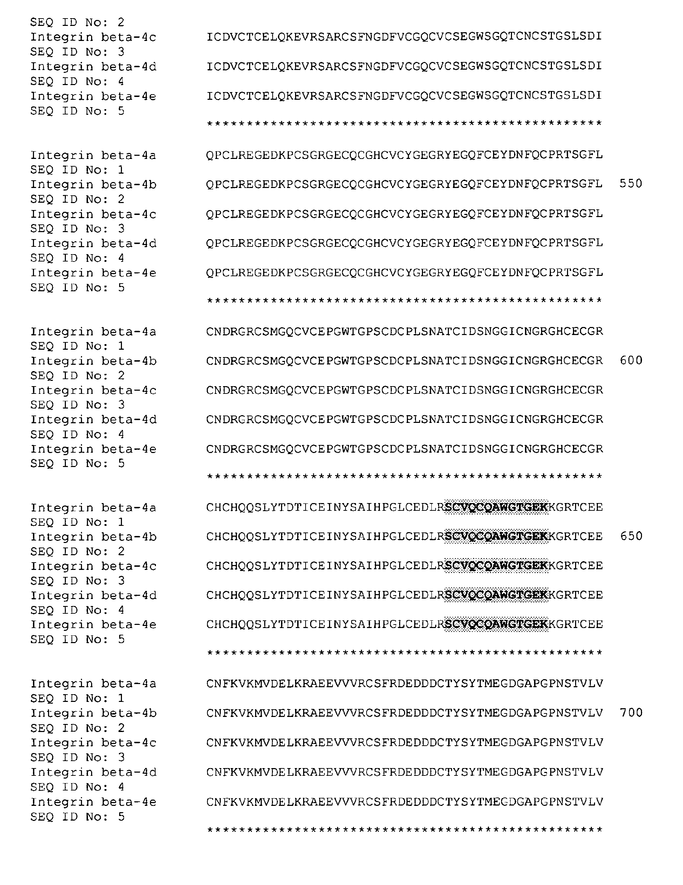

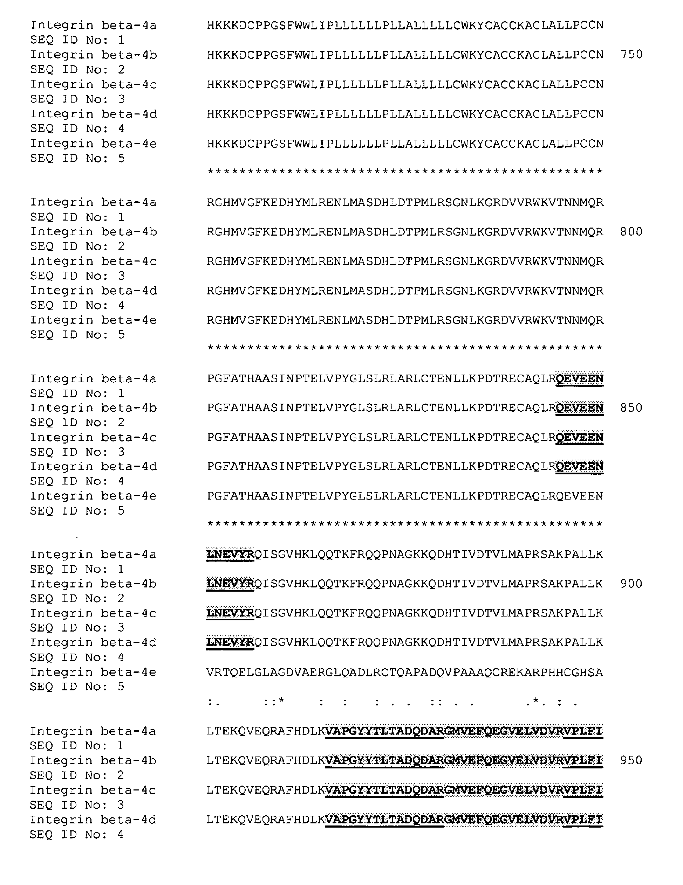

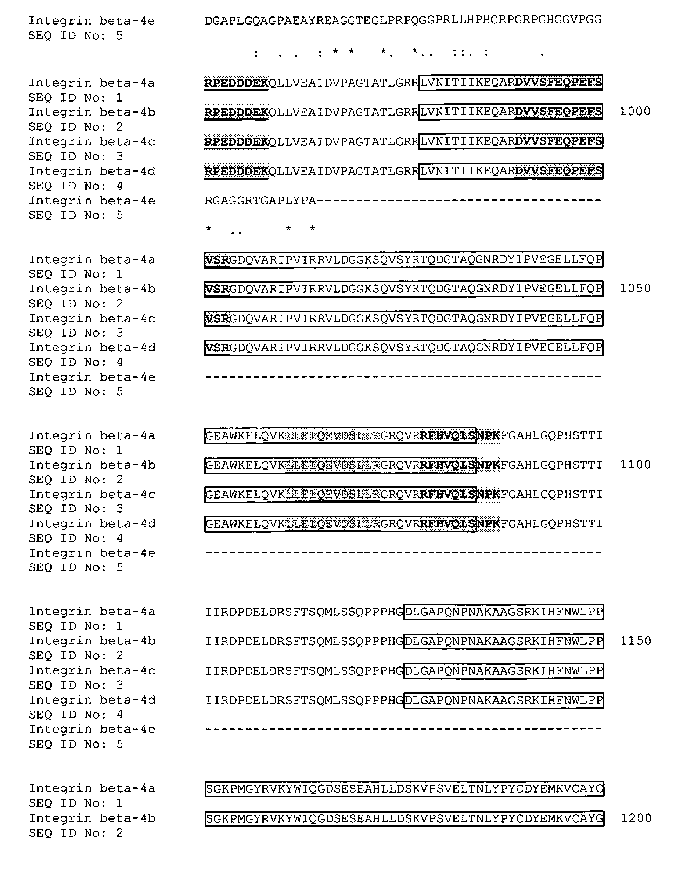

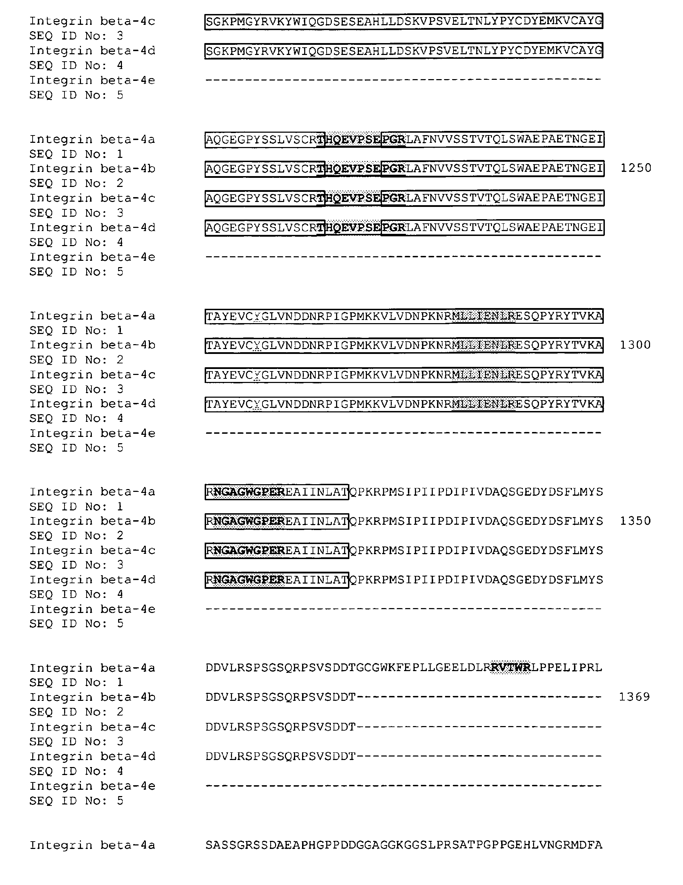

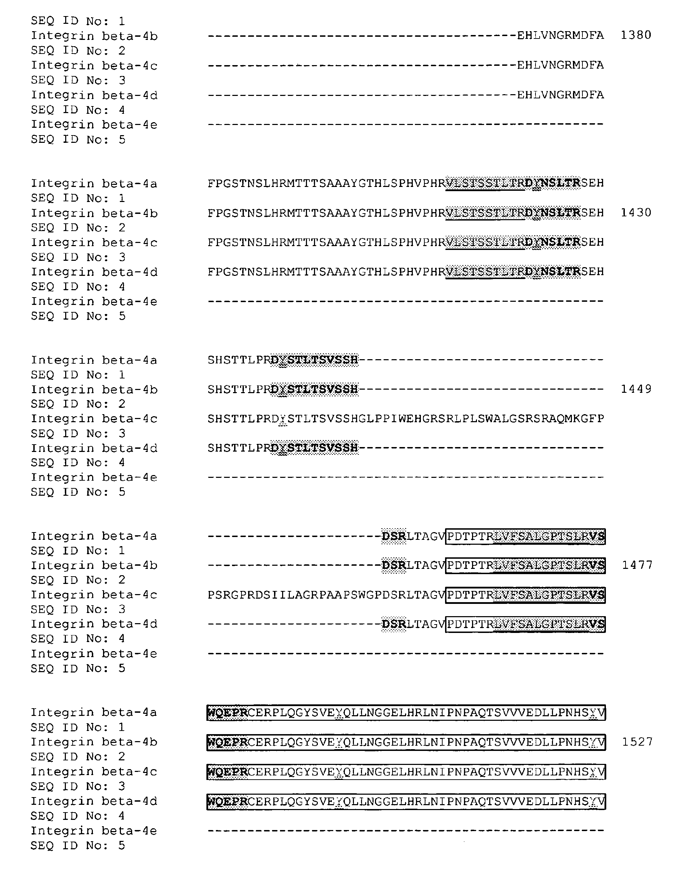

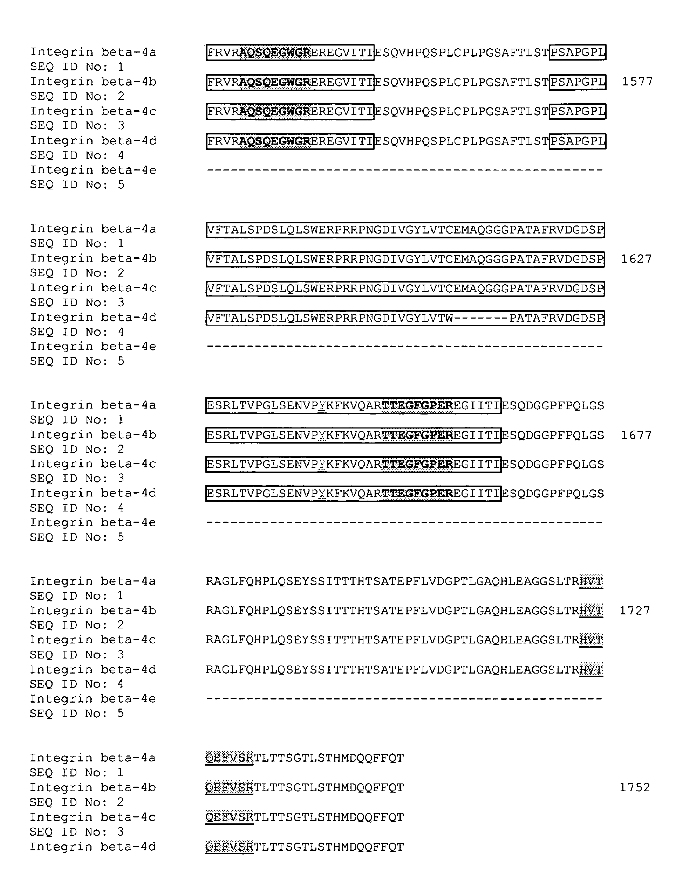

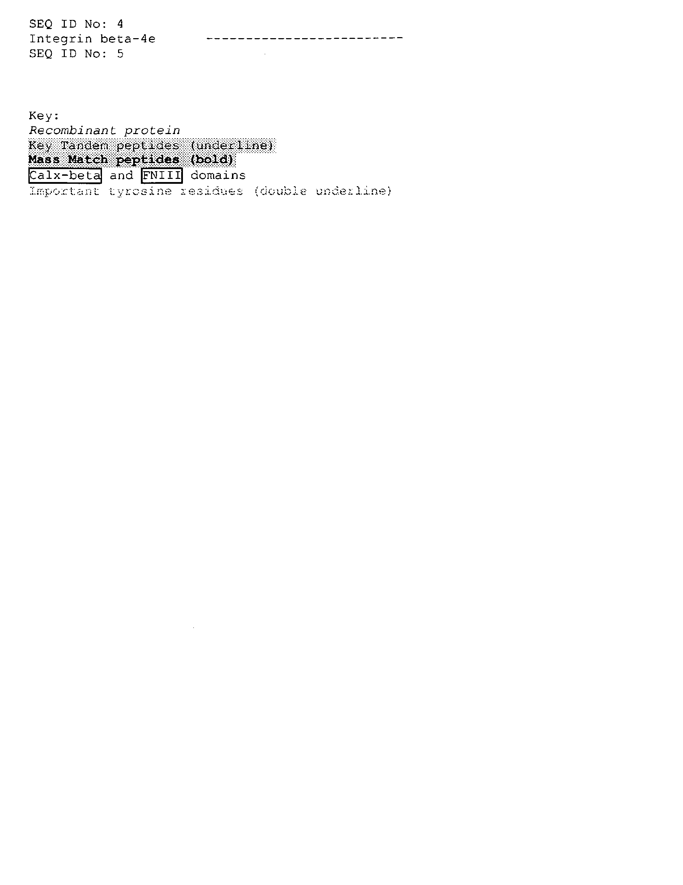

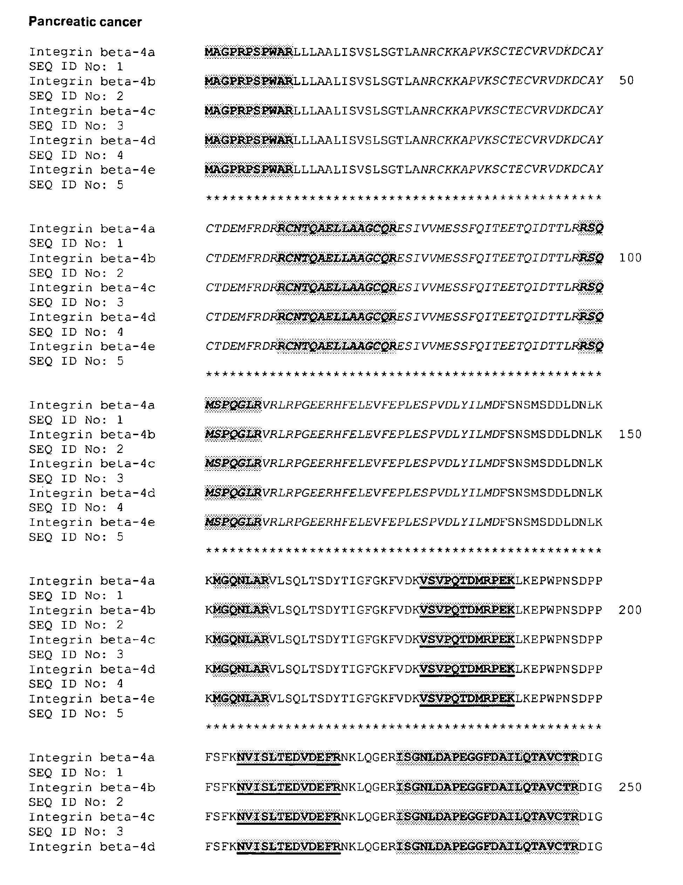

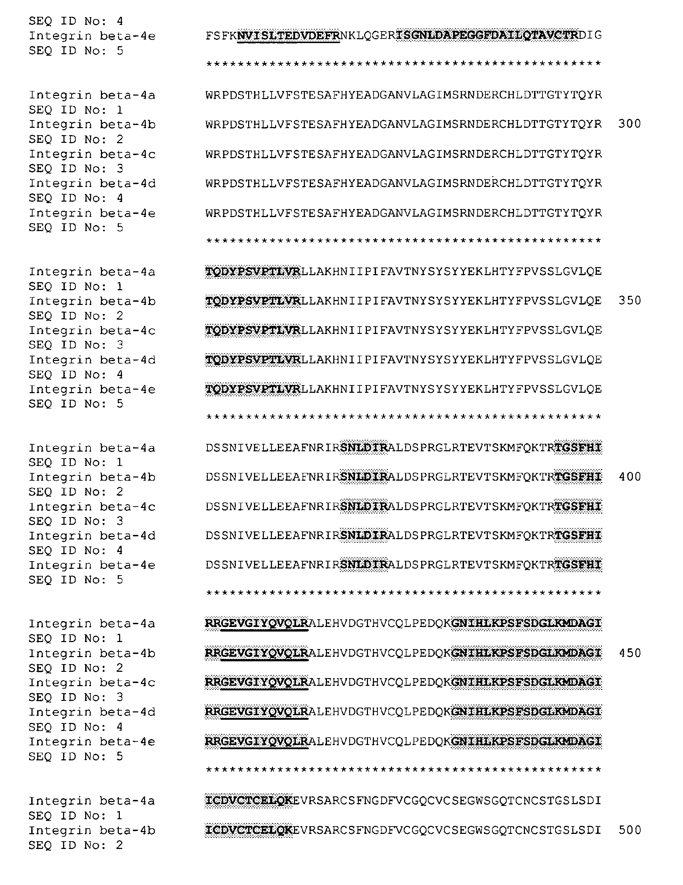

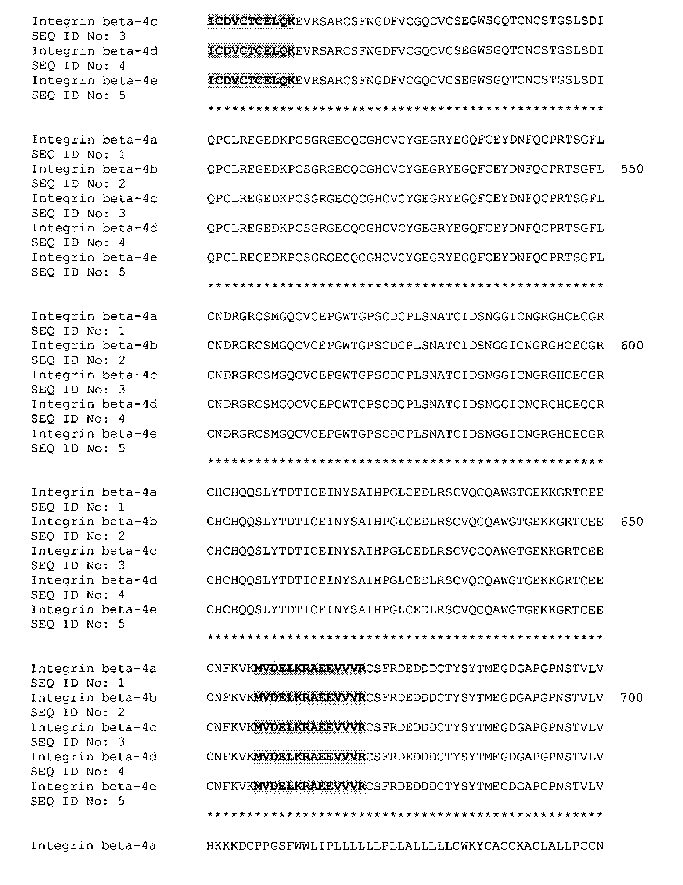

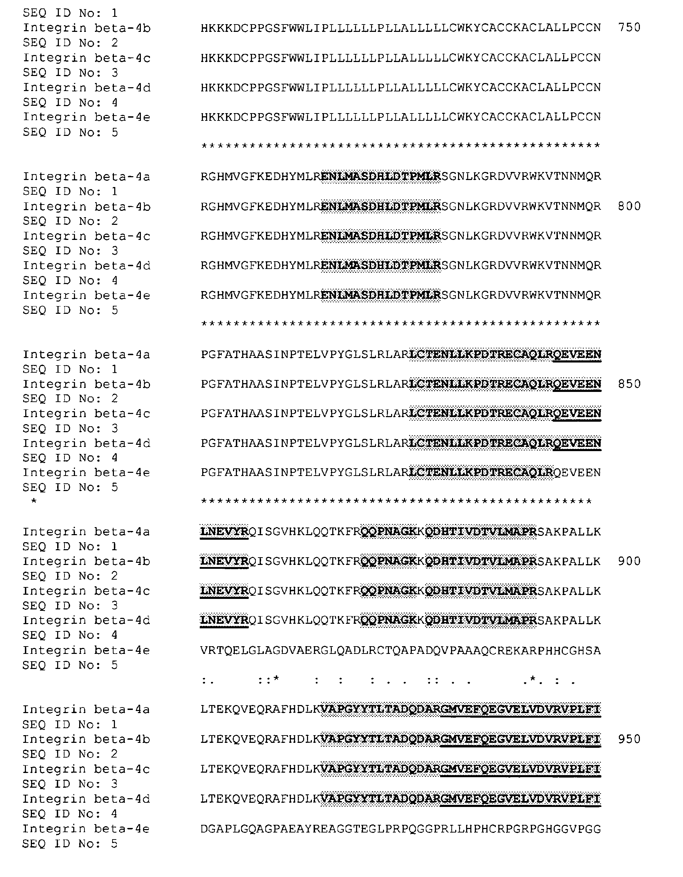

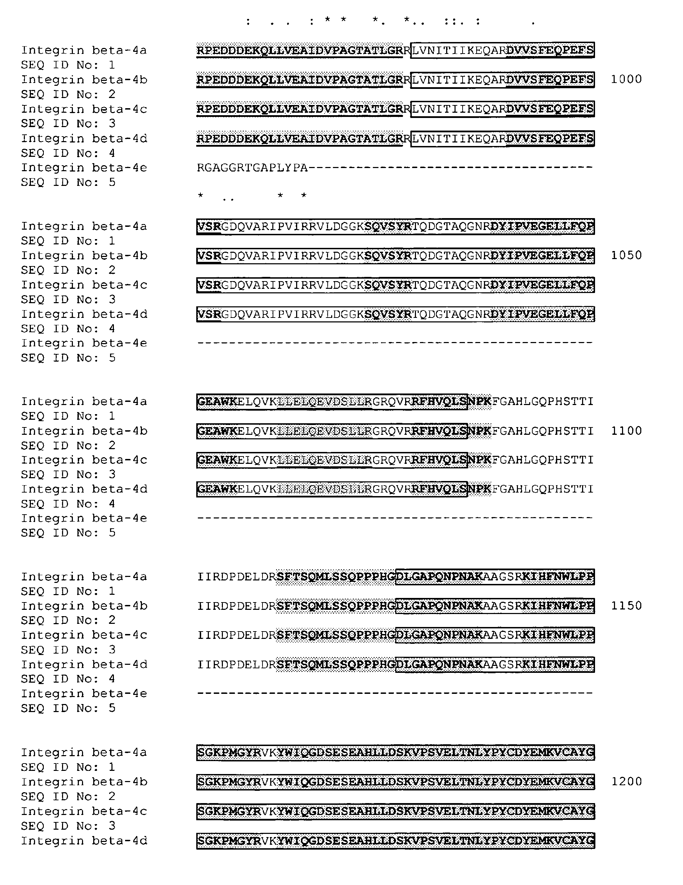

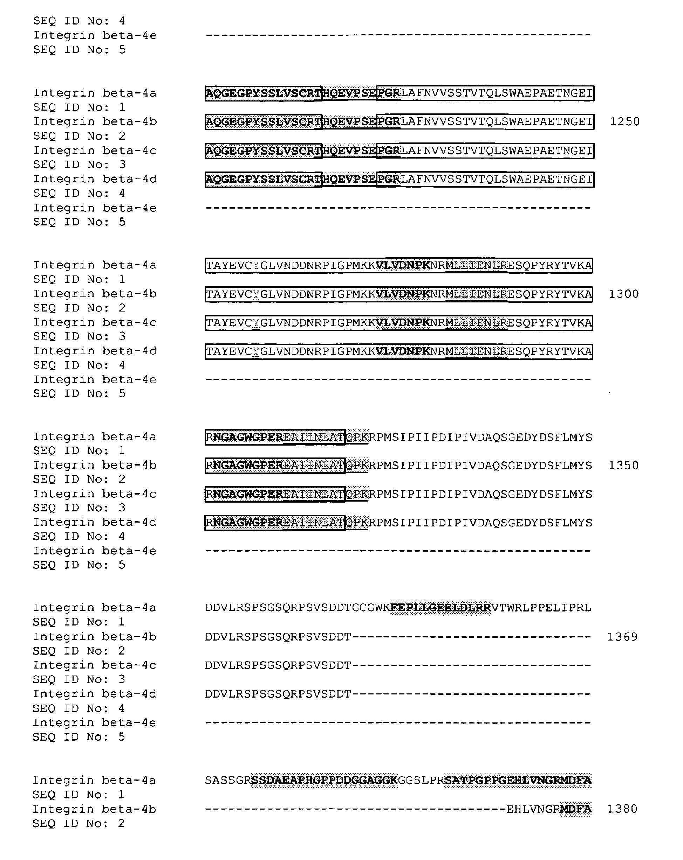

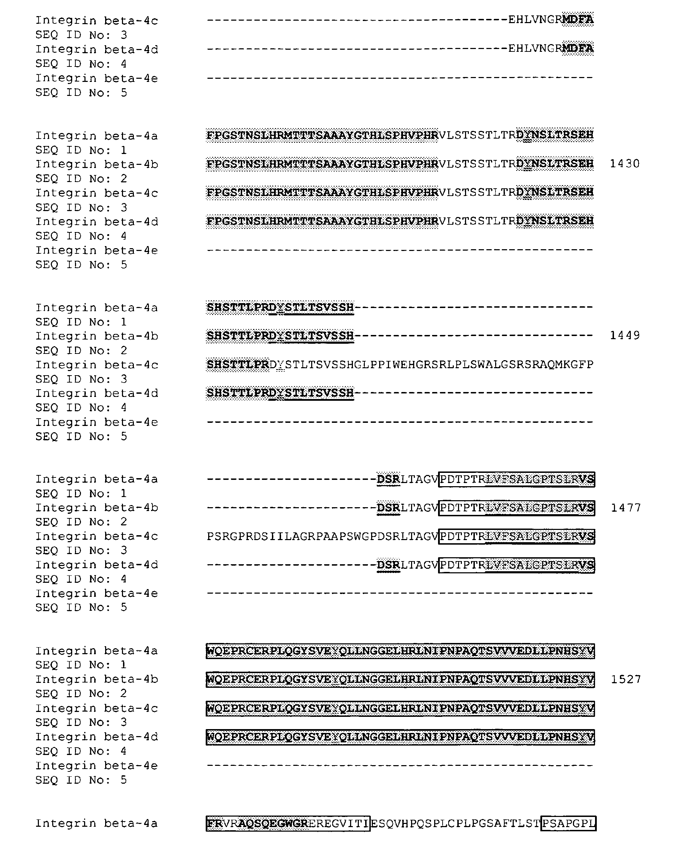

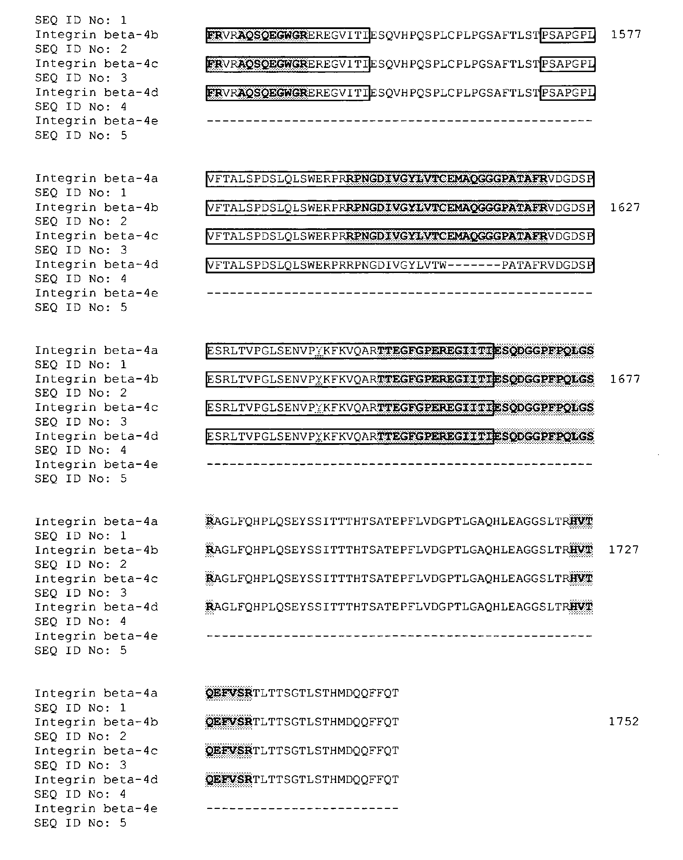

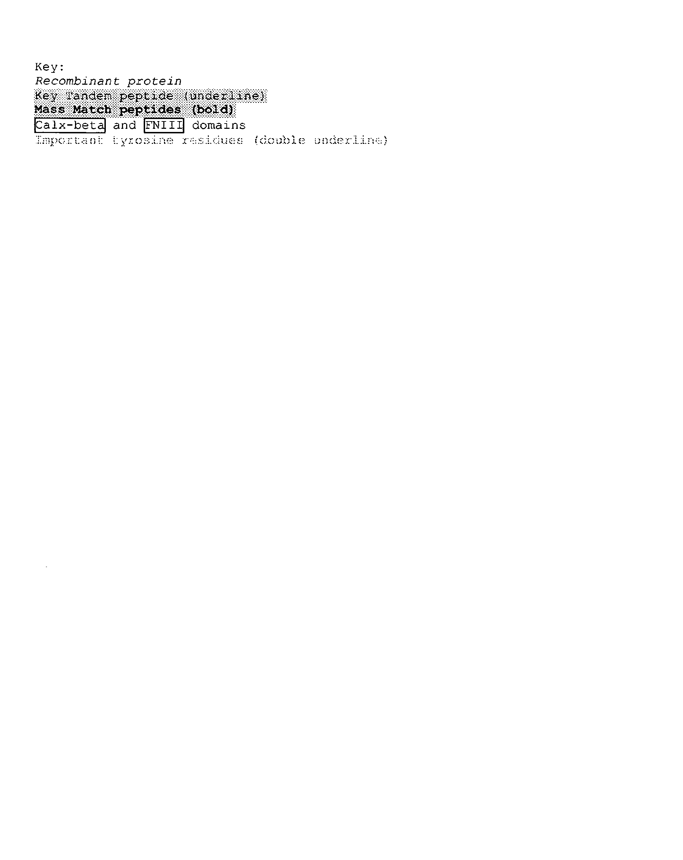

As used herein, the term "Integrin beta 4" refers preferably to the protein illustrated in Figure 1 in all its splice variants, in particular in its five different splice variants detected experimentally by 1D electrophoresis of breast, colorectal, gastric epithelium, liver, lung and pancreatic cancer tissue samples (Integrin beta 4a to Integrin beta 4e). Protein derivatives of these sequences may also be useful for the same purposes as described herein.

This protein has been identified in membrane protein extracts of breast cancer, colorectal cancer, gastric cancer, hepatocellular carcinoma, lung cancer and pancreatic cancer tissue samples from breast cancer, colorectal cancer, gastric cancer, hepatocellular carcinoma, lung cancer and pancreatic cancer patients, through the methods and apparatus of the Preferred Technology (1D gel electrophoresis and tryptic digest of membrane protein extracts). Peptide sequences were compared to the SWISS-PROT and trEMBL databases (held by the Swiss Institute of Bioinformatics (SIB) and the European Bioinformatics Institute (EBI) which are available at www.expasy.com), and the following entry: P16144, Integrin beta-4, was identified.

According to SWISS-PROT, integrin beta-4 is predominantly expressed by epithelia. In addition, Integrin beta 4d is also expressed in colon and placenta while Integrin beta 4e is also expressed in epidermis, lung, duodenum, heart, spleen and stomach. The function of Integrin alpha-6/beta-4 is to be a receptor for laminin. It plays a critical structural role in the hemidesmosome of epithelial cells.

The expression of Integrin beta-4 has been reported to be associated with basal-like cancers and it is hypothesized that Integrin beta-4 may function in concert with a discrete set of proteins to facilitate the aggressive behaviour of a subset of tumours (see, e.g., Lu et al., Analysis of Integrin β4 Expression in Human Breast Cancer: Association with Basal-like Tumors and Prognostic Significance, Clin Cancer Res 2008;14(4):1050-58,

Integrin beta-4 has also been identified as a candidate biomarker for the clinical outcome of tongue squamous cell carcinoma (see, e.g., Kurokawa et al., Diagnostic Value of Integrin α3, β4, and β5 Gene Expression Levels for the Clinical Outcome of Tongue Squamouos Cell Carcinoma, Cancer 2008; 112:1272-81).

Integrin beta-4 has been reported to promote the migratory and invasive phenotype of pancreatic carcinoma cells through the Tiam1-Rac1 pathway in part through the upregulation of Tiam1 (see, e.g., Cruz-Monserrate and O'Connor, Integrin α6β4 Promotes Migration, Invasion through Tiam1 Upregulation, and Subsequent Rac Activation, Neoplasia, 2008; 10(5):408-417 ).

Integrin beta-4 contains two pairs of fibronectin type III (FNIII) repeats in the cytoplasmic domain (see Figure 1). Deletion of the first pair of FNIII repeats has been associated with Carmi syndrome, a rare autosomal recessive disorder (see, e.g., Birnbaum et al., Deletion of the First Pair of Fibronectin Type III Repeats of the Integrin β-4 Gene Is Associated With Epidermolysis Bullosa, Pyloric Atresia and Aplasia Cutis Congenita in the Original Carmi Syndrome Patients, 2008, Am J Med Genet Part A 146A:1063-66).

For further discussion of the function of Integrin beta-4 see Wilhelmsen et al., Multiple Functions of the Integrin α6β4 in Epidermal Homeostasis and Tumorigenesis, Molecular and Cellular Biology, 2006; 26(8);2877-86.

The protein is useful as are fragments particularly epitope containing fragments e.g. antigenic or immunogenic fragments thereof and derivatives thereof. Epitope containing fragments including antigenic or immunogenic fragments will typically be of length 12 amino acids or more e.g. 20 amino acids or more e.g. 50 or 100 amino acids or more. Fragments may be 95% or more of the length of the full protein e.g. 90% or more e.g. 75% or 50% or 25% or 10% or more of the length of the full protein.

Alternatively, the protein/polypeptide employed or referred to herein may be limited to those specifically recited/described in the present specification or a moiety 80, 85, 90, 91, 92, 93, 94, 95, 96, 97, 98 or 99% identical or similar thereto.

Epitope containing fragments including antigenic or immunogenic fragments will be capable of eliciting a relevant immune response in a patient. DNA encoding the protein of the invention is also useful as are fragments thereof e.g. DNA encoding fragments of the protein such as immunogenic fragments thereof. Fragments of nucleic acid (e.g. DNA) encoding the protein may be 95% or more of the length of the full coding region e.g. 90% or more e.g. 75% or 50% or 25% or 10% or more of the length of the full coding region. Fragments of nucleic acid (e.g. DNA) may be 36 nucleotides or more e.g. 60 nucleotides or more e.g. 150 or 300 nucleotides or more in length.

Derivatives of the protein of the invention include variants on the sequence in which one or more (e.g. 1-20 such as 15 amino acids, or up to 20% such as up to 10% or 5% or 1% by number of amino acids based on the total length of the protein) deletions, insertions or substitutions have been made. Substitutions may typically be conservative substitutions. Derivatives will typically have essentially the same biological function as the protein from which they are derived. Derivatives will typically be comparably antigenic or immunogenic to the protein from which they are derived. Derivatives will typically have either the ligand-binding activity, or the active receptor-complex forming ability, or preferably both, of the protein from which they are derived.

Derivatives of proteins also include chemically treated protein such as carboxymethylated, carboxyamidated, acetylated proteins, for example treated during purification.

Tables 1a to 1f below illustrate the different occurrences of Integrin beta 4 as detected by 1D gel electrophoresis and mass spectrometry of membrane protein extracts of breast, colorectal, gastric epithelium, liver, lung and pancreatic tissue , samples from breast cancer, colorectal cancer, gastric cancer, hepatocellular carcinoma, lung cancer and pancreatic cancer patients respectively. The first column provides the molecular weight, the second column gives information on the subfractionation protocol used, if any (see Example 1 below), the third column gives information on the preferred splice variants, based on tandem peptides detected experimentally by mass spectrometry (see Figure 1), and the last column provides a list of the sequences observed by mass spectrometry and the corresponding SEQ ID Nos.

**Table 1a - Breast cancer**

| MW (Da) | Subfractionation | Preferred splice variants | Tryptics identified [SEQ ID No] |
|---|---|---|---|
| 153448 | | Integrin beta 4a, Integrin beta 4b, Integrin beta 4c, Integrin beta 4d | |
| 162825 | | Integrin beta 4a, Integrin beta 4b, Integrin beta 4c, Integrin beta 4d | |
| 164389 | | Integrin beta 4a, Integrin beta 4b, Integrin beta 4c, Integrin beta 4d | |
| 165165 | | Integrin beta 4a, Integrin beta 4b, Integrin beta 4c, Integrin beta 4d | |

**Table 1b Colorectal Cancer**

| MW (Da) | Subfractionation | Preferred splice variants | Tryptics identified [SEQ ID No] |
|---|---|---|---|
| 88905 | | Integrin beta 4a, Integrin beta 4b, Integrin beta 4c, Integrin beta 4d | |
| 104028 | | Integrin beta 4a, Integrin beta 4b, Integrin beta 4c, Integrin beta 4d | |
| 107091 | | Integrin beta 4a, Integrin beta 4b, Integrin beta 4c, Integrin beta 4d | |
| 113823 | | Integrin beta 4a, Integrin beta 4b, Integrin beta 4c, Integrin beta 4d | |
| 117533 | | Integrin beta 4a, Integrin beta 4b, Integrin beta 4c, Integrin beta 4d | |
| 121506 | | Integrin beta 4a, Integrin beta 4b, Integrin beta 4c, Integrin beta 4d | |
| 125772 | | Integrin beta 4a, Integrin beta 4b, Integrin beta 4d | LVFSALGPTSLR [39] |
| 130363 | | Integrin beta 4a, Integrin beta 4b, Integrin beta 4c, Integrin beta 4d | LVFSALGPTSLR [39] |
| 140688 | | Integrin beta 4a, Integrin beta 4b, Integrin beta 4c, Integrin beta 4d | |
| 146522 | | Integrin beta 4b, Integrin beta 4c, Integrin beta 4d | |
| 159861 | | Integrin beta 4a, Integrin beta 4b, Integrin beta 4c, Integrin beta 4d | |
| | Heparin Binding | Integrin beta 4a, Integrin beta 4b, Integrin beta 4c, Integrin beta 4d | |
| | Heparin Binding | Integrin beta 4a, Integrin beta 4b, Integrin beta 4c, Integrin beta 4d | |

**Table 1c - Gastric cancer**

| MW (Da) | Subfractionation | Preferred splice variants | Tryptics identified [SEQ ID No] |
|---|---|---|---|
| 84210 | | Integrin beta 4a, Integrin beta 4b, Integrin beta 4c, Integrin beta 4d | |
| 85955 | | Integrin beta 4a, Integrin beta 4b, Integrin beta 4c, Integrin beta 4d | DPDELDR [12] |
| 87777 | | Integrin beta 4a, Integrin beta 4b, Integrin beta 4c, Integrin beta 4d | GMVEFQEGVELVDVR [27] |
| 98257 | | Integrin beta 4a, Integrin beta 4b, Integrin beta 4c, Integrin beta 4d, Integrin beta 4e | NVISLTEDVDEFR [48] |
| 100678 | | Integrin beta 4a, Integrin beta 4b, Integrin beta 4c, Integrin beta 4d | |
| 114938 | | Integrin beta 4a, Integrin beta 4b, Integrin beta 4c, Integrin beta 4d | LTAGVPDTPTR [37] |
| 121910 | | Integrin beta 4a, Integrin beta 4b, Integrin beta 4c, Integrin beta 4d | |
| 125740 | | Integrin beta 4a, Integrin beta 4b, Integrin beta 4c, Integrin beta 4d | |
| 129831 | | Integrin beta 4a, Integrin beta 4b, Integrin beta 4c, Integrin beta 4d | |

**Table 1d - Hepatocellular carcinoma**

| MW (Da) | Subfractionation | Preferred splice variants | Tryptics identified [SEQ ID No] |
|---|---|---|---|
| 82761 | | Integrin beta 4a, Integrin beta 4b, Integrin beta 4c, Integrin beta 4d | LLELQEVDSLLR [35] |
| 102119 | | Integrin beta 4a, Integrin beta 4b, Integrin beta 4c, Integrin beta 4d | |
| 106345 | | Integrin beta 4a, Integrin beta 4b, Integrin beta 4c, Integrin beta 4d | |
| 114377 | | Integrin beta 4a, Integrin beta 4b, Integrin beta 4c, Integrin beta 4d | |
| 178322 | | Integrin beta 4a, Integrin beta 4b, Integrin beta 4c, Integrin beta 4d | LLELQEVDSLLR [35] |

**Table 1e - Lung cancer**

| MW (Da) | Subfractionation | Preferred splice variants | Tryptics identified [SEQ ID No] |
|---|---|---|---|
| 181240 | | Integrin beta 4a, Integrin beta 4b, Integrin beta 4c, Integrin beta 4d | |

**Table 1f - Pancreatic cancer**

| MW (Da) | Subfractionation | Preferred splice variants | Tryptics identified [SEQ ID No] |
|---|---|---|---|
| 31738 | | Integrin beta 4a, Integrin beta 4b, Integrin beta 4c, Integrin beta 4d, Integrin beta 4e | |
| 105212 | | Integrin beta 4a, Integrin beta 4b, Integrin beta 4c, Integrin beta 4d | |
| 107788 | | Integrin beta 4a, Integrin beta 4b, Integrin beta 4c, Integrin beta 4d | |
| 122734 | | Integrin beta 4a, Integrin beta 4b, Integrin beta 4c, Integrin beta 4d | |
| 126215 | | Integrin beta 4a, Integrin beta 4b, Integrin beta 4c, Integrin beta 4d | |
| 134980 | | Integrin beta 4a, Integrin beta 4b, Integrin beta 4c, Integrin beta 4d | |
| 142690 | | Integrin beta 4a, Integrin beta 4b, Integrin beta 4c, Integrin beta 4d | |
| 148609 | | Integrin beta 4a, Integrin beta 4b, Integrin beta 4c, Integrin beta 4d | |
| 154726 | | Integrin beta 4a, Integrin beta 4b, Integrin beta 4c, Integrin beta 4d | |
| 161314 | | Integrin beta 4a, Integrin beta 4b, Integrin beta 4c, Integrin beta 4d | |
| 167857 | | Integrin beta 4a, Integrin beta 4b, Integrin beta 4c, Integrin beta 4d | |
| 168430 | | Integrin beta 4a, Integrin beta 4b, Integrin beta 4c, Integrin beta 4d | |
| 174222 | | Integrin beta 4a, Integrin beta 4b, Integrin beta 4c, Integrin beta 4d | |
| 179222 | | Integrin beta 4a, Integrin beta 4b, Integrin beta 4d | |
| 192682 | | Integrin beta 4a, Integrin beta 4b, Integrin beta 4c, Integrin beta 4d | |
| 198088 | | Integrin beta 4a, Integrin beta 4b, Integrin beta 4c, Integrin beta 4d | EAIINLATQPK [17] |
| 207419 | | Integrin beta 4a, Integrin beta 4b, Integrin beta 4c, Integrin beta 4d | |
| 208929 | | Integrin beta 4a, Integrin beta 4b, Integrin beta 4c, Integrin beta 4d | |

For Integrin beta 4, the detected level obtained upon analyzing tissue from subjects having breast cancer, colorectal cancer, gastric cancer, hepatocellular carcinoma, lung cancer or pancreatic cancer relative to the detected level obtained upon analyzing tissue from subjects free from breast cancer, colorectal cancer, gastric cancer, hepatocellular carcinoma, lung cancer and pancreatic cancer will depend upon the particular analytical protocol and detection technique that is used. Accordingly, each laboratory may establish a reference range in subjects free from breast cancer, colorectal cancer, gastric cancer, hepatocellular - carcinoma, lung cancer and pancreatic cancer according to the analytical protocol and detection technique in use, as is conventional in the diagnostic art. Preferably, at least one control positive tissue sample from a subject known to have breast cancer, colorectal cancer, gastric cancer, hepatocellular carcinoma, lung cancer or pancreatic cancer or at least one control negative tissue sample from a subject known to be free from breast cancer, colorectal cancer, gastric cancer, hepatocellular carcinoma, lung cancer and pancreatic cancer (and more preferably both positive and negative control samples) are included in each batch of test samples analysed.

Integrin beta 4 can be used for detection, prognosis, diagnosis, or monitoring of breast cancer, colorectal cancer, gastric cancer, hepatocellular carcinoma, lung cancer and pancreatic cancer or for drug development. Tissue from a subject (*e.g.*, a subject suspected of having breast cancer, colorectal cancer, gastric cancer, hepatocellular carcinoma, lung cancer or pancreatic cancer) may be analysed by 1D electrophoresis for detection of Integrin beta 4. An increased abundance of Integrin beta 4 in the tissue from the subject relative to tissue from a subject or subjects free from breast cancer, colorectal cancer, gastric cancer, hepatocellular carcinoma, lung cancer and pancreatic cancer (e.g., a control sample) or a previously determined reference range indicates the presence of breast cancer, colorectal cancer, gastric cancer, hepatocellular carcinoma, lung cancer or pancreatic cancer.

The sequences shown in Table 1 may be employed in any relevant aspect of the invention.

In a particular embodiment, specific splice variants of Integrin beta 4 are involved. As indicated in Table 1, for colorectal cancer the preferred splice variants are Integrin beta 4b, Integrin beta 4c and Integrin beta 4d; for breast cancer the preferred splice variants are Integrin beta 4a, Integrin beta 4b, Integrin beta 4c and Integrin beta 4d; for gastric cancer the preferred splice variants are Integrin beta 4a, Integrin beta 4b, Integrin beta 4c and Integrin beta 4d; for hepatocellular carcinoma the preferred splice variants are Integrin beta 4a, Integrin beta 4b, Integrin beta 4c and Integrin beta 4d; for lung cancer the preferred splice variants are Integrin beta 4a, Integrin beta 4b, Integrin beta 4c and Integrin beta 4d and for pancreatic cancer the preferred splice variants are Integrin beta 4a, Integrin beta 4b and Integrin beta 4d.

Integrin beta 4 may, in particular, be characterized as an isoform having a MW substantially as recited (e.g. +/- 10%, particularly +/-5% of the value) in column 1 of any of the rows of Tables 1a-1f.

In relation to variants, fragments, epitope-containing fragments, immunogenic fragments or antigenic fragments of Integrin beta 4:
- for breast cancer applications: in one aspect of the invention these comprise one or more of the sequences identified as tryptic sequences in the 4^{th} column of Table 1a;
- for colorectal cancer applications: in one aspect of the invention these comprise one or more of the sequences identified as tryptic sequences in the 4^{th} column of Table 1b;
- for gastric cancer applications: in one aspect of the invention these comprise one or more of the sequences identified as tryptic sequences in the 4^{th} column of Table 1c;
- for hepatocellular cancer applications: in one aspect of the invention these comprise one or more of the sequences identified as tryptic sequences in the 4^{th} column of Table 1d;
- for lung cancer applications: in one aspect of the invention these comprise one or more of the sequences identified as tryptic sequences in the 4^{th} column of Table 1e;
- for pancreatic cancer applications: in one aspect of the invention these comprise one or more of the sequences identified as tryptic sequences in the 4^{th} column of Table 1f.

As used herein, Integrin beta 4 is "isolated" when it is present in a preparation that is substantially free of contaminating proteins, i.e., a preparation in which less than 10% (preferably less than 5%, more preferably less than 1%) of the total protein present is contaminating protein(s). A contaminating protein is a protein having a significantly different amino acid sequence from that of isolated Integrin beta 4, as determined by mass spectral analysis. As used herein, a "significantly different" sequence is one that permits the contaminating protein to be resolved from Integrin beta 4 by mass spectral analysis, performed according to the Reference Protocol. fragment thereof and an adjuvant.

Integrin beta 4 can be assayed by any method known to those skilled in the art, including but not limited to, the Preferred Technology described herein, kinase assays, enzyme assays, binding assays and other functional assays, immunoassays, and western blotting. In one embodiment, Integrin beta 4 is separated on a 1-D gel by virtue of its MW and visualized by staining the gel. In one embodiment, Integrin beta 4 is stained with a fluorescent dye and imaged with a fluorescence scanner. Sypro Red (Molecular Probes, Inc., Eugene, Oregon) is a suitable dye for this purpose. A preferred fluorescent dye is disclosed in U.S. Application No. 09/412,168, filed on October 5, 1999, (US 6,335,446).

Alternatively, Integrin beta 4 can be detected in an immunoassay. In one embodiment, an immunoassay is performed by contacting a sample from a subject to be tested with an anti-Integrin beta 4 antibody (or other affinity reagent) under conditions such that binding (e.g. immunospecific binding) can occur if Integrin beta 4 is present, and detecting or measuring the amount of any binding (e.g. immunospecific binding) by the affinity reagent. Integrin beta 4 binding agents can be produced by the methods and techniques taught herein.

Integrin beta 4 may be detected by virtue of the detection of a fragment thereof e.g. an epitope containing (e.g. an immunogenic or antigenic) fragment thereof. Fragments may have a length of at least 10, more typically at least 20 amino acids e.g. at least 50 or 100 amino acids e.g. at least 200 or 500 amino acids e.g. at least 1000 amino acids e.g. at least 1500 amino acids.

Binding of an affinity reagent (e.g. an antibody) in tissue sections can be used to detect aberrant Integrin beta 4 localization or an aberrant level of Integrin beta 4. An antibody (or other affinity reagent) to Integrin beta 4 can be used to assay a patient tissue (e.g., breast, colorectal, gastric epithelium, liver, lung or pancreatic tissue) for the level of Integrin beta 4 where an aberrant level of Integrin beta 4 is indicative of breast cancer, colorectal cancer, gastric cancer, hepatocellular carcinoma, lung cancer or pancreatic cancer. As used herein, an "aberrant level" means a level that is increased compared with the level in a subject free from breast cancer, colorectal cancer, gastric cancer, hepatocellular carcinoma, lung cancer and pancreatic cancer or a reference level.

Any suitable immunoassay can be used, including, without limitation, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

For example, Integrin beta 4 can be detected in a fluid sample (e.g., blood, urine, or saliva) by means of a two-step sandwich assay. In the first step, a capture reagent (e.g., an anti-Integrin beta 4 antibody or other affinity reagent) is used to capture Integrin beta 4. The capture reagent can optionally be immobilized on a solid phase. In the second step, a directly or indirectly labeled detection reagent is used to detect the captured Integrin beta 4. The detection reagent may be a lectin. Any lectin can be used for this purpose that preferentially binds to Integrin beta 4 rather than to other isoforms that have the same core protein as Integrin beta 4 or to other proteins that share the antigenic determinant recognized by the antibody. In a preferred embodiment, the chosen lectin binds Integrin beta 4 with at least 2-fold greater affinity, more preferably at least 5-fold greater affinity, still more preferably at least 10-fold greater affinity, than to said other isoforms that have the same core protein as Integrin beta 4 or to said other proteins that share the antigenic determinant recognized by the affinity reagent. Based on the present description, a lectin that is suitable for detecting Integrin beta 4 can readily be identified by methods well known in the art, for instance upon testing one or more lectins enumerated in Table I on pages 158-159 of Sumar et al., Lectins as Indicators of Disease-Associated Glycoforms, In: Gabius H-J & Gabius S (eds.), 1993, Lectins and Glycobiology, at pp. 158-174 In an alternative embodiment, the detection reagent is an antibody (or other affinity reagent), e.g., an antibody that specifically (e.g. immunospecifically) detects other post-translational modifications, such as-an-antibody that-immunospecifically binds to phosphorylated amino acids. Examples of such antibodies include those that bind to phosphotyrosine (BD Transduction Laboratories, catalog nos.: P11230-050/P11230-150; P11120; P38820; P39020), those that bind to phosphoserine (Zymed Laboratories Inc., South San Francisco, CA, catalog no. 61-8100) and those that bind to phosphothreonine (Zymed Laboratories Inc., South San Francisco, CA, catalogue nos. 71-8200, 13-9200).

If desired, a gene encoding Integrin beta 4, a related gene, or related nucleic acid sequences or subsequences, including complementary sequences, can also be used in hybridization assays. A nucleotide encoding Integrin beta 4, or subsequences thereof comprising at least 8 nucleotides, preferably at least 12 nucleotides, and most preferably at least 15 nucleotides can be used as a hybridization probe. Hybridization assays can be used for detection, prognosis, diagnosis, or monitoring of conditions, disorders, or disease states, associated with aberrant expression of the gene encoding Integrin beta 4, or for differential diagnosis of subjects with signs or symptoms suggestive of breast cancer, colorectal cancer, gastric cancer, hepatocellular carcinoma, lung cancer or pancreatic cancer. In particular, such a hybridization assay can be carried out by a method comprising contacting a subject's sample containing nucleic acid with a nucleic acid probe capable of hybridizing to a DNA or RNA that encodes Integrin beta 4, under conditions such that hybridization can occur, and detecting or measuring any resulting hybridization.

Hence nucleic acid encoding Integrin beta 4 (e.g. DNA or more suitably RNA) may be detected, for example, using a hybridizing agent capable of hybridizing to nucleic acid encoding Integrin beta 4.

One such exemplary method comprises:
(a) contacting one or more oligonucleotide probes comprising 10 or more consecutive nucleotides complementary to a nucleotide sequence encoding Integrin beta 4, with an RNA obtained from a biological sample from the subject or with cDNA copied from the RNA, wherein said contacting occurs under conditions that permit hybridization of the probe to the nucleotide sequence if present;
(b) detecting hybridization, if any, between the probe and the nucleotide sequence; and
(c) comparing the hybridization, if any, detected in step (b) with the hybridization detected in a control sample, or with a previously determined reference range.

Also disclosed are diagnostic kits, comprising an anti-Integrin beta 4 antibody (or other affinity reagent). In addition, such a kit may optionally comprise one or more of the following: (1) instructions for using the anti-Integrin beta 4 affinity reagent for diagnosis, prognosis, therapeutic monitoring or any combination of these applications; (2) a labeled binding partner to the affinity reagent; (3) a solid phase (such as a reagent strip) upon which the anti-Integrin beta 4 affinity, reagent is immobilized; and (4) a label or insert indicating regulatory approval for diagnostic, prognostic or therapeutic use or any combination thereof. If no labeled binding partner to the affinity reagent is provided, the anti-Integrin beta 4 affinity reagent itself can be labeled with a detectable marker, *e.g.,* a chemiluminescent, enzymatic, fluorescent, or radioactive moiety.

### Production of Protein of the Invention and Corresponding Nucleic Acid

Integrin beta 4 may, for example, be provided in isolated form, such as where the Integrin beta 4 polypeptide has been purified at least to some extent. Integrin beta 4 polypeptide may be provided in substantially pure form, that is to say free, to a substantial extent, from other proteins. Integrin beta 4 polypeptide can also be produced using recombinant methods, synthetically produced or produced by a combination of these methods. Integrin beta 4 can be easily prepared by any method known by persons skilled in the art, which involves producing an expression vector containing a DNA or a gene containing a DNA , culturing a transformant transformed using the expression vector, generating and accumulating a polypeptide of the present invention or a recombinant protein containing the polypeptide, and then collecting the resultant.

Recombinant Integrin beta 4 polypeptide may be prepared by processes well known in the art from genetically engineered host cells comprising expression systems. Expression systems may be used which compromise a Integrin beta 4 polypeptide or nucleic acid, to host cells which are genetically engineered with such expression systems and to the production of Integrin beta 4 polypeptide by recombinant techniques. For recombinant Integrin beta 4 polypeptide production, host cells can be genetically engineered to incorporate expression systems or portions thereof for nucleic acids. Such incorporation can be performed using methods well known in the art, such as, calcium phosphate transfection, DEAD-dextran mediated transfection, transvection, microinjection, cationic lipid-mediated transfection, electroporation, transduction, scrape loading, ballistic introduction or infection (see e.g. Davis-et al., Basic Methods in Molecular Biology, 1986-and Sambrook et al. , Molecular Cloning: A Laboratory Manual, 2nd Ed., Cold Spring Harbour laboratory Press, Cold Spring Harbour, NY, 1989).

As host cells, for example, bacteria of the genus Escherichia, Streptococci, Staphylococci, Streptomyces, bacteria of the genus Bacillus, yeast, Aspergillus cells, insect cells, insects, and animal cells are used. Specific examples of bacteria of the genus Escherichia, which are used herein, include Escherichia coli K12 and DH1 (Proc. Natl. Acad. Sci. U.S.A., Vol. 60, 160 (1968)), JM103 (Nucleic Acids Research, Vol. 9, 309 (1981)), JA221 (Journal of Molecular Biology, Vol. 120, 517 (1978)), and HB101 (Journal of Molecular Biology, Vol. 41, 459 (1969)). As bacteria of the genus Bacillus, for example, Bacillus subtilis MI114 (Gene, Vol. 24, 255 (1983)) and 207-21 (Journal of Biochemistry, Vol. 95, 87 (1984)) are used. As yeast, for example, Saccaromyces cerevisiae AH22, AH22R-, NA87-11A, DKD-5D, and 20B-12, Schizosaccaromyces pombe NCYC1913 and NCYC2036, and Pichia pastoris are used. As insect cells, for example, Drosophila S2 and Spodoptera Sf9 cells are used. As animal cells, for example, COS-7 and Vero monkey cells, CHO Chinese hamster cells (hereinafter abbreviated as CHO cells), dhfr-gene-deficient CHO cells, mouse L cells, mouse AtT-20 cells, mouse myeloma cells, rat GH3 cells, human FL cells, COS, HeLa, C127,3T3, HEK 293, BHK and Bowes melanoma cells are used.

Cell-free translation systems can also be employed to produce recombinant polypeptides (e.g. rabbit reticulocyte lysate, wheat germ lysate, SP6/T7 in vitro T&T and RTS 100 E. Coli HY transcription and translation kits from Roche Diagnostics Ltd., Lewes, UK and the TNT Quick coupled Transcription/Translation System from Promega UK, Southampton, UK).

The expression vector can be produced according to a method known in the art. For example, the vector can be produced by (1) excising a DNA fragment containing a DNA of the present invention or a gene containing a DNA of the present invention and (2) ligating the DNA fragment downstream of the promoter in an appropriate expression vector. A wide variety of expression systems can be used, such as and without limitation chromosomal episomal and virus derived systems, e.g. plasmids derived from Escherichia coli (e.g. pBR322, pBR325, pUC18, and pUC118), plasmids derived from Bacillus subtilis (e.g. pUB110, pTP5, and pC194), from bacteriophage, from transposons, from yeast episomes (e.g. pSH19 and pSH15), from insertion elements, from yeast chromosomal elements, from viruses such as baculoviruses, papova viruses such as SV40, vaccinia viruses, adenoviruses, fowl pox viruses, pseudorabies viruses and retroviruses, and vectors derived from combinations thereof, such as those derived from plasmid and bacteriophage (such as [lambda] phage) genetic elements, such as cosmids and phagemids. The expression systems may contain control regions that regulate as well as engender expression. Promoters to be used in the present invention may be any promoters as long as they are appropriate for hosts to be used for gene expression. For example, when a host is Escherichia coli, a trp promoter, a lac promoter, a recA promoter, a pL promoter, an Ipp promoter, and the like are preferred. When a host is Bacillus subtilis, an SPO1 promoter, an SPO2 promoter, a penP promoter, and the like are preferred. When a host is yeast, a PHO5 promoter, a PGK promoter, a GAP promoter, an ADH promoter, and the like are preferred. When an animal cell is used as a host, examples of promoters for use in this case include an SRa promoter, an SV40 promoter, an LTR promoter, a CMV promoter, and an HSV-TK promoter. Generally, any system or vector that is able to maintain, propagate or express a nucleic acid to produce a polypeptide in a host may be used.

The appropriate nucleic acid sequence may be inserted into an expression system by any variety of well known and routine techniques, such as those set forth in Sambrook et al., supra. Appropriate secretion signals may be incorporated into the Integrin beta 4 polypeptide to allow secretion of the translated protein into the lumen of the endoplasmic reticulum, the periplasmic space or the extracellular environment. These signals may be endogenous to the Integrin beta 4 polypeptide or they may be heterologous signals. Transformation of the host cells can be carried out according to methods known in the art. For example, the following documents can be referred to: Proc. Natl. Acad. Sci. U.S.A., Vol. 69, 2110 (1972); Gene, Vol. 17, 107 (1982); Molecular & General Genetics, Vol. 168, 111 (1979); Methods in Enzymology, Vol. 194, 182-187 (1991); Proc. Natl. Acad. Sci. U.S.A.), Vol. 75, 1929 (1978); Cell Technology, separate volume 8, New Cell Technology, Experimental Protocol. 263-267 (1995) (issued by Shujunsha); and Virology, Vol. 52, 456 (1973). The thus obtained transformant transformed with an expression vector containing a DNA or a gene containing a DNA can be cultured according to a method known in the art. For example, when hosts are bacteria of the genus Escherichia, the bacteria are generally cultured at approximately 15°C to 43°C for approximately 3 to 24 hours. If necessary, aeration or agitation can also be added. When hosts are bacteria of the genus Bacillus, the bacteria are generally cultured at approximately 30°C to 40°C for approximately 6 to 24 hours. If necessary, aeration or agitation can also be added. When transformants whose hosts are yeast are cultured, culture is generally carried out at approximately 20°C to 35°C for approximately 24 to 72 hours using media with pH adjusted to be approximately 5 to 8. If necessary, aeration or agitation can also be added. When transformants whose hosts are animal cells are cultured, the cells are generally cultured at approximately 30°C to 40°C for approximately 15 to 60 hours using media with the pH adjusted to be approximately 6 to 8. If necessary, aeration or agitation can also be added.

If a Integrin beta 4 polypeptide is to be expressed for use in cell-based screening assays, it is preferred that the polypeptide be produced at the cell surface. In this event, the cells may be harvested prior to use in the screening assay. If the Integrin beta 4 polypeptide is secreted into the medium, the medium can be recovered in order to isolate said polypeptide. If produced intracellularly, the cells must first be lysed before the Integrin beta 4 polypeptide is recovered.

Integrin beta 4 polypeptide can be recovered and purified from recombinant cell cultures or from other biological sources by well known methods including, ammonium sulphate or ethanol precipitation, acid extraction, anion or cation exchange chromatography, phosphocellulose chromatography, affinity chromatography, hydrophobic interaction chromatography, hydroxylapatite chromatography, molecular sieving chromatography, centrifugation methods, electrophoresis methods and lectin chromatography. In one embodiment, a combination of these methods is used. In another embodiment, high performance liquid chromatography is used. In a further embodiment, an antibody which specifically binds to a Integrin beta 4 polypeptide can be used to deplete a sample comprising a Integrin beta 4 polypeptide of said polypeptide or to purify said polypeptide.

To separate and purify a polypeptide or a protein from the culture products, for example, after culture, microbial bodies or cells are collected by a known method, they are suspended in an appropriate buffer, the microbial bodies or the cells are disrupted by, for example, ultrasonic waves, lysozymes, and/or freeze-thawing, the resultant is then subjected to centrifugation or filtration, and then a crude extract of the protein can be obtained. The buffer may also contain a protein denaturation agent such as urea or guanidine hydrochloride or a surfactant such as Triton X-100(TM). When the protein is secreted in a culture solution, microbial bodies or cells and a supernatant are separated by a known method after the completion of culture and then the supernatant is collected. The protein contained in the thus obtained culture supernatant or the extract can be purified by an appropriate combination of known separation and purification methods. The thus obtained polypeptide (protein) of the present invention can be converted into a salt by a known method or a method according thereto. Conversely, when the polypeptide (protein) of the present invention is obtained in the form of a salt, it can be converted into a free protein or peptide or another salt by a known method or a method according thereto. Moreover, an appropriate protein modification enzyme such as trypsin or chymotrypsin is caused to act on a protein produced by a recombinant before or after purification, so that modification can be arbitrarily added or a polypeptide can be partially removed. The presence of a polypeptide (protein) of the present invention or a salt thereof can be measured by various binding assays, enzyme immunoassays using specific antibodies, and the like.

Techniques well known in the art may be used for refolding to regenerate native or active conformations of the Integrin beta 4 polypeptide when the polypeptide has been denatured during isolation and or purification. In the context of the present invention, Integrin beta 4 polypeptide can be obtained from a biological sample from any source, such as and without limitation, a blood sample or tissue sample, e.g. a breast, colorectal, gastric epithelium, liver, lung or pancreatic tissue sample.

Integrin beta 4 polypeptide may be in the form of a "mature protein" or may be part of a larger protein such as a fusion protein. It is often advantageous to include an additional amino acid sequence which contains secretory or leader sequences, a pre-, pro- or prepro- protein sequence, or a sequence which aids in purification such as an affinity tag, for example, but without limitation, multiple histidine residues, a FLAG tag, HA tag or myc tag.

Integrin beta 4 may, for example, be fused with a heterologous fusion partner such as the surface protein, known as protein D from Haemophilus Influenza B, a non-structural protein from influenzae virus such as NS 1, the S antigen from Hepatitis B or a protein known as LYTA such as the C terminal thereof.

An additional sequence that may provide stability during recombinant production may also be used. Such sequences may be optionally removed as required by incorporating a cleavable sequence as an additional sequence or part thereof. Thus, a Integrin beta 4 polypeptide may be fused to other moieties including other polypeptides or proteins (for example, glutathione S-transferase and protein A). Such a fusion protein can be cleaved using an appropriate protease, and then separated into each protein. Such additional sequences and affinity tags are well known in the art. In addition to the above, features known in the art, such as an enhancer, a splicing signal, a polyA addition signal, a selection marker, and an SV40 replication origin can be added to an expression vector, if desired.

### Production of Affinity Reagents to Integrin beta 4

According to those in the art, there are three main types of immunoaffinity reagent - monoclonal antibodies, phage display antibodies and smaller antibody-derived molecules such as Affibodies, Domain Antibodies (dAbs), Nanobodies, Unibodies, DARPins, Anticalins, Duocalins, Avimers or Versabodies. In general in applications according to the present invention where the use of antibodies is stated, other affinity reagents (e.g. Affibodies, Domain Antibodies, Nanobodies, Unibodies, DARPins, Anticalins, Duocalins, Avimers or Versabodies) may be employed. Such substances may be said to be capable of immunospecific binding to Integrin beta 4. Where appropriate the term "affinity agent" shall be construed to embrace immunoaffinity reagents and other substances capable of specific binding to Integrin beta 4 including but not limited to ligands, lectins, streptavidins, antibody mimetics and synthetic binding agents.

### Production of Antibodies to Integrin beta 4

Integrin beta 4, a Integrin beta 4 analogue, a Integrin beta 4-related protein or a fragment or derivative of any of the foregoing may be used as an immunogen to generate antibodies which immunospecifically bind such an immunogen. Such immunogens can be isolated by any convenient means, including the methods described above. The term "antibody" as used herein refers to a peptide or polypeptide derived from, modelled after or substantially encoded by an immunoglobulin gene or immunoglobulin genes, or fragments thereof, capable of specifically binding an antigen or epitope. See, e.g. Fundamental Immunology, 3rd Edition, W.E. Paul, ed., Raven Press, N.Y. (1993); Wilson (1994) J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989)-Nature 341 544546, which consists of a VH domain; and (vi) an isolated complementarity determining region (CDR). Single chain antibodies are also included by reference in the term "antibody." Antibodies of the invention include, but are not limited to polyclonal, monoclonal, bispecific, humanized or chimeric antibodies, single chain antibodies, Fab fragments and F(ab')₂ fragments, fragments produced by a Fab expression library, anti-idiotypic (anti-Id) antibodies, and epitope-binding fragments of any of the above. The immunoglobulin molecules of the invention can be of any class (e.g., IgG, IgE, IgM, IgD and IgA) or subclass of immunoglobulin molecule.

The term "specifically binds" (or "immunospecifically binds") is not intended to indicate that an antibody binds exclusively to its intended target. Rather, an antibody "specifically binds" if its affinity for its intended target is about 5-fold greater when compared to its affinity for a non-target molecule. Suitably there is no significant cross-reaction or cross-binding with undesired substances, especially naturally occurring proteins or tissues of a healthy person or animal. The affinity of the antibody will, for example, be at least about 5 fold, such as 10 fold, such as 25-fold, especially 50-fold, and particularly 100-fold or more, greater for a target molecule than its affinity for a non-target molecule. In some embodiments, specific binding between an antibody or other binding agent and an antigen means a binding affinity of at least 10⁶ M⁻¹. Antibodies may, for example, bind with affinities of at least about 10⁷ M⁻¹, such as between about 10⁸ M⁻¹ to about 10⁹ M⁻¹, about 10⁹ M⁻¹ to about 10¹⁰ M⁻¹, or about 10¹⁰ M⁻¹ to about 10¹¹ M⁻¹.

Affinity is calculated as K_{d} =k_{off} /kₒₙ (k_{off} is the dissociation rate constant, kₒₙ is the association rate constant and K_{d} is the equilibrium constant. Affinity can be determined at equilibrium by measuring the fraction bound (r) of labelled ligand at various concentrations (c). The data are graphed using the Scatchard equation: r/c = K(n-r):
where
r = moles of bound ligand/mole of receptor at equilibrium;
c= free ligand concentration at equilibrium;
K = equilibrium association constant; and
n = number of ligand binding sites per receptor molecule
By graphical analysis, r/c is plotted on the Y-axis versus r on the X-axis thus producing a Scatchard plot. The affmity is the negative slope of the line. k_{off} can be determined by competing bound labelled ligand with unlabeled excess ligand (see, e.g., U.S. Pat No. 6,316,409). The affinity of a targeting agent for its target molecule is, for example, at least about 1 x 10⁻⁶ moles/litre, such as at least about 1 x 10⁻⁷ moles/litre, such as at least about 1 x 10⁻⁸ moles/litre, especially at least about 1 x 10⁻⁹ moles/litre, and particularly at least about 1 x 10⁻¹⁰ moles/litre. Antibody affinity measurement by Scatchard analysis is well known in the *art. See, e.g.,* van Erp et al., J. Immunoassay 12: 425-43, 1991; Nelson and Griswold, Comput. Methods Programs Biomed. 27: 65-8, 1988.

In one embodiment, antibodies that recognize gene products of genes encoding Integrin beta 4 are publicly available. In another embodiment, methods known to those skilled in the art are used to produce antibodies that recognize Integrin beta 4, a Integrin beta 4 analogue, a Integrin beta 4-related polypeptide, or a fragment or derivative of any of the foregoing. One skilled in the art will recognize that many procedures are available for the production of antibodies, for example, as described in Antibodies, A Laboratory Manual, Ed Harlow and David Lane, Cold Spring Harbor Laboratory (1988), Cold Spring Harbor, N.Y. One skilled in the art will also appreciate that binding fragments or Fab fragments which mimic antibodies can also be prepared from genetic information by various procedures (Antibody Engineering: A Practical Approach (Borrebaeck, C., ed.), 1995, Oxford University Press, Oxford; J. Immunol. 149, 3914-3920 (1992)).

In one embodiment of the invention, antibodies to a specific domain of Integrin beta 4 are produced. In a specific embodiment, hydrophilic fragments of Integrin beta 4 are used as immunogens for antibody production.

In the production of antibodies, screening for the desired antibody can be -accomplished by techniques known in the art, *e.g.* ELISA (enzyme-linked immunosorbent assay). For example, to select antibodies which recognize a specific domain of Integrin beta 4, one may assay generated hybridomas for a product which binds to a Integrin beta 4 fragment containing such domain. For selection of an antibody that specifically binds a first Integrin beta 4 homolog but which does not specifically bind to (or binds less avidly to) a second Integrin beta 4 homolog, one can select on the basis of positive binding to the first Integrin beta 4 homolog and a lack of binding to (or reduced binding to) the second Integrin beta 4 homolog. Similarly, for selection of an antibody that specifically binds Integrin beta 4 but which does not specifically bind to (or binds less avidly to) a different isoform of the same protein (such as a different glycoform having the same core peptide as Integrin beta 4), one can select on the basis of positive binding to Integrin beta 4 and a lack of binding to (or reduced binding to) the different isoform (e.g., a different glycoform). Thus, the present invention provides an antibody (such as a monoclonal antibody) that binds with greater affinity (for example at least 2-fold, such as at least 5-fold, particularly at least 10-fold greater affinity) to Integrin beta 4 than to a different isoform or isoforms (e.g., glycoforms) of Integrin beta 4.

Polyclonal antibodies which may be used in the methods of the invention are heterogeneous populations of antibody molecules derived from the sera of immunized animals. Unfractionated immune serum can also be used. Various procedures known in the art may be used for the production of polyclonal antibodies to Integrin beta 4, a fragment of Integrin beta 4, a Integrin beta 4-related polypeptide, or a fragment of a Integrin beta 4-related polypeptide. For example, one way is to purify polypeptides of interest or to synthesize the polypeptides of interest using, e.g., solid phase peptide synthesis methods well known in the art. See, e.g., Guide to Protein Purification, Murray P. Deutcher, ed., Meth. Enzymol. Vol 182 (1990); Solid Phase Peptide Synthesis, Greg B. Fields ed., Meth. Enzymol. Vol 289 (1997); Kiso et al., Chem. Pharm. Bull. (Tokyo) 38: 1192-99, 1990; Mostafavi et al., Biomed. Pept. Proteins Nucleic Acids 1: 255-60, 1995; Fujiwara et al., Chem. Pharm. Bull. (Tokyo) 44: 1326-31, 1996. The selected polypeptides may then be used to immunize by injection various host animals, including but not limited to rabbits, mice, rats, etc., to generate polyclonal or monoclonal antibodies. The Preferred Technology described herein provides isolated Integrin beta 4 suitable for such immunization. If Integrin beta 4 is purified by gel electrophoresis, Integrin beta 4 can be used for immunization with or without prior extraction from the polyacrylamide gel. Various adjuvants (i.e. immunostimulants) may be used to enhance the immunological response, depending on the host species, including, but not limited to, complete or incomplete Freund's adjuvant, a mineral gel such as aluminium hydroxide, surface active substance such as lysolecithin, pluronic polyol, a polyanion, a peptide, an oil emulsion, keyhole limpet hemocyanin, dinitrophenol and an adjuvant such as BCG (bacille Calmette-Guerin) or corynebacterium parvum. Additional adjuvants are also well known in the art.

For preparation of monoclonal antibodies (mAbs) directed toward Integrin beta 4, a fragment of Integrin beta 4, a Integrin beta 4-related polypeptide, or a fragment of a Integrin beta 4-related polypeptide, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in Monoclonal Antibodies and Cancer Therapy, Alan R. Liss, Inc., pp. 77-96). Such antibodies may be of any immunoglobulin class including IgG, IgM, IgE, IgA, IgD and any subclass thereof. The hybridoma producing the mAbs of the invention may be cultivated *in vitro* or *in vivo.* In an additional embodiment of the invention, monoclonal antibodies can be produced in germ-free animals utilizing known technology (PCT/US90/02545 ).

The monoclonal antibodies include but are not limited to human monoclonal antibodies and chimeric monoclonal antibodies (e.g., human-mouse chimeras). A chimeric antibody is a molecule in which different portions are derived from different animal species, such as those having a human immunoglobulin constant region and a variable region derived from a murine mAb. (See, *e.g.,* Cabilly et al., U.S. Patent No. 4,816,567; and Boss et al., U.S. Patent No. 4,816397). Humanized antibodies are antibody molecules from non-human species having one or more complementarity determining regions (CDRs) from the non-human species and a framework region from a human immunoglobulin molecule. (See, *e.g.,* Queen, U.S. Patent No. 5,585,089).

Chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Canc. Res. 47:999-1005; Wood et al., 1985, Nature 314:446-449; and Shaw et al., 1988, J. Natl. Cancer Inst. 80:1553-1559; Morrison, 1985, Science 229:1202-1207; Oi et al., 1986, Bio/Techniques 4:214; U.S. Patent 5,225,539; Jones et al., 1986, Nature 321:552-525; Verhoeyan et al. (1988) Science 239:1534; and Beidler et al., 1988, J. Immunol. 141:4053-4060.

Completely human antibodies are particularly desirable for therapeutic treatment of human subjects. Such antibodies can be produced using transgenic mice which are incapable of expressing endogenous immunoglobulin heavy and light chain genes, but which can express human heavy and light chain genes. The transgenic mice are immunized in the normal fashion with a selected antigen, e.g., all or a portion of Integrin beta 4. Monoclonal antibodies directed against the antigen can be obtained using conventional hybridoma technology. The human immunoglobulin transgenes harboured by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, *see, e.g.,* U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA) and Genpharm (San Jose, CA) can be engaged to provide human antibodies directed against a selected antigen using technology similar to that described above.

Completely human antibodies which recognize a selected epitope can be generated using a technique referred to as "guided selection." In this approach a selected non-human monoclonal antibody, *e.g.,* a mouse antibody, is used to guide the selection of a completely human antibody recognizing the same epitope. (Jespers et al. (1994) Bio/technology 12:899-903).

The antibodies can also be generated by the use of phage display technology to produce and screen libraries of polypeptides for binding to a selected target. See, e.g., Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. See, e.g., U.S. Patent No. 6,057,098, including all tables, figures, and claims. In particular, such phage can be utilized to display antigen binding domains expressed from a repertoire or combinatorial antibody library (*e.g.,* human or murine). Phage expressing an antigen binding domain that binds the antigen of interest can be selected or identified with antigen, *e.g.,* using labelled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Phage display methods that can be used to make the antibodies of the present invention include those disclosed in Brinkman et aL, J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT Application No. PCT/GB91/01134; PCT Publications WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g., as described in detail below. For example, techniques to recombinantly produce Fab, Fab' and F(ab')₂ fragments can also be employed using methods known in the art such as those disclosed in PCT publication WO 92/22324; Mullinax et al., BioTechniques 12(6):864-869 (1992); and Sawai et al., AJRI 34:26-34 (1995); and Better et al., Science 240:1041-1043 (1988).

Examples of techniques which can be used to produce single-chain Fvs and antibodies include those described in U.S. Patents 4,946,778 and 5,258,498; Huston et al., Methods in Enzymology 203:46-88 (1991); Shu et al., PNAS 90:7995-7999 (1993); and Skerra et al., Science 240:1038-1040 (1988).

The invention further provides for the use of bispecific antibodies, which can be made by methods known in the art. Traditional production of full length bispecific antibodies is based on the coexpression of two immunoglobulin heavy chain-light chain pairs, where the two chains have different specificities (Milstein et al., 1983, Nature 305:537-539). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, published 13 May 1993, and in Traunecker et al., 1991, EMBO J. 10:3655-3659.

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690 published March 3, 1994. For further details for generating bispecific antibodies see, for example, Suresh et al., Methods in Enzymology, 1986, 121:210.

Functionally active fragments, derivatives or analogues of the anti-Integrin beta 4 immunoglobulin molecules may be used. Functionally active means that the fragment, derivative or analogue is able to elicit anti-anti-idiotype antibodies (*i.e.,* tertiary antibodies) that recognize the same antigen that is recognized by the antibody from which the fragment, derivative or analogue is derived. Specifically, in a particular embodiment the antigenicity of the idiotype of the immunoglobulin molecule may be enhanced by deletion of framework and CDR sequences that are C-terminal to the CDR sequence that specifically recognizes the antigen. To determine which CDR sequences bind the antigen, synthetic peptides containing the CDR sequences can be used in binding assays with the antigen by any binding assay method known in the art.

The antibody fragments include F(ab')₂ fragments and Fab fragments. Antibody fragments which recognize specific epitopes may be generated by known techniques. F(ab')₂ fragments consist of the variable region, the light chain constant region and the CH1 domain of the heavy chain and are generated by pepsin digestion of the antibody molecule. Fab fragments are generated by reducing the disulfide bridges of the F(ab')₂ fragments. Heavy chain and light chain dimers of the antibodies may be used or any minimal fragment thereof such as Fvs or single chain antibodies (SCAs) (*e.g.,* as described in U.S. Patent 4,946,778; Bird, 1988, Science 242:423-42; Huston et al., 1988, Proc. Natl. Acad. Sci. USA 85:5879-5883; and Ward et al., 1989, Nature 334:544-54), or any other molecule with the same specificity as the antibody of the invention. Single chain antibodies are formed by linking the heavy and light chain fragments of the Fv region via an amino acid bridge, resulting in a single chain polypeptide. Techniques for the assembly of functional Fv fragments in *E. coli* may be used (Skerra et al., 1988, Science 242:1038-1041).

Fusion proteins of the immunoglobulins may be used (or functionally active fragments thereof), for example in which the immunoglobulin is fused via a covalent bond (*e.g.,* a peptide bond), at either the N-terminus or the C-terminus to an amino acid sequence of another protein (or portion thereof, preferably at least 10, 20 or 50 amino acid portion of the protein) that is not the immunoglobulin. Preferably the immunoglobulin, or fragment thereof, is covalently linked to the other protein at the N-terminus of the constant domain. As stated above, such fusion proteins may facilitate purification, increase half-life *in vivo,* and enhance the delivery of an antigen across an epithelial barrier to the immune system.

The immunoglobulins include analogues and derivatives that are modified, i.e., by the covalent attachment of any type of molecule as long as such covalent attachment does not impair immunospecific binding. For example, but not by way of limitation, the derivatives and analogues of the immunoglobulins include those that have been further modified, e.g., by glycosylation, acetylation, pegylation, phosphylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, etc. Additionally, the analogue or derivative may contain one or more non-classical amino acids.

The foregoing antibodies can be used in methods known in the art relating to the localization and activity of Integrin beta 4, e.g., for imaging this protein, measuring levels thereof in appropriate physiological samples, in diagnostic methods, etc.

### Production of Affibodies to Integrin beta 4

Affibody molecules represent a new class of affinity proteins based on a 58-amino acid residue protein domain, derived from one of the IgG-binding domains of staphylococcal protein A. This three helix bundle domain has been used as a scaffold for the construction of combinatorial phagemid libraries, from which Affibody variants that target the desired molecules can be selected using phage display technology (Nord K, Gunneriusson E, Ringdahl J, Stahl S, Uhlen M, Nygren PA, Binding proteins selected from combinatorial libraries of an α-helical bacterial receptor domain, Nat Biotechnol 1997;15:772-7. Ronmark J, Gronlund H, Uhlen M, Nygren PA, Human immunoglobulin A (IgA)-specific ligands from combinatorial engineering of protein A, Eur J Biochem 2002;269:2647-55.). The simple, robust structure of Affibody molecules in combination with their low molecular weight (6 kDa), make them suitable for a wide variety of applications, for instance, as detection reagents (Ronmark J, Hansson M, Nguyen T, et al, Construction and characterization of affibody-Fc chimeras produced in Escherichia coli, J Immunol Methods 2002;261:199-211) and to inhibit receptor interactions (Sandstorm K, Xu Z, Forsberg G, Nygren PA, Inhibition of the CD28-CD80 co-stimulation signal by a CD28-binding Affibody ligand developed by combinatorial protein engineering, Protein Eng 2003;16:691-7). Further details of Affibodies and methods of production thereof may be obtained by reference to US Patent No 5831012.

Labelled Affibodies may also be useful in imaging applications for determining abundance of Isoforms.

### Production of Domain Antibodies to Integrin beta 4

References to antibodies herein embrace references to Domain Antibodies. Domain Antibodies (dAbs) are the smallest functional binding units of antibodies, corresponding to the variable regions of either the heavy (V_{H}) or light (V_{L}) chains of human antibodies. Domain Antibodies have a molecular weight of approximately 13 kDa. Domantis has developed a series of large and highly functional libraries of fully human V_{H} and V_{L} dAbs (more than ten billion different sequences in each library), and uses these libraries to select dAbs that are specific to therapeutic targets. In contrast to many conventional antibodies, Domain Antibodies are well expressed in bacterial, yeast, and mammalian cell systems. Further details of domain antibodies and methods of production thereof may be obtained by reference to US Patent 6,291,158; 6,582,915; 6,593,081; 6,172,197; 6,696,245; US Serial No. 2004/0110941; European patent application No. 1433846 and European Patents 0368684 & 0616640; WO05/035572, WO04/101790, WO04/081026, WO04/058821, WO04/003019 and WO03/002609.

### Production of Nanobodies to Integrin beta 4

Nanobodies are antibody-derived therapeutic proteins that contain the unique structural and functional properties of naturally-occurring heavy-chain antibodies. These heavy-chain antibodies contain a single variable domain (VHH) and two constant domains (C_{H}2 and C_{H}3). Importantly, the cloned and isolated VHH domain is a perfectly stable polypeptide harbouring the full antigen-binding capacity of the original heavy-chain antibody. Nanobodies have a high homology with the VH domains of human antibodies and can be further humanised without any loss of activity. Importantly, Nanobodies have a low immunogenic potential, which has been confirmed in primate studies with Nanobody lead compounds.

Nanobodies combine the advantages of conventional antibodies with important features of small molecule drugs. Like conventional antibodies, Nanobodies show high target specificity, high affinity for their target and low inherent toxicity. However, like small molecule drugs they can inhibit enzymes and readily access receptor clefts. Furthermore, Nanobodies are extremely stable, can be administered by means other than injection (see e.g. WO 04/041867) and are easy to manufacture. Other advantages of Nanobodies include recognising uncommon or hidden epitopes as a result of their small size, binding into cavities or active sites of protein targets with high affinity and selectivity due to their unique 3-dimensional, drug format flexibility, tailoring of half-life and ease and speed of drug discovery.

Nanobodies are encoded by single genes and are efficiently produced in almost all prokaryotic and eukaryotic hosts e.g. *E. coli* (see e.g. US 6,765,087) moulds (for example *Aspergillus* or *Trichoderma)* and yeast (for example *Saccharomyces, Kluyveromyces, Hansenula* or *Pichia*) (see e.g. US 6,838,254). The production process is scalable and multi-kilogram quantities of Nanobodies have been produced. Because Nanobodies exhibit a superior stability compared with conventional antibodies, they can be formulated as a long shelf-life, ready-to-use solution.

The Nanoclone method (see e.g. WO 06/079372) is a proprietary method for generating Nanobodies against a desired target, based on automated high-throughput selection of B-cells.

### Production of Unibodies to Integrin beta 4

UniBodies are another antibody fragment technology; however this one is based upon the removal of the hinge region of IgG4 antibodies. The deletion of the hinge region results in a molecule that is essentially half the size of traditional IgG4 antibodies and has a univalent binding region rather than the bivalent binding region of IgG4 antibodies. It is also well known that IgG4 antibodies are inert and thus do not interact with the immune system, which may be advantageous for the treatment of diseases where an immune response is not desired, and this advantage is passed onto UniBodies. For example, UniBodies may function to inhibit or silence, but not kill, the cells to which they are bound. Additionally, UniBody binding to cancer cells do not stimulate them to proliferate. Furthermore, because UniBodies are about half the size of traditional IgG4 antibodies, they may show better distribution over larger solid tumours with potentially advantageous efficacy. UniBodies are cleared from the body at a similar rate to whole IgG4 antibodies and are able to bind with a similar affinity for their antigens as whole antibodies. Further details of UniBodies may be obtained by reference to patent WO2007/059782.

### Production of DARPins to Integrin beta 4

DARPins (Designed Ankyrin Repeat Proteins) are one example of an antibody mimetic DRP (Designed Repeat Protein) technology that has been developed to exploit the binding abilities ofnon-antibody polypeptides. Repeat proteins such as ankyrin or leucine-rich repeat proteins, are ubiquitous binding molecules, which occur, unlike antibodies, intra- and extracellularly. Their unique modular architecture features repeating structural units (repeats), which stack together to form elongated repeat domains displaying variable and modular target-binding surfaces. Based on this modularity, combinatorial libraries of polypeptides with highly diversified binding specificities can be generated. This strategy includes the consensus design of self-compatible repeats displaying variable surface residues and their random assembly into repeat domains.

DARPins can be produced in bacterial expression systems at very high yields and they belong to the most stable proteins known. Highly specific, high-affinity DARPins to a broad range of target proteins, including human receptors, cytokines, kinases, human proteases, viruses and membrane proteins, have been selected. DARPins having affinities in the single-digit nanomolar to picomolar range can be obtained.

DARPins have been used in a wide range of applications, including ELISA, sandwich ELISA, flow cytometric analysis (FACS), immunohistochemistry (IHC), chip applications, affinity purification or Western blotting. DARPins also proved to be highly active in the intracellular compartment for example as intracellular marker proteins fused to green fluorescent protein (GFP). DARPins were further used to inhibit viral entry with IC50 in the pM range. DARPins are not only ideal to block protein-protein interactions, but also to inhibit enzymes. Proteases, kinases and transporters have been successfully inhibited, most often an allosteric inhibition mode. Very fast and specific enrichments on the tumour and very favourable tumour to blood ratios make DARPins well suited for in vivo diagnostics or therapeutic approaches.

Additional information regarding DARPins and other DRP technologies can be found in US Patent Application Publication No. 2004/0132028, and International Patent Application Publication No. WO 02/20565.

### Production of Anticalins to Integrin beta 4

Anticalins are an additional antibody mimetic technology, however in this case the binding specificity is derived from lipocalins, a family of low molecular weight proteins that are naturally and abundantly expressed in human tissues and body fluids. Lipocalins have evolved to perform a range of functions in vivo associated with the physiological transport and storage of chemically sensitive or insoluble compounds. Lipocalins have a robust intrinsic structure comprising a highly conserved ß-barrel which supports four loops at one terminus of the protein. These loops form the entrance to a binding pocket and conformational differences in this part of the molecule account for the variation in binding specificity between individual lipocalins.

While the overall structure of hypervariable loops supported by a conserved ß-sheet framework is reminiscent of immunoglobulins, lipocalins differ considerably from antibodies in terms of size, being composed of a single polypeptide chain of 160-180 amino acids which is marginally larger than a single immunoglobulin domain.

Lipocalins are cloned and their loops are subjected to engineering in order to create Anticalins. Libraries of structurally diverse Anticalins have been generated and Anticalin display allows the selection and screening of binding function, followed by the expression and production of soluble protein for further analysis in prokaryotic or eukaryotic systems. Studies have successfully demonstrated that Anticalins can be developed that are specific for virtually any human target protein; they can be isolated and binding affinities in the nanomolar or higher range can be obtained.

Anticalins can also be formatted as dual targeting proteins, so-called Duocalins. A Duocalin binds two separate therapeutic targets in one easily produced monomeric protein using standard manufacturing processes while retaining target specificity and affinity regardless of the structural orientation of its two binding domains.

Modulation of multiple targets through a single molecule is particularly advantageous in diseases known to involve more than a single causative factor. Moreover, bi- or multivalent binding formats such as Duocalins have significant potential in targeting cell surface molecules in disease, mediating agonistic effects on signal transduction pathways or inducing enhanced internalization effects via binding and clustering of cell surface receptors. Furthermore, the high intrinsic stability of Duocalins is comparable to monomeric Anticalins, offering flexible formulation and delivery potential for Duocalins.

Additional information regarding Anticalins can be found in US Patent No. 7,250,297 and International Patent Application Publication No. WO 99/16873.

### Production of Avimers to Integrin beta 4

Avimers are evolved from a large family of human extracellular receptor domains by in vitro exon shuffling and phage display, generating multidomain proteins with binding and inhibitory properties. Linking multiple independent binding domains has been shown to create avidity and results in improved affinity and specificity compared with conventional single-epitope binding proteins. Other potential advantages include simple and efficient production of multitarget-specific molecules in Escherichia coli, improved thermostability and resistance to proteases. Avimers with sub-nanomolar affinities have been obtained against a variety of targets.

Additional information regarding Avimers can be found in US Patent Application Publication Nos. 2006/0286603, 2006/0234299, 2006/0223114, 2006/0177831, 2006/0008844, 2005/0221384, 2005/0164301, 2005/0089932, 2005/0053973, 2005/0048512, 2004/0175756.

### Production of Versabodies to Integrin beta 4

Versabodies are small proteins of 3-5 kDa with > 15% cysteines, which form a high disulfide density scaffold, replacing the hydrophobic core that typical proteins have. The replacement of a large number of hydrophobic amino acids, comprising the hydrophobic core, with a small number of disulfides results in a protein that is smaller, more hydrophilic (less aggregation and non-specific binding), more resistant to proteases and heat, and has a lower density of T-cell epitopes, because the residues that contribute most to MHC presentation are hydrophobic. All four of these properties are well-known to affect immunogenicity, and together they are expected to cause a large decrease in immunogenicity.

The inspiration for Versabodies comes from the natural injectable biopharmaceuticals produced by leeches, snakes, spiders, scorpions, snails, and anemones, which are known to exhibit unexpectedly low immunogenicity. Starting with selected natural protein families, by design and by screening the size, hydrophobicity, proteolytic antigen processing, and epitope density are minimized to levels far below the average for natural injectable proteins.

Given the structure of Versabodies, these antibodymimetics offer a versatile format that includes multi-valency, multi-specificity, a diversity of half-life mechanisms, tissue targeting modules and the absence of the antibody Fc region. Furthermore, Versabodies are manufactured in E. coli at high yields, and because of their hydrophilicity and small size, Versabodies are highly soluble and can be formulated to high concentrations. Versabodies are exceptionally heat stable (they can be boiled) and offer extended shelf life.

Additional information regarding Versabodies can be found in US Patent Application Publication No. 2007/0191272.

### Expression of Affinity Reagents

### Expression of Antibodies

The antibodies used herein can be produced by any method known in the art for the synthesis of antibodies, in particular, by chemical synthesis or by recombinant expression, and are preferably produced by recombinant expression techniques.

Recombinant expression of antibodies, or fragments, derivatives or analogues thereof, requires construction of a nucleic acid that encodes the antibody. If the nucleotide sequence of the antibody is known, a nucleic acid encoding the antibody may be assembled from chemically synthesized oligonucleotides (e.g., as described in Kutmeier et al., 1994, BioTechniques 17:242), which, briefly, involves the synthesis of overlapping oligonucleotides containing portions of the sequence encoding antibody, annealing and ligation of those oligonucleotides, and then amplification of the ligated oligonucleotides by PCR

Alternatively, the nucleic acid encoding the antibody may be obtained by cloning the antibody. If a clone containing the nucleic acid encoding the particular antibody is not available, but the sequence of the antibody molecule is known, a nucleic acid encoding the antibody may be obtained from a suitable source (*e.g.,* an antibody cDNA library, or cDNA library generated from any tissue or cells expressing the antibody) by PCR amplification using synthetic primers hybridizable to the 3' and 5' ends of the sequence or by cloning using an oligonucleotide probe specific for the particular gene sequence.

If an antibody molecule that specifically recognizes a particular antigen is not available (or a source for a cDNA library for cloning a nucleic acid encoding such an antibody), antibodies specific for a particular antigen may be generated by any method known in the art, for example, by immunizing an animal, such as a rabbit, to generate polyclonal antibodies or, more preferably, by generating monoclonal antibodies. Alternatively, a clone encoding at least the Fab portion of the antibody may be obtained by screening Fab expression libraries (*e.g.,* as described in Huse et al., 1989, Science 246:1275-1281) for clones of Fab fragments that bind the specific antigen or by screening antibody libraries (See, *e.g.,* Clackson et al., 1991, Nature 352:624; Hane et al., 1997 Proc. Natl. Acad. Sci. USA 94:4937).

Once a nucleic acid encoding at least the variable domain of the antibody molecule is obtained, it may be introduced into a vector containing the nucleotide sequence encoding the constant region of the antibody molecule (see, *e.g.,* PCT Publication WO 86/05807; PCT Publication WO 89/01036; and U.S. Patent No. 5,122,464). Vectors containing the complete light or heavy chain for co-expression with the nucleic acid to allow the expression of a complete antibody molecule are also available. Then, the nucleic acid encoding the antibody can be used to introduce the nucleotide substitution(s) or deletion(s) necessary to substitute (or delete) the one or more variable region cysteine residues participating in an intrachain disulfide bond with an amino acid residue that does not contain a sulfhydyl group. Such modifications can be carried out by any method known in the art for the introduction of specific mutations or deletions in a nucleotide sequence, for example, but not limited to, chemical mutagenesis, in vitro site directed mutagenesis (Hutchinson et al., 1978, J. Biol. Chem. 253:6551), PCT based methods, etc.

In addition, techniques developed for the production of "chimeric antibodies" (Morrison et al., 1984, Proc. Natl. Acad. Sci. 81:851-855; Neuberger et al., 1984, Nature 312:604-608; Takeda et al., 1985, Nature 314:452-454) by splicing genes from a mouse antibody molecule of appropriate antigen specificity together with genes from a human antibody molecule of appropriate biological activity can be used. As described *supra,* a chimeric antibody is a molecule in which different portions are derived from different animal species, such as those having a variable region derived from a murine mAb and a human antibody constant region, *e.g.,* humanized antibodies.

Once a nucleic acid encoding an antibody molecule of the invention has been obtained, the vector for the production of the antibody molecule may be produced by recombinant DNA technology using techniques well known in the art. Thus, methods for preparing the protein of the invention by expressing nucleic acid containing the antibody molecule sequences are described herein. Methods which are well known to those skilled in the art can be used to construct expression vectors containing an antibody-molecule coding-sequences and appropriate transcriptional and translational control signals. These methods include, for example, *in vitro* recombinant DNA techniques, synthetic techniques, and *in vivo* genetic recombination. See, for example, the techniques described in Sambrook et al. (1990, Molecular Cloning, A Laboratory Manual, 2nd Ed., Cold Spring Harbor Laboratory, Cold Spring Harbor, NY) and Ausubel et al. (eds., 1998, Current Protocols in Molecular Biology, John Wiley & Sons, NY).

The expression vector is transferred to a host cell by conventional techniques and the transfected cells are then cultured by conventional techniques to produce an antibody of the invention.

The host cells used to express a recombinant antibody of the invention may be either bacterial cells such as *Escherichia coli,* or, preferably, eukaryotic cells, especially for the expression of whole recombinant antibody molecule. In particular, mammalian cells such as Chinese hamster ovary cells (CHO), in conjunction with a vector such as the major intermediate early gene promoter element from human cytomegalovirus are an effective expression system for antibodies (Foecking et al., 1986, Gene 45:101; Cockett et al., 1990, Bio/Technology 8:2).

A variety of host-expression vector systems may be utilized to express an antibody molecule of the invention. Such host-expression systems represent vehicles by which the coding sequences of interest may be produced and subsequently purified, but also represent cells which may, when transformed or transfected with the appropriate nucleotide coding sequences, express the antibody molecule of the invention *in situ.* These include but are not limited to microorganisms such as bacteria (*e.g., E. coli, B. subtilis*) transformed with recombinant bacteriophage DNA, plasmid DNA or cosmid DNA expression vectors containing antibody coding sequences; yeast *(e.g., Saccharomyces, Pichia*) transformed with recombinant yeast expression vectors containing antibody coding sequences; insect cell systems infected with recombinant virus expression vectors *(e.g.,* baculovirus) containing the antibody coding sequences; plant cell systems infected with recombinant virus expression vectors *(e.g.,* cauliflower mosaic virus, CaMV; tobacco mosaic virus, TMV) or transformed with recombinant plasmid expression vectors *(e.g.,* Ti plasmid) containing antibody coding sequences; or mammalian cell systems *(e.g.,* COS, CHO, BHK, 293, 3T3 cells) harbouring recombinant expression constructs containing promoters derived from the genome of mammalian cells *(e.g.,* metallothionein promoter) or from mammalian viruses *(e.g.,* the adenovirus late promoter; the vaccinia virus 7.5K promoter).

In bacterial systems, a number of expression vectors may be advantageously selected depending upon the use intended for the antibody molecule being expressed. For example, when a large quantity of such a protein is to be produced, for the generation of pharmaceutical compositions comprising an antibody molecule, vectors which direct the expression of high levels of fusion protein products that are readily purified may be desirable. Such vectors include, but are not limited, to the E. *coli* expression vector pUR278 (Ruther et al., 1983, EMBO J. 2:1791), in which the antibody coding sequence may be ligated individually into the vector in frame with the *lac* Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter). In mammalian host cells, a number of viral-based expression systems *(e.g.,* an adenovirus expression system) may be utilized.

As discussed above, a host cell strain may be chosen which modulates the expression of the inserted sequences, or modifies and processes the gene product in the specific fashion desired. Such modifications *(e.g.,* glycosylation) and processing *(e.g.,* cleavage) of protein products may be important for the function of the protein.

For long-term, high-yield production of recombinant antibodies, stable expression is preferred. For example, cell lines that stably express an antibody of interest can be produced by transfecting the cells with an expression vector comprising the nucleotide sequence of the antibody and the nucleotide sequence of a selectable (e.g., neomycin or hygromycin), and selecting for expression of the selectable marker. Such engineered cell lines may be particularly useful in screening and evaluation of compounds that interact directly or indirectly with the antibody molecule.

The expression levels of the antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot, 1986, Nature 322:52; Kohler, 1980, Proc. Natl. Acad. Sci. USA 77:2197). The coding sequences for the heavy and light chains may comprise cDNA or genomic DNA.

Once the antibody molecule of the invention has been recombinantly expressed, it may be purified by any method known in the art for purification of an antibody molecule, for example, by chromatography *(e.g.,* ion exchange chromatography, affinity chromatography such as with protein A or specific antigen, and sizing column chromatography), centrifugation, differential solubility, or by any other standard technique for the purification of proteins.

Alternatively, any fusion protein may be readily purified by utilizing an antibody specific for the fusion protein being expressed. For example, a system described by Janknecht et al. allows for the ready purification of non-denatured fusion proteins expressed in human cell lines (Janknecht et al., 1991, Proc. Natl. Acad. Sci. USA 88:8972-897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with recombinant vaccinia virus are loaded onto Ni²⁺ nitriloacetic acid-agarose columns and histidine-tagged proteins are selectively eluted with imidazole-containing buffers.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labelled secondary antibody (for example, an anti-mouse antibody conjugated to alkaline phosphatase if the raised antibodies are mouse antibodies) is added to the wells and incubated for about 30 min and then washed. Substrate is added to the wells and a colour reaction will appear where antibody to the immobilized polypeptide(s) is present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs *(e.g.,* in sandwich assays) may interfere with one another sterically, etc., assay performance of an antibody may be a more important measure than absolute affinity and specificity of an antibody.

Those skilled in the art will recognize that many approaches can be taken in producing antibodies or binding fragments and screening and selecting for affinity and specificity for the various polypeptides, but these approaches do not change the scope of the invention.

For therapeutic applications, antibodies (particularly monoclonal antibodies) may suitably be human or humanized animal (e.g. mouse) antibodies. Animal antibodies may be raised in animals using the human protein (e.g. Integrin beta 4) as immunogen. Humanisation typically involves grafting CDRs identified thereby into human framework regions. Normally some subsequent retromutation to optimize the conformation of chains is required. Such processes are known to persons skilled in the art.

### Expression of Affibodies

The construction of Affibodies has been described elsewhere (Ronnmark J, Gronlund H, Uhle' n, M., Nygren P.A°, Human immunoglobulin A (IgA)-specific ligands from combinatorial engineering of protein A, 2002, Eur. J. Biochem. 269, 2647-2655.), including the construction of affibody phage display libraries (Nord, K., Nilsson, J., Nilsson, B., Uhle' n, M. & Nygren, P.A°, A combinatorial library of an a-helical bacterial receptor domain, 1995, Protein Eng. 8, 601-608. Nord, K., Gunneriusson, E., Ringdahl, J., Sta° hl, S., Uhle' n, M. & Nygren, P.A°, Binding proteins selected from combinatorial libraries of an a-helical bacterial receptor domain, 1997, Nat. Biotechnol.15, 772-777.)

The biosensor analyses to investigate the optimal Affibody variants using biosensor binding studies has also been described elsewhere (Ronnmark J, Gronlund H, Uhle n, M., Nygren P.A°, Human immunoglobulin A (IgA)-specific ligands from combinatorial engineering of protein A, 2002, Eur. J. Biochem. 269, 2647-2655.).

### Affinity Reagent Modifications

In a preferred embodiment, anti-Integrin beta 4 affinity reagents such as antibodies or fragments thereof are conjugated to a diagnostic moiety (such as a detectable label) or a therapeutic moiety. The antibodies can be used for diagnosis or to determine the efficacy of a given treatment regimen. Detection can be facilitated by coupling the antibody to a detectable substance (label). Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics according to the present invention. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include ¹²⁵I, ¹³¹I, ¹¹¹In and ⁹⁹Tc. ⁶⁸Ga may also be employed.

Anti-Integrin beta 4 antibodies or fragments thereof as well as other affinity reagents can be conjugated to a therapeutic agent or drug moiety to modify a given biological response. An exemplary therapeutic agent to which the affinity reagent may be conjugated is a cytotoxic moiety. The therapeutic agent or drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumour necrosis factor, α-interferon, β-interferon, nerve growth factor, platelet derived growth factor, tissue plasminogen activator, a thrombotic agent or an anti-angiogenic agent, *e.g.,* angiostatin or endostatin; or, a biological response modifier such as a lymphokine, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF) or other growth factor.

Techniques for conjugating such therapeutic moiety to antibodies are well known, see, *e.g.,* Amon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

Alternatively, an antibody can be conjugated to a second antibody to form an antibody heteroconjugate as described by Segal in U.S. Patent No. 4,676,980.

An antibody with or without a therapeutic moiety conjugated to it can be used as a therapeutic that is administered alone or in combination with cytotoxic factor(s) and/or cytokine(s).

Fully human, or humanised antibodies may be used that induce antibody-directed cell-mediated cytotoxicity (ADCC). A fully human antibody is one in which the protein sequences are encoded by naturally occurring human immunoglobulin sequences, either from isolated antibody-producing human B-lymphocytes, or from transgenic murine B-lymphocytes of mice in which the murine immunoglobulin coding chromosomal regions have been replaced by orthologous human sequences. Transgenic antibodies of the latter type include, but are not restricted to, HuMab (Medarex, Inc., CA) and Xenomouse (Abgenix Inc., CA). A humanised antibody is one in which the constant region of a non-human antibody molecule of appropriate antigen specificity, is replaced by the constant region of a human antibody, preferably of the IgG subtype, with appropriate effector functions (Morrison et al., 1984, Proc. Natl. Acad. Sci. 81:851-855; Neuberger et al., 1984, Nature 312:604-608; Takeda et al., 1985, Nature 314:452-454). Appropriate effector functions include ADCC, which is a natural process by which fully-human antibodies or humanized antibodies, when bound to targets on the surface of cancer cells, switch on the cell killing properties of lymphocytes that are part of the normal immune system. These active lymphocytes, called Natural Killer (NK) cells, use a cytotoxic process to destroy living cells to which the antibodies are bound. ADCC activity may be detected and quantified by measuring release of Europium (Eu3+) from Eu3+ labelled, living cells in the presence of an antigen-specific antibody and peripheral blood mononuclear cells extracted from an immunocompetent, living human subject. The ADCC process is described in detail in Janeway Jr. C.A. et al., Immunobiology, 5th ed., 2001, Garland Publishing, ISBN 0-8153-3642-X; Pier G.B. et al., Immunology, Infection, and Immunity, 2004, p246-5; Albanell J. et al., Advances in Experimental Medicine and Biology, 2003, 532:p2153-68 and Weng, W.-K. et al., Journal of Clinical Oncology, 2003, 21:p 3940-3947. Suitable methods for the detection and quantification of ADCC can be found in Blomberg et al., Journal of Immunological Methods. 1986, 86:p225-9; Blomberg et al., Journal of Immunological Methods. 1986, 21;92:p117-23 and Patel & Boyd, Journal of Immunological Methods. 1995, 184:p29-38.

ADCC typically involves activation ofNK cells and is dependent on the recognition of antibody-coated cells by Fc receptors on the surface of the NK cell. The Fc receptors recognize the Fc (crystalline) portion of antibodies such as IgG, bound specifically to the surface of a target cell. The Fc receptor that triggers activation of the NK cell is called CD16 or FcγRIIIa. Once the FcγRIIIa receptor is bound to the IgG Fc, the NK cell releases cytokines such as IFN-γ, and cytotoxic granules containing perforin and granzymes that enter the target cell and promote cell death by triggering apoptosis.

The induction of antibody-dependent cellular cytotoxicity (ADCC) by an antibody can be enhanced by modifications that alter interactions between the antibody constant region (Fc) and various receptors that are present on the surface of cells of the immune system. Such modifications include the reduction or absence of alpha1,6-linked fucose moieties in the complex oligosaccharide chains that are normally added to the Fc of antibodies during natural or recombinant synthesis in mammalian cells. In a particular embodiment, non-fucosylated anti-Integrin beta 4 affinity reagents such as antibodies or fragments thereof are produced for the purpose of enhancing their ability to induce the ADCC response.

Techniques for reducing or ablating alpha1,6-linked fucose moieties in the oligosaccharide chains of the Fc are well established. In one example, the recombinant antibody is synthesized in a cell line that is impaired in its ability to add fucose in an alpha 1,6 linkage to the innermost N-acetylglucosamine of the N-linked biantennary complex-type Fc oligosaccharides. Such cell lines include, but are not limited to, the rat hybridoma YB2/0, which expresses a reduced level of the alpha 1,6-fucosyltransferase gene, FUT8. Preferably, the antibody is synthesized in a cell line that is incapable of adding alpha 1,6-linked fucosyl moieties to complex oligosaccharide chains, due to the deletion of both copies of the FUT8 gene. Such cell lines include, but are not limited to, FUT8-/- CHO/DG44 cell lines. Techniques for synthesizing partially fucosylated, or non-fucosylated antibodies and affinity reagents are described in Shinkawa et al., J. Biol. Chem. 278:3466-34735 (2003); Yamane-Ohnuki et al., Biotechnology and Bioengineering 87: 614-22 (2004) and in WO00/61739 A1, WO02/31140 A1 and WO03/085107 A1. In a second example, the fucosylation of a recombinant antibody is reduced or abolished by synthesis in a cell line that has been genetically engineered to overexpress a glycoprotein-modifying glycosyl transferase at a level that maximizes the production of complex N-linked oligosaccharides carrying bisecting N-acetylglucosamine. For example, the antibody is synthesized in a Chinese Hamster Ovary cell line expressing the enzyme N-acetyl glucosamine transferase III (GnT III). Cell lines stably transfected with suitable glycoprotein-modifying glycosyl transferases, and methods of synthesizing antibodies using these cells are described in WO9954342.

A non-fucosylated antibody or affinity reagent can be used as a therapeutic that is administered alone or in combination with cytotoxic factor(s) and/or cytokine(s).

In a further modification, the amino acid sequences of the antibody Fc are altered in a way that enhances ADCC activation, without affecting ligand affinity. Examples of such modifications are described in Lazar et al., Proceedings of the National Academy of Sciences 2006, 103:p4005-4010; WO03074679 and WO2007039818. In these examples, substitution of amino acids in the antibody Fc, such as aspartate for serine at position 239, and isoleucine for glutamate at position 332, altered the binding affinity of an antibody for Fc receptors, leading to an increase in ADCC activation.

An antibody reagent with enhanced ADCC activation due to amino acid substitutions can be used as a therapeutic that is administered alone or in combination with cytotoxic factor(s) and/or cytokine(s).

### Diagnosis of Breast Cancer, Colorectal Cancer, Gastric Cancer, Hepatocellular Carcinoma, Lung Cancer and Pancreatic Cancer

Test samples of breast, colorectal, gastric epithelium, liver, lung or pancreatic tissue, serum, plasma or urine obtained from a subject suspected of having or known to have breast cancer, colorectal cancer, gastric cancer, hepatocellular carcinoma, lung cancer or pancreatic cancer can be used for diagnosis or monitoring. A change in the abundance of Integrin beta 4 in a test sample relative to a control sample (from a subject or subjects free from breast cancer, colorectal cancer, gastric cancer, hepatocellular carcinoma, lung cancer and pancreatic cancer) or a previously determined reference range may indicate the presence of breast cancer, colorectal cancer, gastric cancer, hepatocellular carcinoma, lung cancer or pancreatic cancer. In another embodiment, the relative abundance of Integrin beta 4 in a test sample compared to a control sample or a previously determined reference range indicates a subtype of breast cancer, colorectal cancer, gastric cancer, hepatocellular carcinoma, lung cancer or pancreatic cancer (e.g. inflammatory breast cancer, familial or sporadic colorectal cancer, gastrointestinal stromal tumours, fibrolamellar hepatocellular carcinoma, squamous cell lung carcinoma or endocrine tumours of the pancreas). In yet another embodiment, the relative abundance of Integrin beta 4 in a test sample relative to a control sample or a previously determined reference range indicates the degree or severity of breast cancer, colorectal cancer, gastric cancer, hepatocellular carcinoma, lung cancer or pancreatic cancer (e.g. the likelihood for metastasis). In any of the aforesaid methods, detection of Integrin beta 4 may optionally be combined with detection of one or more of additional biomarkers for breast cancer, colorectal cancer, gastric cancer, hepatocellular carcinoma, lung cancer or pancreatic cancer. Any suitable method in the art can be employed to measure the level of Integrin beta 4, including but not limited to the Preferred Technology described herein, kinase assays, immunoassays to detect and/or visualize Integrin beta 4 *(e.g.,* Western blot, immunoprecipitation followed by sodium dodecyl sulfate polyacrylamide gel electrophoresis, immunocytochemistry, etc.). In a further embodiment, a change in the abundance of mRNA encoding Integrin beta 4 in a test sample relative to a control sample or a previously determined reference range indicates the presence of breast cancer, colorectal cancer, gastric cancer, hepatocellular carcinoma, lung cancer or pancreatic cancer. Any suitable hybridization assay can be used to detect Integrin beta 4 expression by detecting and/or visualizing mRNA encoding the Integrin beta 4 *(e.g.,* Northern assays, dot blots, *in situ* hybridization, etc.).

In another embodiment , labelled antibodies (or other affinity reagents), derivatives and analogues thereof, which specifically bind to Integrin beta 4 can be used for diagnostic purposes to detect, diagnose, or monitor breast cancer, colorectal cancer, gastric cancer, hepatocellular carcinoma, lung cancer and pancreatic cancer. For example, breast cancer, colorectal cancer, gastric cancer, hepatocellular carcinoma, lung cancer or pancreatic cancer may be detected in an animal, such as in a mammal and particularly in a human.

### Therapeutic Use of Integrin beta 4

Breast cancer, colorectal cancer, gastric cancer, hepatocellular carcinoma, lung cancer or pancreatic cancer can be treated (e.g. to ameliorate symptoms or to retard onset or progression) or prevented by administration of a therapeutic compound that reduces function or expression of Integrin beta 4 in the serum or tissue of subjects having breast cancer, colorectal cancer, gastric cancer, hepatocellular carcinoma, lung cancer or pancreatic cancer.

One or more antibodies (or other affinity reagents) each specifically binding to Integrin beta 4 may be administered alone or in combination with one or more additional therapeutic compounds or treatments.

A biological product such as an antibody (or other affinity reagent) is, for example, allogeneic to the subject to which it is administered. An antibody to a human Integrin beta 4 or a human Integrin beta 4-related polypeptide, may be administered to a human subject for therapy (e.g. to ameliorate symptoms or to retard onset or progression) or prophylaxis.

Without being limited by theory, it is conceived that the therapeutic activity of antibodies (or other affinity reagents) which specifically bind to Integrin beta 4 may be achieved through the phenomenon of Antibody -Dependent Cell-mediated Cytotoxicity (ADCC) (see e.g. Janeway Jr. C.A. et al., Immunobiology, 5th ed., 2001, Garland Publishing, ISBN 0-8153-3642-X; Pier G.B. et al., Immunology, Infection, and Immunity, 2004, p246-5; Albanell J. et al., Advances in Experimental Medicine and Biology, 2003, 532:p2153-68 and Weng, W.-K. et al., Journal of Clinical Oncology, 2003, 21:p 3940-3947).

### Treatment and Prevention of Breast Cancer, Colorectal Cancer, Gastric Cancer, Hepatocellular Carcinoma, Lung Cancer and Pancreatic Cancer

Breast cancer, colorectal cancer, gastric cancer, hepatocellular carcinoma, lung cancer or pancreatic cancer may be treated or prevented by administration to a subject suspected of having or known to have breast cancer, colorectal cancer, gastric cancer, hepatocellular carcinoma, lung cancer or pancreatic cancer or to be at risk of developing breast cancer, colorectal cancer, gastric cancer, hepatocellular carcinoma, lung cancer or pancreatic cancer of a compound that modulates (*i.e.,* increases or decreases) the level or activity *(i.e.,* function) of Integrin beta 4 that is differentially present in the serum or tissue of subjects having breast cancer, colorectal cancer, gastric cancer, hepatocellular carcinoma, lung cancer or pancreatic cancer compared with serum or tissue of subjects free from breast cancer, colorectal cancer, gastric cancer, hepatocellular carcinoma, lung cancer and pancreatic cancer. In one embodiment, breast cancer, colorectal cancer, gastric cancer, hepatocellular carcinoma, lung cancer or pancreatic cancer may be treated or prevented by administering to a subject suspected of having or known to have breast cancer, colorectal cancer, gastric cancer, hepatocellular carcinoma, lung cancer or pancreatic cancer or to be at risk of developing breast cancer, colorectal cancer, gastric cancer, hepatocellular carcinoma, lung cancer or pancreatic cancer a compound that upregulates (*i.e.,* increases) the level or activity *(i.e.,* function) of Integrin beta 4 that are decreased in the serum or tissue of subjects having breast cancer, colorectal cancer, gastric cancer, hepatocellular carcinoma, lung cancer or pancreatic cancer. Examples of such a compound include, but are not limited to, Integrin beta 4 antisense oligonucleotides, ribozymes, antibodies (or other affinity reagents) directed against Integrin beta 4, and compounds that inhibit the enzymatic activity of Integrin beta 4. Other useful compounds *e.g.,* Integrin beta 4 antagonists and small molecule Integrin beta 4 antagonists, can be identified using *in vitro* assays.

Breast cancer, colorectal cancer, gastric cancer, hepatocellular carcinoma, lung cancer or pancreatic cancer may also be treated or prevented by administration to a subject suspected of having or known to have breast cancer, colorectal cancer, gastric cancer, hepatocellular carcinoma, lung cancer or pancreatic cancer or to be at risk of developing breast cancer, colorectal cancer, gastric cancer, hepatocellular carcinoma, lung cancer or pancreatic cancer of a compound that downregulates the level or activity (i.e. function) of Integrin beta 4 that are increased in the serum or tissue of subjects having breast cancer, colorectal cancer, gastric cancer, hepatocellular carcinoma, lung cancer or pancreatic cancer. Examples of such a compound include but are not limited to: Integrin beta 4, Integrin beta 4 fragments and Integrin beta 4-related polypeptides; nucleic acids encoding Integrin beta 4, a Integrin beta 4 fragment and a Integrin beta 4-related polypeptide *(e.g.,* for use in gene therapy); and, for those Integrin beta 4 or Integrin beta 4-related polypeptides with enzymatic activity, compounds or molecules known to modulate that enzymatic activity. Other compounds that can be used, *e.g.,* Integrin beta 4 agonists, can be identified using in *in vitro* assays.

Therapy or prophylaxis may be tailored to the needs of an individual subject. Thus, in specific embodiments, compounds that promote the level or function of Integrin beta 4 are therapeutically or prophylactically administered to a subject suspected of having or known to have breast cancer, colorectal cancer, gastric cancer, hepatocellular carcinoma, lung cancer or pancreatic cancer, in whom the levels or functions of said Integrin beta 4 are absent or are decreased relative to a control or normal reference range. In further embodiments, compounds that promote the level or function of Integrin beta 4 are therapeutically or prophylactically administered to a subject suspected of having or known to have breast cancer, colorectal cancer, gastric cancer, hepatocellular carcinoma, lung cancer or pancreatic cancer in whom the levels or functions of said Integrin beta 4 are increased relative to a control or to a reference range. In further embodiments, compounds that decrease the level or function of Integrin beta 4 are therapeutically or prophylactically administered to a subject suspected of having or known to have breast cancer, colorectal cancer, gastric cancer, hepatocellular carcinoma, lung cancer or pancreatic cancer in whom the levels or functions of said Integrin beta 4 are increased relative to a control or to a reference range. In further embodiments, compounds that decrease the level or function of Integrin beta 4 are therapeutically or prophylactically administered to a subject suspected of having or known to have breast cancer, colorectal cancer, gastric cancer, hepatocellular carcinoma, lung cancer or pancreatic cancer in whom the levels or functions of said Integrin beta 4 are decreased relative to a control or to a reference range. The change in Integrin beta 4 function or level due to the administration of such compounds can be readily detected, *e.g.,* by obtaining a sample *(e.g.,* blood or urine) and assaying *in vitro* the levels or activities of said Integrin beta 4, or the levels of mRNA encoding said Integrin beta 4, or any combination of the foregoing. Such assays can be performed before and after the administration of the compound as described herein.

The antibody may be given in combination with any other chemotherapy drugs.

### Vaccine Therapy

Integrin beta 4 may be useful as antigenic material, and may be used in the production of vaccines for treatment or prophylaxis of breast cancer, colorectal cancer, gastric cancer, hepatocellular carcinoma, lung cancer and pancreatic cancer. Such material can be "antigenic" and/or "immunogenic". Generally, "antigenic" is taken to mean that the protein is capable of being used to raise antibodies (or other affinity reagents) or indeed is capable of inducing an antibody response in a subject or experimental animal. "Immunogenic" is taken to mean that the protein is capable of eliciting a protective immune response in a subject or experimental animal. Thus, in the latter case, the protein may be capable of not only generating an antibody response but, in addition, non-antibody based immune responses. "Immunogenic" also embraces whether the protein may elicit an immune-like response in an in-vitro setting e.g. a T-cell proliferation assay. The generation of an appropriate immune response may require the presence of one or more adjuvants and/or appropriate presentation of an antigen.

The skilled person will appreciate that homologues or derivatives of Integrin beta 4 will also find use as antigenic/immunogenic material. Thus, for instance proteins which include one or more additions, deletions, substitutions or the like may be used. In addition, it may be possible to replace one amino acid with another of similar "type". For instance, replacing one hydrophobic amino acid with another. One can use a program such as the CLUSTAL program to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of analysis may be used.

In the case of homologues and derivatives, the degree of identity with a protein as described herein is less important than that the homologue or derivative should retain its antigenicity and/or immunogenicity. However, suitably, homologues or derivatives having at least 60% similarity (as discussed above) with the proteins or polypeptides described herein are provided, for example, homologues or derivatives having at least 70% similarity, such as at least 80% similarity. Particularly, homologues or derivatives having at least 90% or even 95% similarity are provided. Suitably, homologues or derivatives have at least 60% sequence identity with the proteins or polypeptides described herein, for example, homologues or derivatives have at least 70% identity, such as at least 80% identity. Particularly, homologues or derivatives have at least 90% or even 95% identity.

In an alternative approach, the homologues or derivatives could be fusion proteins, incorporating moieties which render purification easier, for example by effectively tagging the desired protein or polypeptide. It may be necessary to remove the "tag" or it may be the case that the fusion protein itself retains sufficient antigenicity to be useful.

It is well known that it is possible to screen an antigenic protein or polypeptide to identify epitopic regions, i.e. those regions which are responsible for the protein or polypeptide's antigenicity or immunogenicity. Methods well known to the skilled person can be used to test fragments and/or homologues and/or derivatives for antigenicity. Thus, the fragments should include one or more such epitopic regions or be sufficiently similar to such regions to retain their antigenic/immunogenic properties. Thus, for fragments the degree of identity is perhaps irrelevant, since they may be 100% identical to a particular part of a protein or polypeptide, homologue or derivative as described herein. The key issue, once again, is that the fragment retains the antigenic/immunogenic properties of the protein from which it is derived.

What is important for homologues, derivatives and fragments is that they possess at least a degree of the antigenicity/immunogenicity of the protein or polypeptide from which they are derived.

### Inhibition of Integrin beta 4 to Treat Breast Cancer, Colorectal Cancer, Gastric Cancer, Hepatocellular Carcinoma, Lung Cancer and Pancreatic Cancer

Breast cancer, colorectal cancer, gastric cancer, hepatocellular carcinoma, lung cancer or pancreatic cancer may be treated or prevented by administration of a compound that antagonizes (inhibits) the level and/or function of Integrin beta 4 which are elevated in the serum or tissue of subjects having breast cancer, colorectal cancer, gastric cancer, hepatocellular carcinoma, lung cancer or pancreatic cancer as compared with serum or tissue of subjects free from breast cancer, colorectal cancer, gastric cancer, hepatocellular carcinoma, lung cancer and pancreatic cancer.

Compounds useful for this purpose include but are not limited to anti-Integrin beta 4 antibodies (or other affinity reagents, and fragments and derivatives containing the binding region thereof), Integrin beta 4 antisense or ribozyme nucleic acids, and nucleic acids encoding dysfunctional Integrin beta 4 that are used to "knockout" endogenous Integrin beta 4 function by homologous recombination (see, *e.g.,* Capecchi, 1989, Science 244:1288-1292). Other compounds that inhibit Integrin beta 4 function can be identified by use of known *in vitro* assays, *e.g.,* assays for the ability of a test compound to inhibit binding of Integrin beta 4 to another protein or a binding partner, or to inhibit a known Integrin beta 4 function.

Such inhibition is, for example, assayed *in vitro* or in cell culture, but genetic assays may also be employed. The Preferred Technology described herein can also be used to detect levels of Integrin beta 4 before and after the administration of the compound. Suitable *in vitro* or *in vivo* assays are utilized to determine the effect of a specific compound and whether its administration is indicated for treatment of the affected tissue, as described in more detail below.

A compound that inhibits Integrin beta 4 function (activity) may be administered therapeutically or prophylactically to a subject in whom an increased serum or tissue level or functional activity of Integrin beta 4 *(e.g.,* greater than the normal level or desired level) is detected as compared with serum or tissue of subjects with breast cancer, colorectal cancer, gastric cancer, hepatocellular carcinoma, lung cancer or pancreatic cancer who do not receive treatment or to bring the level or activity to that found in subjects free from breast cancer, colorectal cancer, gastric cancer, hepatocellular carcinoma, lung cancer and pancreatic cancer or a predetermined reference range. Methods standard in the art can be employed to measure the increase in Integrin beta 4 level or function, as outlined above.

Compounds for use in therapy can be tested in suitable animal model systems prior to testing in humans, including but not limited to rats, mice, chicken, cows, monkeys, rabbits, etc. For *in vivo* testing, prior to administration to humans, any animal model system known in the art may be used. Examples of animal models of breast cancer, colorectal cancer, gastric cancer, hepatocellular carcinoma, lung cancer and pancreatic cancer include, but are not limited to xenografts of breast cell lines such as MCF-7 (Ozzello L, Sordat M., Eur J Cancer. 1980;16:553-559) and MCF10AT (Miller et al., J Natl Cancer Inst. 1993;85:1725-1732) in nude or SCID mice; xenografts of colorectal cancer cell lines such as MDA-MB-435 in oestrogen-deprived Severe Combined Immunodeficient (SCID) mice (Eccles et al., 1994 Cell Biophysics 24/25, 279); xenografts of gastric cell lines such as AZ-521 in nude mice; xenografts of hepatocellular carcinoma cell lines such as MHCC97 in nude mice (Tian et al., Br J Cancer 1999 Nov;81(5):814-21); xenografts of non small cell lung cancer cell lines such as A549 and H460 and xenografts of small cell lung cancer cell lines such as NCI-H345 and xenografts of pancreatic cancer cell lines such as MIA PaCa-2 in nude mice (Marincola et al., J Surg Res 1989 Dec;47(6):520-9). These can be utilized to test compounds that modulate Integrin beta 4 levels, since the pathology exhibited in these models is similar to that of breast cancer, colorectal cancer, gastric cancer, hepatocellular carcinoma, lung cancer and pancreatic cancer. It is also apparent to the skilled artisan that based upon the present disclosure, transgenic animals can be produced with "knock-out" mutations of the gene or genes encoding Integrin beta 4. A "knock-out" mutation of a gene is a mutation that causes the mutated gene to not be expressed, or expressed in an aberrant form or at a low level, such that the activity associated with the gene product is nearly or entirely absent. The transgenic animal is, for example, a mammal; such as a mouse.

### Therapeutic and Prophylactic Compositions and their Use

Also disclosed are methods of treatment (and prophylaxis) comprising administering to a subject an effective amount of a compound of the invention. In a particular aspect, the compound is substantially purified *(e.g.,* substantially free from substances that limit its effect or produce undesired side effects). The subject is, for example, an animal, including but not limited to animals such as cows, pigs, horses, chickens, cats, dogs, etc., and is, for example, a mammal, such as a human. In a specific embodiment, a non-human mammal is the subject.

Formulations and methods of administration that can be employed when the compound comprises a nucleic acid are described above; additional appropriate formulations and routes of administration are described below.

Various delivery systems are known and can be used to administer a compound of the invention, *e.g.,* encapsulation in liposomes, microparticles, microcapsules, recombinant cells capable of expressing the compound, receptor mediated endocytosis (see, *e.g.,* Wu and Wu, 1987, J. Biol. Chem. 262:4429-4432), construction of a nucleic acid as part of a retroviral or other vector, etc. Methods of introduction can be enteral or parenteral and include but are not limited to intradermal, intramuscular, intraperitoneal, intravenous, subcutaneous, intranasal, epidural, and oral routes. The compounds may be administered by any convenient route, for example by infusion or bolus injection, by absorption through epithelial or mucocutaneous linings *(e.g.,* oral mucosa, rectal and intestinal mucosa, etc.) and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, *e.g.,* by use of an inhaler or nebulizer, and formulation with an aerosolizing agent.

In a specific embodiment, it may be desirable to administer the pharmaceutical compositions of the invention locally to the area in need of treatment; this may be achieved, for example, and not by way of limitation, by local infusion during surgery, topical application, *e.g.,* by injection, by means of a catheter, or by means of an implant, said implant being of a porous, non-porous, or gelatinous material, including membranes, such as sialastic membranes, or fibers. In one embodiment, administration can be by direct injection into breast, colorectal, gastric epithelium, liver, lung or pancreatic tissue or at the site (or former site) of a malignant tumour or neoplastic or pre-neoplastic tissue.

In another embodiment, the compound can be delivered in a vesicle, in particular a liposome (*see* Langer, 1990, Science 249:1527-1533; Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; *see* generally *ibid.*)

In yet another embodiment, the compound can be delivered in a controlled release system. In one embodiment, a pump may be used (see Langer, *supra;* Sefton, 1987, CRC Crit. Ref. Biomed. Eng. 14:201; Buchwald et al., 1980, Surgery 88:507; Saudek et al., 1989, N. Engl. J. Med. 321:574). In another embodiment, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J., 1983, Macromol. Sci. Rev. Macromol. Chem. 23:61; see also Levy et al., 1985, Science 228:190; During et al., 1989, Ann. Neurol. 25:351; Howard et al., 1989, J. Neurosurg. 71:105). In yet another embodiment, a controlled release system can be placed in proximity of the therapeutic target, i.e., the breast, colon, stomach, liver, lung or pancreas thus requiring only a fraction of the systemic dose (see, *e.g.,* Goodson, in Medical Applications of Controlled Release, supra, vol. 2, pp. 115-138 (1984)).

Other controlled release systems are discussed in the review by Langer (1990, Science 249:1527-1533).

The present invention also provides pharmaceutical compositions. Such compositions comprise a therapeutically effective amount of a compound, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the compound, preferably in purified form, together with a suitable amount of carrier so as to provide the form for proper administration to the subject. The formulation should suit the mode of administration.

In one embodiment, for example where one or more antibodies are employed, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anaesthetic such as lidocaine to ease pain at the site of the injection. Generally, the ingredients are supplied either separately or mixed together in unit dosage form, for example, as a dry lyophilized powder or water free concentrate in a hermetically sealed container such as an ampoule or sachet indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds for use in the invention can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.

The amount of the compound which will be effective in the treatment of breast cancer, colorectal cancer, gastric cancer, hepatocellular carcinoma, lung cancer or pancreatic cancer can be determined by standard clinical techniques. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each subject's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from *in vitro* or animal model test systems.

Suppositories generally contain active ingredient in the range of 0.5% to 10% by weight; oral formulations preferably contain 10% to 95% active ingredient.

Also disclosed is a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects (a) approval by the agency of manufacture, use or sale for human administration, (b) directions for use, or both.

The kit may comprise antibodies for example the antibodies may be lyophilized for reconstitution before administration or use. Where the kit is for use in therapy/treatment such as cancer the antibody or antibodies may be reconstituted with an isotonic aqueous solution, which may optionally be provided with the kit. In one aspect the kit may comprise a polypeptide such as an immunogenic polypeptide employed in the invention, which may for example be lyophilized. The latter kit may further comprise an adjuvant for reconstituting the immunogenic polypeptide.

### Determining Abundance of Integrin beta 4 by Imaging Technology

An advantage of determining abundance of Integrin beta 4 by imaging technology may be that such a method is non-invasive (save that reagents may need to be administered) and there is no need to extract a sample from the subject.

Suitable imaging technologies include positron emission tomography (PET) and single photon emission computed tomography (SPECT). Visualisation of Integrin beta 4 using such techniques requires incorporation or binding of a suitable label e.g. a radiotracer such as ¹⁸F, ¹¹C or ¹²³I (see e.g. NeuroRx - The Journal of the American Society for Experimental NeuroTherapeutics (2005) 2(2), 348-360 and idem pages 361-371 for further details of the techniques). Radiotracers or other labels may be incorporated into Integrin beta 4 by administration to the subject (e.g. by injection) of a suitably labelled specific ligand. Alternatively they may be incorporated into a binding affinity reagent (e.g. an antibody) specific for Integrin beta 4 which may be administered to the subject (e.g. by injection). For discussion of use of Affibodies for imaging see e.g. Orlova A, Magnusson M, Eriksson TL, Nilsson M, Larsson B, Hoiden-Guthenberg I, Widstrom C, Carlsson J, Tolmachev V, Stahl S, Nilsson FY, Tumour imaging using a picomolar affinity HER2 binding affibody molecule, Cancer Res. 2006 Apr 15;66(8):4339-48).

### Diagnosis and Treatment of Breast Cancer, Colorectal Cancer, Gastric Cancer, Hepatocellular Carcinoma, Lung Cancer or Pancreatic Cancer using Immunohistochemistry

Immunohistochemistry is an excellent detection technique and may therefore be very useful in the diagnosis and treatment of breast cancer, colorectal cancer, gastric cancer, hepatocellular carcinoma, lung cancer or pancreatic cancer. Immunohistochemistry may be used to detect, diagnose, or monitor breast cancer, colorectal cancer, gastric cancer, hepatocellular carcinoma, lung cancer or pancreatic cancer through the localization of Integrin beta 4 antigens in tissue sections by the use of labelled antibodies (or other affinity reagents), derivatives and analogues thereof, which specifically bind to Integrin beta 4, as specific reagents through antigen-antibody interactions that are visualized by a marker such as fluorescent dye, enzyme, radioactive element or colloidal gold.

The advancement of monoclonal antibody technology has been of great significance in assuring the place of immunohistochemistry in the modern accurate microscopic diagnosis of human neoplasms. The identification of disseminated neoplastically transformed cells by immunohistochemistry allows for a clearer picture of cancer invasion and metastasis, as well as the evolution of the tumour cell associated immunophenotype towards increased malignancy. Future antineoplastic therapeutic approaches may include a variety of individualized immunotherapies, specific for the particular immunophenotypical pattern associated with each individual patient's neoplastic disease. For further discussion see e.g. Bodey B, The significance of immunohistochemistry in the diagnosis and therapy of neoplasms, Expert Opin Biol Ther. 2002 Apr;2(4):371-93.

### EXAMPLE 1: IDENTIFICATION OF MEMBRANE PROTEINS EXPRESSED IN BREAST CANCER, COLORECTAL CANCER, GASTRIC CANCER, HEPATOCELLULAR CARCINOMA, LUNG CANCER AND PANCREATIC CANCER BLOOD AND TISSUE SAMPLES

Using the following Reference Protocol, membrane proteins extracted from breast cancer, colorectal cancer, gastric cancer, hepatocellular carcinoma, lung cancer and pancreatic cancer tissue samples were separated by 1D gel and analysed.

### 1.1 MATERIALS AND METHODS

### 1.1.1 - Plasma Membrane Fractionation

The cells recovered from the epithelium of a breast adenocarcinoma, colorectal adenocarcinoma, gastric cancer, hepatocellular carcinoma, lung carcinoma and pancreatic adenocarcinoma were lysed and submitted to centrifugation at 1000G. The supernatant was taken, and it was subsequently centrifuged at 3000G. Once again, the supernatant was taken, and it was then centrifuged at 100 000G.

The resulting pellet was recovered and put on 15-60% sucrose gradient.

A Western blot was used to identify sub cellular markers, and the Plasma Membrane fractions were pooled.

The pooled solution was either run directly on 1D gels (see section 1.1.4 below), or further fractionated into heparin binding and nucleotide binding fractions as described below.

### 1.1.2 - Plasma Membrane Heparin-binding Fraction

The pooled solution from 1.1.1 above was applied to a Heparin column, eluted from column and run on 1D gels (see section 1.1.4 below).

### 1.1.3 - Plasma Nucleotide-binding Fraction

The pooled solution from 1.1.1 above was applied to a Cibacrom Blue 3GA column, eluted from column and run on 1D gels (see section 1.1.4 below).

### 1.1.4 - 1D gel technology

Protein or membrane pellets were solubilised in 1D sample buffer (1-2 µg/µl). The sample buffer and protein mixture was then heated to 95°C for 3 min.

A 9-16% acrylamide gradient gel was cast with a stacking gel and a stacking comb according to the procedure described in Ausubel F.M. et al., eds., 1989, Current Protocols in Molecular Biology, Vol. II, Green Publishing Associates, Inc., and John Wiley & Sons, Inc., New York, section 10.2, incorporated herein by reference in its entirety.

30-50 micrograms of the protein mixtures obtained from detergent and the molecular weight standards (66, 45 ,31, 21,14 kDa) were added to the stacking gel wells using a 10 microlitre pipette tip and the samples run at 40mA for 5 hours.

The plates were then prised open, the gel placed in a tray of fixer (10% acetic acid, 40% ethanol, 50% water) and shaken overnight. Following this, the gel was primed by 30 minutes shaking in a primer solution (7.5% acetic acid (75ml), 0.05% SDS (5ml of 10%)). The gel was then incubated with a fluorescent dye (7.5% acetic acid, 0.06% OGS in-house dye (600µl)) with shaking for 3 hrs. Sypro Red (Molecular Probes, Inc., Eugene, Oregon) is a suitable dye for this purpose. A preferred fluorescent dye is disclosed in U.S. Application No. 09/412,168, filed on October 5, 1999, which is incorporated herein by reference in its entirety.

A computer-readable output was produced by imaging the fluorescently stained gels with an Apollo 3 scanner (Oxford Glycosciences, Oxford, UK). This scanner is developed from the scanner described in WO 96/36882 and in the Ph.D. thesis of David A. Basiji, entitled "Development of a High-throughput Fluorescence Scanner Employing Internal Reflection Optics and Phase-sensitive Detection (Total Internal Reflection, Electrophoresis)", University of Washington (1997), Volume 58/12-B of Dissertation Abstracts International, page 6686, the contents of each of which are incorporated herein by reference. The latest embodiment of this instrument includes the following improvements: The gel is transported through the scanner on a precision lead-screw drive system. This is preferable to laying the glass plate on the belt-driven system that is defined in the Basiji thesis as it provides a reproducible means of accurately transporting the gel past the imaging optics.

The gel is secured into the scanner against three alignment stops that rigidly hold the glass plate in a known position. By doing this in conjunction with the above precision transport system and the fact that the gel is bound to the glass plate, the absolute position of the gel can be predicted and recorded. This ensures that accurate co-ordinates of each feature on the gel can be communicated to the cutting robot for excision. This cutting robot has an identical mounting arrangement for the glass plate to preserve the positional accuracy.

The carrier that holds the gel in place has integral fluorescent markers (Designated M1, M2, M3) that are used to correct the image geometry and are a quality control feature to confirm that the scanning has been performed correctly.

The optical components of the system have been inverted. The laser, mirror, waveguide and other optical components are now above the glass plate being scanned. The embodiment of the Basiji thesis has these underneath. The glass plate is therefore mounted onto the scanner gel side down, so that the optical path remains through the glass plate. By doing this, any particles of gel that may break away from the glass plate will fall onto the base of the instrument rather than into the optics.

In scanning the gels, they were removed from the stain, rinsed with water and allowed to air dry briefly and imaged on the Apollo 3. After imaging, the gels were sealed in polyethylene bags containing a small volume of staining solution, and then stored at 4°C.

Apparent molecular weights were calculated by interpolation from a set of known molecular weight markers run alongside the samples.

### 1.1.5 - Recovery and analysis of selected proteins

Proteins were robotically excised from the gels by the process described in U.S. Patent No. 6,064,754, Sections 5.4 and 5.6, 5.7, 5.8 (incorporated herein by reference), as is applicable to 1D-electrophoresis, with modification to the robotic cutter as follows: the cutter begins at the top of the lane, and cuts a gel disc 1.7mm in diameter from the left edge of the lane. The cutter then moves 2mm to the right, and 0.7mm down and cuts a further disc. This is then repeated. The cutter then moves back to a position directly underneath the first gel cut, but offset by 2.2mm downwards, and the pattern of three diagonal cuts are repeated. This is continued for the whole length of the gel.

NOTE: If the lane is observed to broaden significantly then a correction can be made also sideways i.e. instead of returning to a position directly underneath a previous gel cut, the cut can be offset slightly to the left (on the left of the lane) and/or the right (on the right of the lane). The proteins contained within the gel fragments were processed to generate tryptic peptides; partial amino acid sequences of these peptides were determined by mass spectroscopy as described in WO98/53323 and Application No. 09/094,996, filed June 15, 1998.

Proteins were processed to generate tryptic digest peptides. Tryptic peptides were analyzed by mass spectrometry using a PerSeptive Biosystems Voyager- DETM STR Matrix-Assisted Laser Desorption Ionization Time-of-Flight (MALDI-TOF) mass spectrometer, and selected tryptic peptides were analyzed by tandem mass spectrometry (MS/MS) using a Micromass Quadrupole Time-of-Flight (Q-TOF) mass spectrometer (Micromass, Altrincham, U.K.) equipped with a nanoflowTM electrospray Z-spray source. For partial amino acid sequencing and identification of Integrin beta 4, uninterpreted tandem mass spectra of tryptic peptides were searched using the SEQUEST search program (Eng et al., 1994, J. Am. Soc. Mass Spectrom. 5:976-989), version v.C.1. Criteria for database identification included: the cleavage specificity of trypsin; the detection of a suite of a, b and y ions in peptides returned from the database, and a mass increment for all Cys residues to account for

carbamidomethylation. The database searched was a database constructed of protein entries in the non redundant database held by the National Centre for Biotechnology Information (NCBI) which is accessible at www.ncbi.nlm.nih.gov. Following identification of proteins through spectral-spectral correlation using the SEQUEST program, masses detected in MALDI-TOF mass spectra were assigned to tryptic digest peptides within the proteins identified. In cases where no amino acid sequences could be identified through searching with uninterpreted MS/MS spectra of tryptic digest peptides using the SEQUEST program, tandem mass spectra of the peptides were interpreted manually, using methods known in the art. (In the case of interpretation of low-energy fragmentation mass spectra of peptide ions see Gaskell et al., 1992, Rapid Commun. Mass Spectrom. 6:658-662).

### 1.1.6 - Discrimination of breast cancer, colorectal cancer, gastric cancer, hepatocellular carcinoma, lung cancer and pancreatic cancer associated proteins

The process to identify Integrin beta 4 uses the peptide sequences obtained experimentally by mass spectrometry described above of naturally occurring human proteins to identify and organize coding exons in the published human genome sequence.

Recent dramatic advances in defining the chemical sequence of the human genome have led to the near completion of this immense task (Venter, J.C. et al. (2001). The sequence of the human genome. Science 16: 1304-51; International Human Genome Sequencing Consortium. (2001). Initial sequencing and analysis of the human genome Nature 409: 860-921). There is little doubt that this sequence information will have a substantial impact on our understanding of many biological processes, including molecular evolution, comparative genomics, pathogenic mechanisms and molecular medicine. For the full medical value inherent in the sequence of the human genome to be realised, the genome needs to be 'organised' and annotated. By this, is meant at least the following three things: (i) The assembly of the sequences of the individual portions of the genome into a coherent, continuous sequence for each chromosome. (ii) The unambiguous identification of those regions of each chromosome that contain genes. (iii) Determination of the fine structure of the genes and the properties of its mRNA and protein products. While the definition of a 'gene' is an increasingly complex issue (H Pearson: What is a gene? Nature (2006) 24: 399 - 401), what is of immediate interest for drug discovery and development is a catalogue of those genes that encode functional, expressed proteins. A subset of these genes will be involved in the molecular basis of most if not all pathologies. Therefore an important and immediate goal for the pharmaceutical industry is to identify all such genes in the human genome and describe their fine structure.

### Processing and integration of peptide masses, peptide signatures, ESTs and Public Domain Genomic Sequence Data to form OGAP® database

Discrete genetic units (exons, transcripts and genes) were identified using the following sequential steps:
1. A 'virtual transcriptome' is generated, containing the tryptic peptides which map to the human genome by combining the gene identifications available from Ensembl and various gene prediction programs. This also incorporates SNP data (from dbSNP) and all alternate splicing of gene identifications. Known contaminants were also added to the virtual transcriptome.
2. All tandem spectra in the OGeS Mass Spectrometry Database are interpreted in order to produce a peptide that can be mapped to one in the virtual transcriptome. A set of automated spectral interpretation algorithms were used to produce the peptide identifications.
3. The set of all mass-matched peptides in the OGeS Mass Spectrometry Database is generated by searching all peptides from transcripts hit by the tandem peptides using a tolerance based on the mass accuracy of the mass spectrometer, typically 20ppm.
4. All tandem and mass-matched peptides are combined in the form of "protein clusters". This is done using a recursive process which groups sequences into clusters based on common peptide hits. Biological sequences are considered to belong to the same eluster if they share one or more tandem or mass-matched peptide.
5. After initial filtering to screen out incorrectly identified peptides, the resulting clusters are then mapped on the human genome.
6. The protein clusters are then aggregated into regions that define preliminary gene boundaries using their proximity and the co-observation of peptides within protein clusters. Proximity is defined as the peptide being within 80,000 nucleotides on the same strand of the same chromosome. Various elimination rules, based on cluster observation scoring and multiple mapping to the genome are used to refine the output. The resulting 'confirmed genes' are those which best account for the peptides and masses observed by mass spectrometry in each cluster. Nominal co-ordinates for the gene are also an output of this stage.
7. The best set of transcripts for each confirmed gene are created from the protein clusters, peptides, ESTs, candidate exons and molecular weight of the original protein spot.
8. Each identified transcript was linked to the sample providing the observed peptides.
9. Use of an application for viewing and mining the data. The result of steps 1 - 8 was a database containing genes, each of which consisted of a number of exons and one or more transcripts. An application was written to display and search this integrated genome / proteome data. Any features (OMIM disease locus, InterPro etc.) that had been mapped to the same Golden Path co-ordinate system by Ensembl could be cross-referenced to these genes by coincidence of location and fine structure.

### Results

The process was used to generate approximately 1 million peptide sequences to identify protein-coding genes and their exons resulted in the identification of protein sequences for 18083 genes across 67 different tissues and 57 diseases including 506 genes in Bladder cancer, 4,713 genes in Breast cancer, 766 genes in Burkitt's lymphoma, 1,371 genes in Cervical cancer, 949 genes in Colorectal cancer, 524 genes in Gastric cancer, 1,782 genes in Hepatocellular carcinoma, 2,424 genes in chronic lymphocytic leukaemia (CLL), 978 genes in Lung cancer, 1,764 genes in Melanoma, 1,033 genes in Ovarian Cancer, 2,961 genes in Pancreatic cancer and 3,307 genes in Prostate cancer, illustrated here by Integrin beta 4 isolated and identified from breast cancer, colorectal cancer, gastric cancer, hepatocellular carcinoma, lung cancer and pancreatic cancer samples. Following comparison of the experimentally determined sequences with sequences in the OGAP® database, Integrin beta 4 showed a high degree of specificity to breast cancer, colorectal cancer, gastric cancer, hepatocellular carcinoma, lung cancer and pancreatic cancer indicative of the prognostic and diagnostic nature.

### 1.2 RESULTS

These experiments identified Integrin beta 4, in its five different splice variants, as further described herein. The full-length Integrin beta 4 was detected in the plasma membrane of breast cancer, colorectal cancer, gastric cancer, hepatocellular carcinoma, lung cancer and pancreatic cancer samples and was not detected in the cytosol.

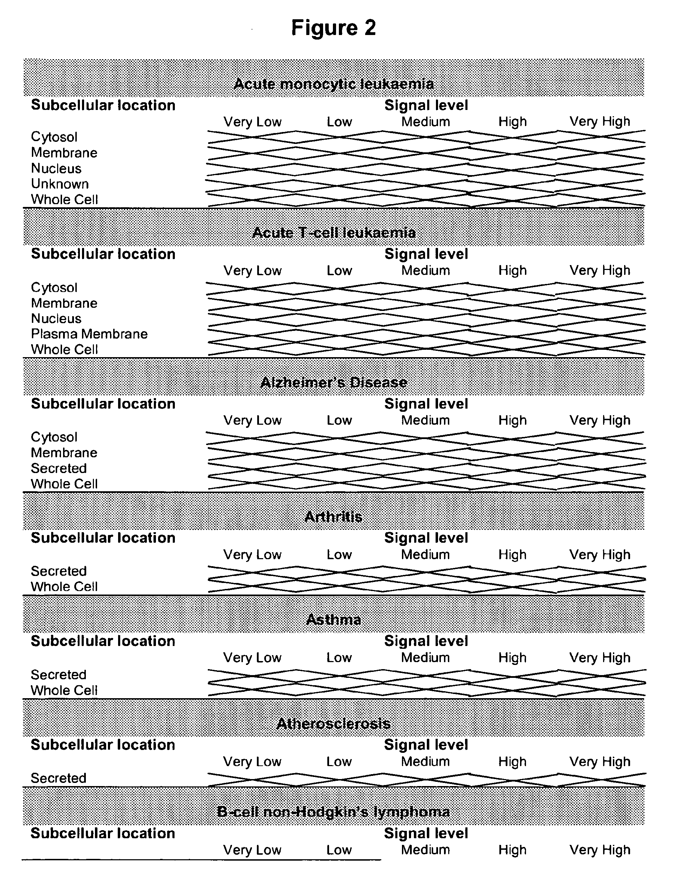

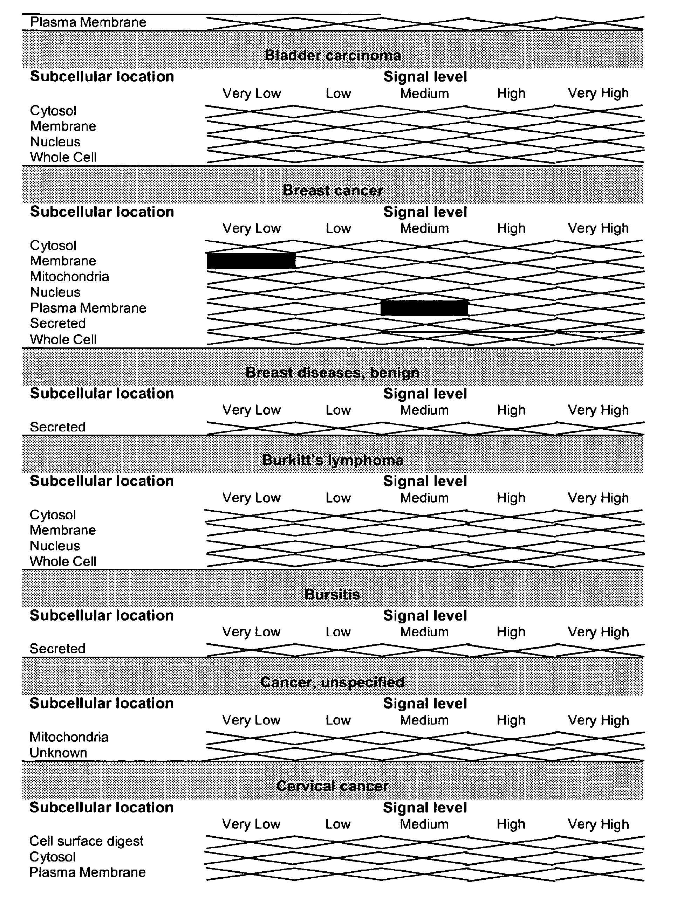

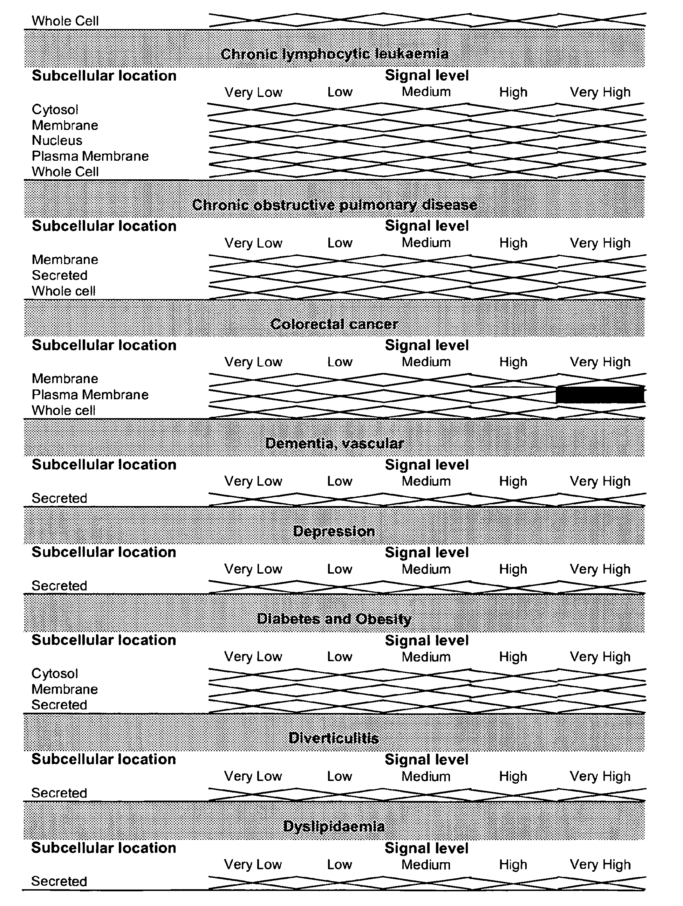

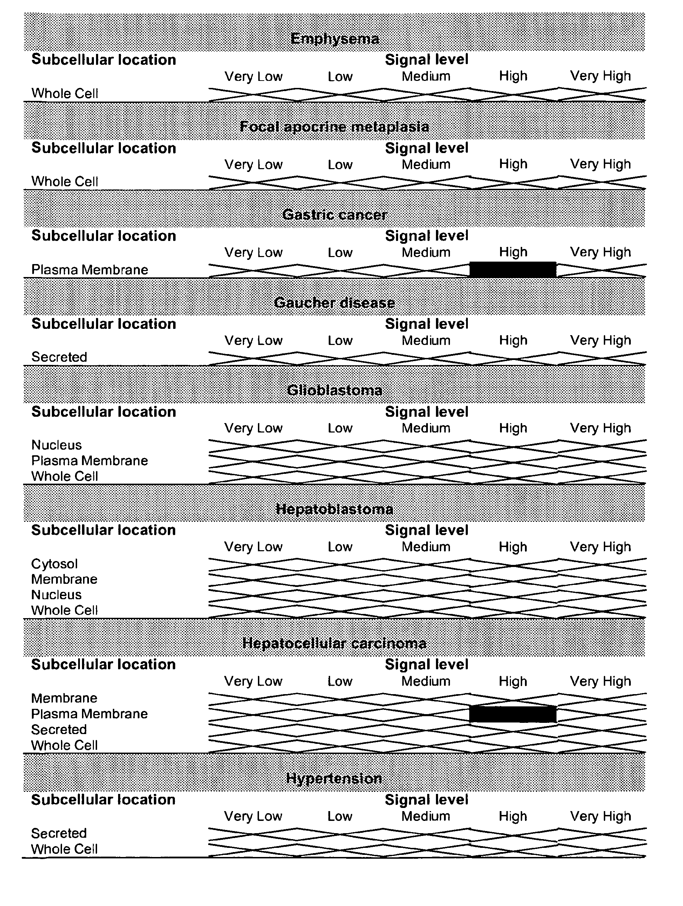

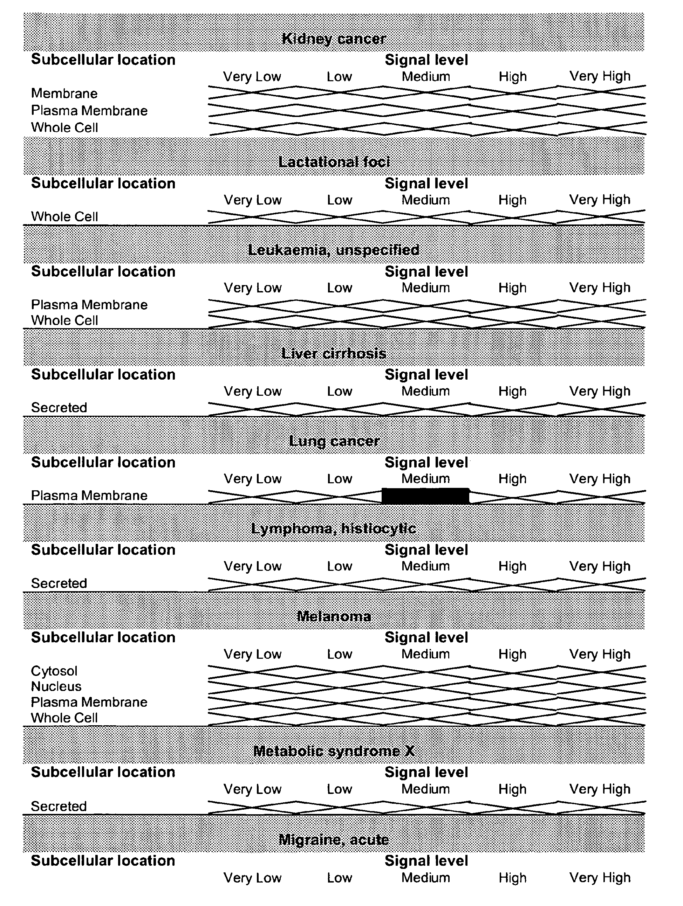

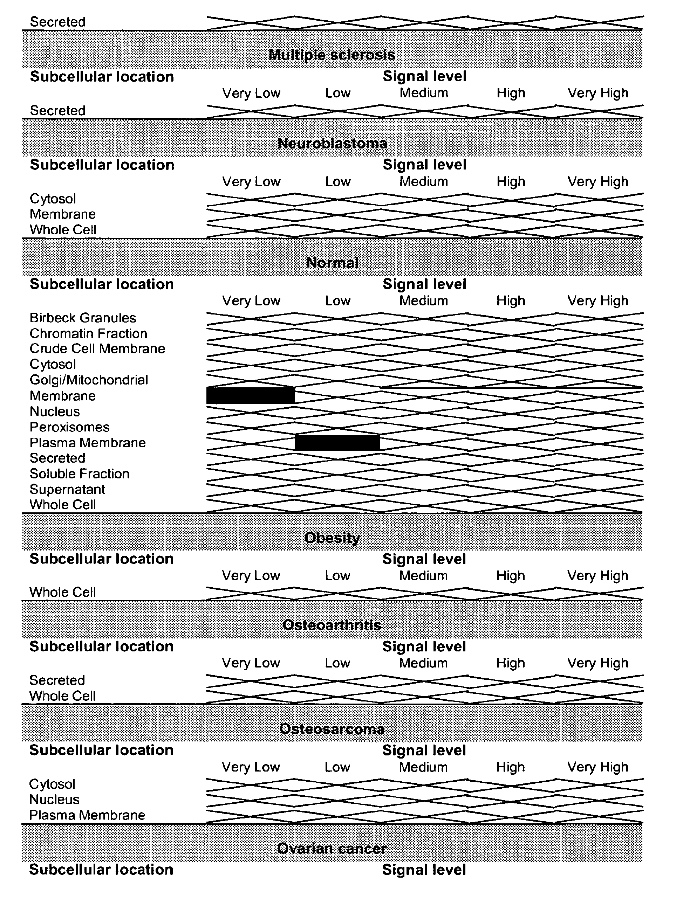

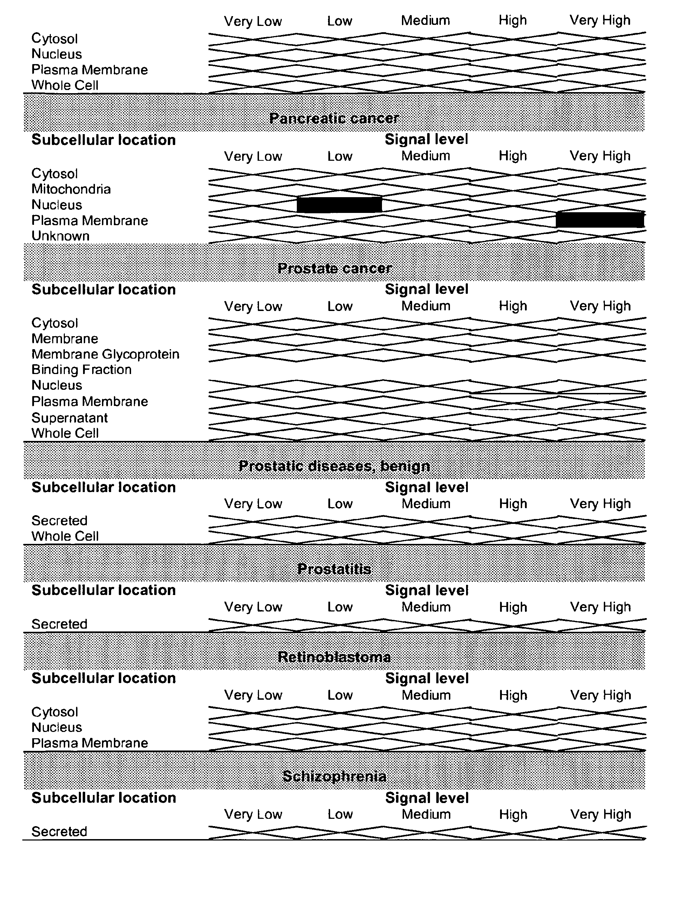

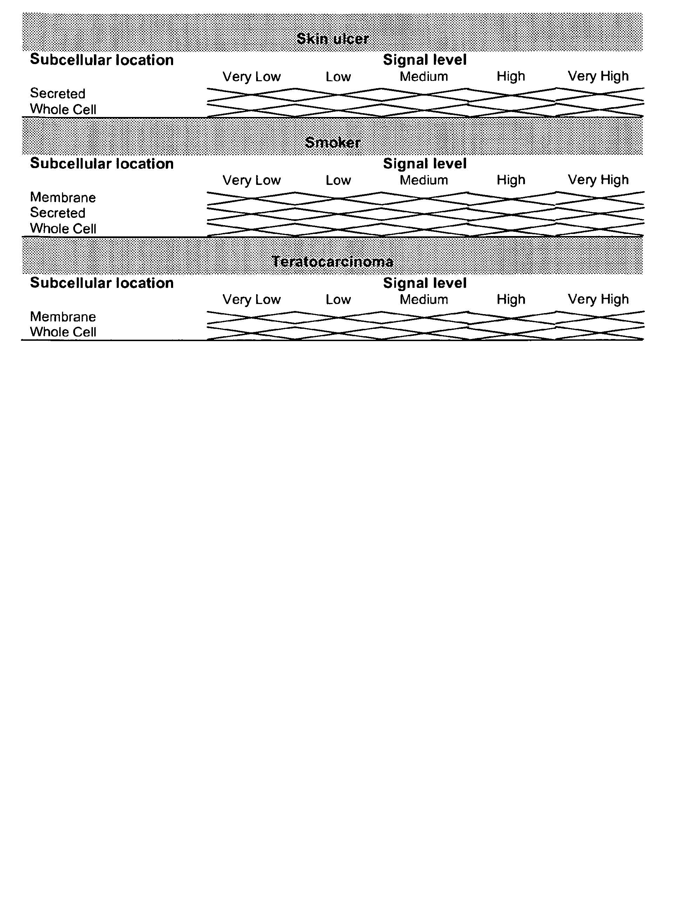

Figure 2 shows the Protein Index for Integrin beta 4. For each gene, the protein index uses the mass spectrometry data to assign a score to each disease, relative to the global database. The Protein Index can then be used to identify cancer specific genes with a high score in cancer indications and low/negligible scores in normal and other diseases. The index contains ~ 1 million peptides sequenced via mass spectrometry from 56 diseases. For each gene, this yields a score for each disease and subcellular location. The results are summarized below:

### Protein Index Report for Integrin beta 4

### Indications positive:

Breast cancer
Colorectal cancer
Gastric cancer
Hepatocellular carcinoma
Lung cancer
Pancreatic cancer

### Disease controls

| | | |
|---|---|---|
| Acute monocytic leukaemia | Diabetes and Obesity | Migraine, acute |
| Acute T-cell leukaemia | Diverticulitis | Multiple sclerosis |
| Alzheimer's Disease | Dyslipidaemia | Neuroblastoma |
| Arthritis | Emphysema | Normal |
| Asthma | Focal apocrine metaplasia | Obesity |
| Atherosclerosis | **Gastric cancer** | Osteoarthritis |
| B-cell non-Hodgkin's lymphoma | Gaucher disease | Osteosarcoma |
| Bladder carcinoma | Glioblastoma | Ovarian cancer |
| **Breast cancer** | Hepatoblastoma | **Pancreatic cancer** |
| Breast diseases, benign | **Hepatocellular carcinoma** | Prostate cancer |
| Burkitt's lymphoma | Hypertension | Prostatic diseases, benign |
| Bursitis | Kidney cancer | Prostatitis |
| Cancer, unspecified | Lactational foci | Retinoblastoma |
| Cervical cancer | Leukaemia, unspecified | Schizophrenia |
| Chronic lymphocytic leukaemia | Liver cirrhosis | Skin ulcer |
| Chronic obstructive pulmonary disease | **Lung cancer** | Smoker |
| **Colorectal cancer** | Lymphoma, histiocytic | Teratocarcinoma |
| Dementia, vascular | Melanoma | |
| Depression | Metabolic syndrome X | |

### Subcellular location

| | | |
|---|---|---|
| Birbeck Granules | **Membrane** | Secreted |
| | Membrane Glycoprotein | |
| Cell surface digest | Binding Fraction | Soluble Fraction |
| Chromatin Fraction | Mitochondria | Supernatant |
| Crude Cell Membrane | **Nucleus** | Whole Cell |
| Cytosol | Peroxisomes | |
| Golgi/Mitochondrial | **Plasma Membrane** | |

Figure 2 shows the Protein Index for Integrin beta 4 is medium in plasma membrane and very low in membrane for breast cancer, very high in colorectal cancer plasma membrane, high in gastric cancer plasma membrane, high in hepatocellular carcinoma plasma membrane, medium in lung cancer plasma membrane and very high in plasma membrane and low in nucleus for pancreatic cancer. It was also detected as very low in membrane and low in plasma membrane in normal samples. Integrin beta 4 was not detected in any other diseases. This indicates that Integrin beta 4 is potentially a good marker for breast cancer, colorectal cancer, gastric cancer, hepatocellular carcinoma, lung cancer and pancreatic cancer.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

**SEQUENCE LISTING**

| **Sequence** | **Sequence ID** |
|---|---|
| | 1 |
| | 2 |
| | |
| | 3 |
| | 4 |
| | 5 |
| ACLALLPCCNR | 6 |
| AEEVVVR | 7 |
| AFHDLK | 8 |
| ALEHVDGTHVCQLPEDQK | 9 |
| AQSQEGWGR | 10 |
| CERPLQGYSVEYQLLNGGELHR | 11 |
| DPDELDR | 12 |
| DVVSFEQPEFSVSR | 13 |
| DYIPVEGELLFQPGEAWK | 14 |
| DYNSLTR | 15 |
| DYSTLTSVSSHDSR | 16 |
| EAIINLATQPK | 17 |
| ECAQLR | 18 |
| EDHYMLR | 19 |
| EGEDKPCSGR | 20 |
| EGIITIESQDGGPFPQLGSR | 21 |
| ENLMASDHLDTPMLR | 22 |
| FEPLLGEELDLR | 23 |
| FEPLLGEELDLRR | 24 |
| FHVQLSNPK | 25 |
| GEVGIYQVQLR | 26 |
| GMVEFQEGVELVDVR | 27 |
| GNIHLKPSFSDGLK | 28 |
| HNIIPIFAVTNYSYSYYEK | 29 |
| HVTQEFVSR | 30 |
| IHFNWLPPSGKPMGYR | 31 |
| ISGNLDAPEGGFDAILQTAVCTR | 32 |
| KIHFNWLPPSGKPMGYR | 33 |
| LCTENLLKPDTR | 34 |
| LLELQEVDSLLR | 35 |
| LNIPNPAQTSWVEDLLPNHSYVFR | 36 |
| LTAGVPDTPTR | 37 |
| LTVPGLSENVPYK | 38 |
| LVFSALGPTSLR | 39 |
| MAGPRPSPWAR | 40 |
| MDAGIICDVCTCELQK | 41 |
| MDFAFPGSTNSLHR | 42 |
| MGQNLAR | 43 |
| MLLIENLR | 44 |
| MTTTSAAAYGTHLSPHVPHR | 45 |
| MVDELK | 46 |
| NGAGWGPER | 47 |
| NVISLTEDVDEFR | 48 |
| PSVSDDTEHLVNGR | 49 |
| QDHTIVDTVLMAPR | 50 |
| QEVEENLNEVYR | 51 |
| QISGVHK | 52 |
| QLLVEAIDVPAGTATLGR | 53 |
| QQPNAGK | 54 |
| RAEEVVVR | 55 |
| RCNTQAELLAAGCQR | 56 |
| RFHVQLSNPK | 57 |
| RGEVGIYQVQLR | 58 |
| RPNGDIVGYLVTCEMAQGGGPATAFR | 59 |
| RSQMSPQGLR | 60 |
| RVTWR | 61 |
| SATPGPPGEHLVNGR | 62 |
| SCVQCQAWGTGEK | 63 |
| SEHSHSTTLPR | 64 |
| SFTSQMLSSQPPPHGDLGAPQNPNAK | 65 |
| SNLDIR | 66 |
| SQMSPQGLR | 67 |
| SQVSYR | 68 |
| SSDAEAPHGPPDDGGAGGK | 69 |
| TGSFHIR | 70 |
| TGSFHIRR | 71 |
| THQEVPSEPGR | 72 |
| TLTTSGTLSTHMDQQFFQT | 73 |
| TQDYPSVPTLVR | 74 |
| TTEGFGPER | 75 |
| VAPGYYTLTADQDAR | 76 |
| VCAYGAQGEGPYSSLVSCR | 77 |
| VDGDSPESR | 78 |
| VLSTSSTLTR | 79 |
| VLVDNPK | 80 |
| VPLFIRPEDDDEK | 81 |
| VPSVELTNLYPYCDYEMK | 82 |
| VSVPQTDMRPEK | 83 |
| VSWQEPR | 84 |
| YWIQGDSESEAHLLDSK | 85 |

## Claims

1. A composition comprising an antibody capable of specific binding to Integrin beta 4 or a fragment thereof, and a pharmaceutically acceptable diluent or carrier, for use in treating or preventing breast cancer, colorectal cancer, gastric cancer, hepatocellular carcinoma, lung cancer or pancreatic cancer, wherein Integrin beta 4 is overexpressed in said cancers.

2. The composition according to claim 1 for use according to claim 1 wherein the antibody contains or is conjugated to a therapeutic moiety.

3. The composition according to claim 2 for use according to claim 1 wherein the therapeutic moiety is a cytotoxic moiety or a radioactive isotope.

4. The composition according to claim 1 for use according to claim 1 wherein the antibody contains or is conjugated to a detectable label.

5. The composition according to any one of claims 1 to 4 for use according to claim 1 wherein the antibody is an isolated monoclonal antibody, or an antigen-binding portion thereof, an antibody fragment, or an antibody mimetic.

6. The composition according to claim 5 for use according to claim 1 wherein the antibody is selected from the group consisting of: a full-length antibody of an IgG1, IgG2, IgG3, or IgG4 isotype, an antibody fragment, a humanised antibody, a single chain antibody, an immunoconjugate, a defucosylated antibody, and a bispecific antibody.

7. The composition according to claim 5 for use according to claim 1 wherein the antibody fragment is selected from the group consisting of: a UniBody, a domain antibody and a Nanobody.

8. The composition according to claim 5 for use according to claim 1 wherein the antibody mimetic is selected from the group consisting of: an Affibody, a DARPin, an Anticalin, an Avimer, a Versabody, and a Duocalin.

9. The composition according to claim 5 for use according to claim 1 wherein the monoclonal antibody has cytotoxicity against Integrin beta 4 antigen expressing cells in the presence of a human complement or in the presence of human immune effector cells.

10. Use an antibody capable of specific binding to Integrin beta 4 or a fragment thereof in the manufacture of a medicament for use in treating or preventing breast cancer, colorectal cancer, gastric cancer, hepatocellular carcinoma, lung cancer or pancreatic cancer, wherein Integrin beta 4 is overexpressed in said cancers.

## Patentansprüche

1. Zusammensetzung, umfassend einen Antikörper, der spezifisch an Integrin-beta 4 oder ein Fragment davon binden kann, und ein pharmazeutisch akzeptables Verdünnungsmittel oder einen pharmazeutisch akzeptablen Träger, zur Verwendung bei der Behandlung oder Vorbeugung von Brustkrebs, dem kolorektalen Karzinom, Magenkrebs, Leberzellenkarzinom, Lungenkrebs oder Bauchspeicheldrüsenkrebs, wobei Integrin-beta 4 bei diesen Krebsarten überexprimiert wird.

2. Zusammensetzung nach Anspruch 1 zur Verwendung nach Anspruch 1, wobei der Antikörper eine therapeutische Komponente enthält oder daran konjugiert ist.

3. Zusammensetzung nach Anspruch 2 zur Verwendung nach Anspruch 1, wobei die therapeutische Komponente eine zytotoxische Komponente oder ein radioaktives Isotop ist.

4. Zusammensetzung nach Anspruch 1 zur Verwendung nach Anspruch 1, wobei der Antikörper eine detektierbare Markierung enthält oder daran konjugiert ist.

5. Zusammensetzung nach einem der Ansprüche 1 bis 4 zur Verwendung nach Anspruch 1, wobei der Antikörper ein isolierter monoklonaler Antikörper oder ein Antigenbindender Teil davon, ein Antikörperfragment oder ein Antikörper-Mimetikum ist.

6. Zusammensetzung nach Anspruch 5 zur Verwendung nach Anspruch 1, wobei der Antikörper ausgewählt ist aus der Gruppe, bestehend aus: einem Volllängen-Antikörper eines IgG1-, IgG2-, IgG3- oder IgG4-Isotyps, einem Antikörperfragment, einem humanisierten Antikörper, einem Einkettenantikörper, einem Imznunkonjugat, einem defucosylierten Antikörper und einem bispezifischen Antikörper.

7. Zusammensetzung nach Anspruch 5 zur Verwendung nach Anspruch 1, wobei das Antikörperfragment ausgewählt ist aus der Gruppe, bestehend aus: einem UniBody, einem Domänen-Antikörper und einem Nanoantikörper.

8. Zusammensetzung nach Anspruch 5 zur Verwendung nach Anspruch 1, wobei das Antikörper-Mimetikum ausgewählt ist aus der Gruppe, bestehend aus: einem Affibody, einem DARPin, einem Anticalin, einem Avimer, einem Versabody und einem Duocalin.

9. Zusammensetzung nach Anspruch 5 zur Verwendung nach Anspruch 1, wobei der monoklonale Antikörper Zytotoxizität gegen Integrin-beta-4-Antigen-exprimierende Zellen in Gegenwart eines humanen Komplements oder in Gegenwart humaner Immuneffektorzellen aufweist.

10. Verwendung eines Antikörpers, der spezifisch an Integrin-beta 4 oder ein Fragment davon binden kann, bei der Herstellung eines Medikaments zur Verwendung bei der Behandlung oder Vorbeugung von Brustkrebs, dem kolorektalen Karzinom, Magenkrebs, Leberzellenkarzinom, Lungenkrebs oder Bauchspeicheldrüsenkrebs, wobei Integrin-beta 4 bei diesen Krebsarten überexprimiert wird.

## Revendications

1. Une composition comprenant un anticorps capable de se lier spécifiquement à l'intégrine beta 4 ou un fragment de celle-ci, et un diluant ou support pharmaceutiquement acceptable, destinée à être utilisée pour le traitement ou la prévention du cancer du sein, du cancer colorectal, du cancer gastrique, du carcinome hépatocellulaire, du cancer du poumon ou du cancer du pancréas, dans laquelle l'intégrine beta 4 est surexprimée dans lesdits cancers.

2. La composition selon la revendication 1, destinée à être utilisée conformément à la revendication 1, dans laquelle l'anticorps contient ou est conjugué à un groupement thérapeutique.

3. La composition selon la revendication 2, destinée à être utilisée conformément à la revendication 1, dans laquelle le groupement thérapeutique est un groupement cytotoxique ou un isotope radioactif.

4. La composition selon la revendication 1, destinée à être utilisée conformément à la revendication 1, dans laquelle l'anticorps contient ou est conjugué à un marqueur détectable.

5. La composition selon l'une quelconque des revendications 1 à 4, destinée à être utilisée conformément à la revendication 1, dans laquelle l'anticorps est un anticorps monoclonal isolé ou une partie de celui-ci liant l'antigène, un fragment d'anticorps, ou un anticorps mimétique.

6. La composition selon la revendication 5, destinée à être utilisée conformément à la revendication 1, dans laquelle l'anticorps est choisi parmi le groupe consistant en : un anticorps de pleine longueur d'un isotype d'IgG1, IgG2, IgG3 ou IgG4, un fragment d'anticorps, un anticorps humanisé, un anticorps simple chaîne, un immunoconjugué, un anticorps défucosylé, et un anticorps bispécifique.

7. La composition selon la revendication 5, destinée à être utilisée conformément à la revendication 1, dans laquelle le fragment d'anticorps est choisi dans le groupe consistant en : un UniBody, un anticorps à domaine et un Nanobody.

8. La composition selon la revendication 5, destinée à être utilisée conformément à la revendication 1, dans laquelle l'anticorps mimétique est choisi dans le groupe consistant en : un Affibody, un DARPin, un Anticalin, un Avimer, un Versabody, et un Duocalin.

9. La composition selon la revendication 5, destinée à être utilisée conformément à la revendication 1, dans laquelle l'anticorps monoclonal possède une cytotoxicité contre les cellules exprimant l'antigène intégrine beta 4 en présence d'un complément humain ou en présence de cellules effectrices immunitaires humaines.

10. Utilisation d'un anticorps capable de se lier spécifiquement à l'intégrine beta 4 ou un fragment de celle-ci, pour la fabrication d'un médicament destiné à être utilisé pour le traitement ou la prévention du cancer du sein, du cancer colorectal, du cancer gastrique, du carcinome hépatocellulaire, du cancer du poumon ou du cancer du pancréas, dans laquelle l'intégrine beta 4 est surexprimée dans lesdits cancers.


### SEQUENCE LISTING

<110> Oxford Genome Sciences (UK) Ltd Rohlff, Christian
<120> Protein
<130> OGL-P801PCT
<150> US 60/997,444
   <151> 2007-10-03
<150> GB0719231.3
   <151> 2007-10-03
<160> 85
<170> PatentIn version 3.5
<210> 1
   <211> 1822
   <212> PRT
   <213> Homo sapiens
<400> 1
<210> 2
   <211> 1752
   <212> PRT
   <213> Homo sapiens
<400> 2
   Met Ala Gly Pro Arg Pro Ser Pro Trp Ala Arg Leu Leu Leu Ala Ala
   1               5                   10                  15
   Leu Ile Ser Val Ser Leu Ser Gly Thr Leu Ala Asn Arg Cys Lys Lys
               20                  25                  30
   Ala Pro Val Lys Ser Cys Thr Glu Cys Val Arg Val Asp Lys Asp Cys
           35                  40                  45
   Ala Tyr Cys Thr Asp Glu Met Phe Arg Asp Arg Arg Cys Asn Thr Gln
       50                  55                  60
   Ala Glu Leu Leu Ala Ala Gly Cys Gln Arg Glu Ser Ile Val Val Met
   65                  70                  75                  80
   Glu Ser Ser Phe Gln Ile Thr Glu Glu Thr Gln Ile Asp Thr Thr Leu
                   85                  90                  95
   Arg Arg Ser Gln Met Ser Pro Gln Gly Leu Arg Val Arg Leu Arg Pro
               100                 105                 110
   Gly Glu Glu Arg His Phe Glu Leu Glu Val Phe Glu Pro Leu Glu Ser
           115                 120                 125
   Pro Val Asp Leu Tyr Ile Leu Met Asp Phe Ser Asn Ser Met Ser Asp
       130                 135                 140
   Asp Leu Asp Asn Leu Lys Lys Met Gly Gln Asn Leu Ala Arg Val Leu
   145                 150                 155                 160
   Ser Gln Leu Thr Ser Asp Tyr Thr Ile Gly Phe Gly Lys Phe Val Asp
                   165                 170                 175
   Lys Val Ser Val Pro Gln Thr Asp Met Arg Pro Glu Lys Leu Lys Glu
               180                 185                 190
   Pro Trp Pro Asn Ser Asp Pro Pro Phe Ser Phe Lys Asn Val Ile Ser
           195                 200                 205
   Leu Thr Glu Asp Val Asp Glu Phe Arg Asn Lys Leu Gln Gly Glu Arg
       210                 215                 220
   Ile Ser Gly Asn Leu Asp Ala Pro Glu Gly Gly Phe Asp Ala Ile Leu
   225                 230                 235                 240
   Gln Thr Ala Val Cys Thr Arg Asp Ile Gly Trp Arg Pro Asp Ser Thr
                   245                 250                 255
   His Leu Leu Val Phe Ser Thr Glu Ser Ala Phe His Tyr Glu Ala Asp
   260 265 270
   Gly Ala Asn Val Leu Ala Gly Ile Met Ser Arg Asn Asp Glu Arg Cys
   275 280 285
   His Leu Asp Thr Thr Gly Thr Tyr Thr Gln Tyr Arg Thr Gln Asp Tyr
   290 295 300
   Pro Ser Val Pro Thr Leu Val Arg Leu Leu Ala Lys His Asn Ile Ile
   305 310 315 320
   Pro Ile Phe Ala Val Thr Asn Tyr Ser Tyr Ser Tyr Tyr Glu Lys Leu
   325 330 335
   His Thr Tyr Phe Pro Val Ser Ser Leu Gly Val Leu Gln Glu Asp Ser
   340 345 350
   Ser Asn Ile Val Glu Leu Leu Glu Glu Ala Phe Asn Arg Ile Arg Ser
   355 360 365
   Asn Leu Asp Ile Arg Ala Leu Asp Ser Pro Arg Gly Leu Arg Thr Glu
   370 375 380
   Val Thr Ser Lys Met Phe Gln Lys Thr Arg Thr Gly Ser Phe His Ile
   385 390 395 400
   Arg Arg Gly Glu Val Gly Ile Tyr Gln Val Gln Leu Arg Ala Leu Glu
   405 410 415
   His Val Asp Gly Thr His Val Cys Gln Leu Pro Glu Asp Gln Lys Gly
   420 425 430
   Asn Ile His Leu Lys Pro Ser Phe Ser Asp Gly Leu Lys Met Asp Ala
   435 440 445
   Gly Ile Ile Cys Asp Val Cys Thr Cys Glu Leu Gln Lys Glu Val Arg
   450 455 460
   Ser Ala Arg Cys Ser Phe Asn Gly Asp Phe Val Cys Gly Gln Cys Val
   465 470 475 480
   Cys Ser Glu Gly Trp Ser Gly Gln Thr Cys Asn Cys Ser Thr Gly Ser
   485 490 495
   Leu Ser Asp Ile Gln Pro Cys Leu Arg Glu Gly Glu Asp Lys Pro Cys
                   500                 505                 510
   Ser Gly Arg Gly Glu Cys Gln Cys Gly His Cys Val Cys Tyr Gly Glu
               515                 520                 525
   Gly Arg Tyr Glu Gly Gln Phe Cys Glu Tyr Asp Asn Phe Gln Cys Pro
       530                 535                 540
   Arg Thr Ser Gly Phe Leu Cys Asn Asp Arg Gly Arg Cys Ser Met Gly
   545                 550                 555                 560
   Gln Cys Val Cys Glu Pro Gly Trp Thr Gly Pro Ser Cys Asp Cys Pro
                   565                 570                 575
   Leu Ser Asn Ala Thr Cys Ile Asp Ser Asn Gly Gly Ile Cys Asn Gly
               580                 585                 590
   Arg Gly His Cys Glu Cys Gly Arg Cys His Cys His Gln Gln Ser Leu
           595                 600                 605
   Tyr Thr Asp Thr Ile Cys Glu Ile Asn Tyr Ser Ala Ile His Pro Gly
       610                 615                 620
   Leu Cys Glu Asp Leu Arg Ser Cys Val Gln Cys Gln Ala Trp Gly Thr
   625                 630                 635                 640
   Gly Glu Lys Lys Gly Arg Thr Cys Glu Glu Cys Asn Phe Lys Val Lys
                   645                 650                 655
   Met Val Asp Glu Leu Lys Arg Ala Glu Glu Val Val Val Arg Cys Ser
               660                 665                 670
   Phe Arg Asp Glu Asp Asp Asp Cys Thr Tyr Ser Tyr Thr Met Glu Gly
           675                 680                 685
   Asp Gly Ala Pro Gly Pro Asn Ser Thr Val Leu Val His Lys Lys Lys
       690                 695                 700
   Asp Cys Pro Pro Gly Ser Phe Trp Trp Leu Ile Pro Leu Leu Leu Leu
   705                 710                 715                 720
   Leu Leu Pro Leu Leu Ala Leu Leu Leu Leu Leu Cys Trp Lys Tyr Cys
                   725                 730                 735
   Ala Cys Cys Lys Ala Cys Leu Ala Leu Leu Pro Cys Cys Asn Arg Gly
               740                 745                 750
   His Met Val Gly Phe Lys Glu Asp His Tyr Met Leu Arg Glu Asn Leu
           755                 760                 765
   Met Ala Ser Asp His Leu Asp Thr Pro Met Leu Arg Ser Gly Asn Leu
       770                 775                 780
   Lys Gly Arg Asp Val Val Arg Trp Lys Val Thr Asn Asn Met Gln Arg
   785                 790                 795                 800
   Pro Gly Phe Ala Thr His Ala Ala Ser Ile Asn Pro Thr Glu Leu Val
                   805                 810                 815
   Pro Tyr Gly Leu Ser Leu Arg Leu Ala Arg Leu Cys Thr Glu Asn Leu
               820                 825                 830
   Leu Lys Pro Asp Thr Arg Glu Cys Ala Gln Leu Arg Gln Glu Val Glu
           835                 840                 845
   Glu Asn Leu Asn Glu Val Tyr Arg Gln Ile Ser Gly Val His Lys Leu
       850                 855                 860
   Gln Gln Thr Lys Phe Arg Gln Gln Pro Asn Ala Gly Lys Lys Gln Asp
   865                 870                 875                 880
   His Thr Ile Val Asp Thr Val Leu Met Ala Pro Arg Ser Ala Lys Pro
                   885                 890                 895
   Ala Leu Leu Lys Leu Thr Glu Lys Gln Val Glu Gln Arg Ala Phe His
               900                 905                 910
   Asp Leu Lys Val Ala Pro Gly Tyr Tyr Thr Leu Thr Ala Asp Gln Asp
           915                 920                 925
   Ala Arg Gly Met Val Glu Phe Gln Glu Gly Val Glu Leu Val Asp Val
       930                 935                 940
   Arg Val Pro Leu Phe Ile Arg Pro Glu Asp Asp Asp Glu Lys Gln Leu
   945                 950                 955                 960
   Leu Val Glu Ala Ile Asp Val Pro Ala Gly Thr Ala Thr Leu Gly Arg
                   965                 970                 975
   Arg Leu Val Asn Ile Thr Ile Ile Lys Glu Gln Ala Arg Asp Val Val
               980                 985                 990
   Ser Phe Glu Gln Pro Glu Phe Ser  Val Ser Arg Gly Asp  Gln Val Ala
           995                 1000                1005
   Arg Ile Pro Val Ile Arg Arg Val Leu Asp Gly Gly Lys Ser Gln
   1010 1015 1020
   Val Ser Tyr Arg Thr Gln Asp Gly Thr Ala Gln Gly Asn Arg Asp
   1025 1030 1035
   Tyr Ile Pro Val Glu Gly Glu Leu Leu Phe Gln Pro Gly Glu Ala
   1040 1045 1050
   Trp Lys Glu Leu Gln Val Lys Leu Leu Glu Leu Gln Glu Val Asp
   1055 1060 1065
   Ser Leu Leu Arg Gly Arg Gln Val Arg Arg Phe His Val Gln Leu
   1070 1075 1080
   Ser Asn Pro Lys Phe Gly Ala His Leu Gly Gln Pro His Ser Thr
   1085 1090 1095
   Thr Ile Ile Ile Arg Asp Pro Asp Glu Leu Asp Arg Ser Phe Thr
   1100 1105 1110
   Ser Gln Met Leu Ser Ser Gln Pro Pro Pro His Gly Asp Leu Gly
   1115 1120 1125
   Ala Pro Gln Asn Pro Asn Ala Lys Ala Ala Gly Ser Arg Lys Ile
   1130 1135 1140
   His Phe Asn Trp Leu Pro Pro Ser Gly Lys Pro Met Gly Tyr Arg
   1145 1150 1155
   Val Lys Tyr Trp Ile Gln Gly Asp Ser Glu Ser Glu Ala His Leu
   1160 1165 1170
   Leu Asp Ser Lys Val Pro Ser Val Glu Leu Thr Asn Leu Tyr Pro
   1175 1180 1185
   Tyr Cys Asp Tyr Glu Met Lys Val Cys Ala Tyr Gly Ala Gln Gly
   1190 1195 1200
   Glu Gly Pro Tyr Ser Ser Leu Val Ser Cys Arg Thr His Gln Glu
   1205 1210 1215
   Val Pro Ser Glu Pro Gly Arg Leu Ala Phe Asn Val Val Ser Ser
   1220 1225 1230
   Thr Val Thr Gln Leu Ser Trp Ala Glu Pro Ala Glu Thr Asn Gly
   1235 1240 1245
   Glu Ile Thr Ala Tyr Glu Val Cys Tyr Gly Leu Val Asn Asp Asp
   1250 1255 1260
   Asn Arg Pro Ile Gly Pro Met Lys Lys Val Leu Val Asp Asn Pro
   1265 1270 1275
   Lys Asn Arg Met Leu Leu Ile Glu Asn Leu Arg Glu Ser Gln Pro
   1280 1285 1290
   Tyr Arg Tyr Thr Val Lys Ala Arg Asn Gly Ala Gly Trp Gly Pro
   1295 1300 1305
   Glu Arg Glu Ala Ile Ile Asn Leu Ala Thr Gln Pro Lys Arg Pro
   1310 1315 1320
   Met Ser Ile Pro Ile Ile Pro Asp Ile Pro Ile Val Asp Ala Gln
   1325 1330 1335
   Ser Gly Glu Asp Tyr Asp Ser Phe Leu Met Tyr Ser Asp Asp Val
   1340 1345 1350
   Leu Arg Ser Pro Ser Gly Ser Gln Arg Pro Ser Val Ser Asp Asp
   1355 1360 1365
   Thr Glu His Leu Val Asn Gly Arg Met Asp Phe Ala Phe Pro Gly
   1370 1375 1380
   Ser Thr Asn Ser Leu His Arg Met Thr Thr Thr Ser Ala Ala Ala
   1385 1390 1395
   Tyr Gly Thr His Leu Ser Pro His Val Pro His Arg Val Leu Ser
   1400 1405 1410
   Thr Ser Ser Thr Leu Thr Arg Asp Tyr Asn Ser Leu Thr Arg Ser
   1415 1420 1425
   Glu His Ser His Ser Thr Thr Leu Pro Arg Asp Tyr Ser Thr Leu
   1430 1435 1440
   Thr Ser Val Ser Ser His Asp Ser Arg Leu Thr Ala Gly Val Pro
   1445 1450 1455
   Asp Thr Pro Thr Arg Leu Val Phe Ser Ala Leu Gly Pro Thr Ser
   1460 1465 1470
   Leu Arg Val Ser Trp Gln Glu Pro Arg Cys Glu Arg Pro Leu Gln
   1475 1480 1485
   Gly Tyr Ser Val Glu Tyr Gln Leu Leu Asn Gly Gly Glu Leu His
   1490 1495 1500
   Arg Leu Asn Ile Pro Asn Pro Ala Gln Thr Ser Val Val Val Glu
   1505 1510 1515
   Asp Leu Leu Pro Asn His Ser Tyr Val Phe Arg Val Arg Ala Gln
   1520 1525 1530
   Ser Gln Glu Gly Trp Gly Arg Glu Arg Glu Gly Val Ile Thr Ile
   1535 1540 1545
   Glu Ser Gln Val His Pro Gln Ser Pro Leu Cys Pro Leu Pro Gly
   1550 1555 1560
   Ser Ala Phe Thr Leu Ser Thr Pro Ser Ala Pro Gly Pro Leu Val
   1565 1570 1575
   Phe Thr Ala Leu Ser Pro Asp Ser Leu Gln Leu Ser Trp Glu Arg
   1580 1585 1590
   Pro Arg Arg Pro Asn Gly Asp Ile Val Gly Tyr Leu Val Thr Cys
   1595 1600 1605
   Glu Met Ala Gln Gly Gly Gly Pro Ala Thr Ala Phe Arg Val Asp
   1610 1615 1620
   Gly Asp Ser Pro Glu Ser Arg Leu Thr Val Pro Gly Leu Ser Glu
   1625 1630 1635
   Asn Val Pro Tyr Lys Phe Lys Val Gln Ala Arg Thr Thr Glu Gly
   1640 1645 1650
   Phe Gly Pro Glu Arg Glu Gly Ile Ile Thr Ile Glu Ser Gln Asp
   1655 1660 1665
   Gly Gly Pro Phe Pro Gln Leu Gly Ser Arg Ala Gly Leu Phe Gln
   1670 1675 1680
   His Pro Leu Gln Ser Glu Tyr Ser Ser Ile Thr Thr Thr His Thr
   1685 1690 1695
   Ser Ala Thr Glu Pro Phe Leu Val Asp Gly Pro Thr Leu Gly Ala
   1700 1705 1710
   Gln His Leu Glu Ala Gly Gly Ser Leu Thr Arg His Val Thr Gln
   1715 1720 1725
   Glu Phe Val Ser Arg Thr Leu Thr Thr Ser Gly Thr Leu Ser Thr
   1730 1735 1740
   His Met Asp Gln Gln Phe Phe Gln Thr
   1745 1750
<210> 3
   <211> 1805
   <212> PRT
   <213> Homo sapiens
<400> 3
   Met Ala Gly Pro Arg Pro Ser Pro Trp Ala Arg Leu Leu Leu Ala Ala
   1 5 10 15
   Leu Ile Ser Val Ser Leu Ser Gly Thr Leu Ala Asn Arg Cys Lys Lys
   20 25 30
   Ala Pro Val Lys Ser Cys Thr Glu Cys Val Arg Val Asp Lys Asp Cys
   35 40 45
   Ala Tyr Cys Thr Asp Glu Met Phe Arg Asp Arg Arg Cys Asn Thr Gln
   50 55 60
   Ala Glu Leu Leu Ala Ala Gly Cys Gln Arg Glu Ser Ile Val Val Met
   65 70 75 80
   Glu Ser Ser Phe Gln Ile Thr Glu Glu Thr Gln Ile Asp Thr Thr Leu
   85 90 95
   Arg Arg Ser Gln Met Ser Pro Gln Gly Leu Arg Val Arg Leu Arg Pro
   100 105 110
   Gly Glu Glu Arg His Phe Glu Leu Glu Val Phe Glu Pro Leu Glu Ser
   115 120 125
   Pro Val Asp Leu Tyr Ile Leu Met Asp Phe Ser Asn Ser Met Ser Asp
   130 135 140
   Asp Leu Asp Asn Leu Lys Lys Met Gly Gln Asn Leu Ala Arg Val Leu
   145 150 155 160
   Ser Gln Leu Thr Ser Asp Tyr Thr Ile Gly Phe Gly Lys Phe Val Asp
   165 170 175
   Lys Val Ser Val Pro Gln Thr Asp Met Arg Pro Glu Lys Leu Lys Glu
               180                 185                 190
   Pro Trp Pro Asn Ser Asp Pro Pro Phe Ser Phe Lys Asn Val Ile Ser
           195                 200                 205
   Leu Thr Glu Asp Val Asp Glu Phe Arg Asn Lys Leu Gln Gly Glu Arg
       210                 215                 220
   Ile Ser Gly Asn Leu Asp Ala Pro Glu Gly Gly Phe Asp Ala Ile Leu
   225                 230                 235                 240
   Gln Thr Ala Val Cys Thr Arg Asp Ile Gly Trp Arg Pro Asp Ser Thr
                   245                 250                 255
   His Leu Leu Val Phe Ser Thr Glu Ser Ala Phe His Tyr Glu Ala Asp
               260                 265                 270
   Gly Ala Asn Val Leu Ala Gly Ile Met Ser Arg Asn Asp Glu Arg Cys
           275                 280                 285
   His Leu Asp Thr Thr Gly Thr Tyr Thr Gln Tyr Arg Thr Gln Asp Tyr
       290                 295                 300
   Pro Ser Val Pro Thr Leu Val Arg Leu Leu Ala Lys His Asn Ile Ile
   305                 310                 315                 320
   Pro Ile Phe Ala Val Thr Asn Tyr Ser Tyr Ser Tyr Tyr Glu Lys Leu
                   325                 330                 335
   His Thr Tyr Phe Pro Val Ser Ser Leu Gly Val Leu Gln Glu Asp Ser
               340                 345                 350
   Ser Asn Ile Val Glu Leu Leu Glu Glu Ala Phe Asn Arg Ile Arg Ser
           355                 360                 365
   Asn Leu Asp Ile Arg Ala Leu Asp Ser Pro Arg Gly Leu Arg Thr Glu
       370                 375                 380
   Val Thr Ser Lys Met Phe Gln Lys Thr Arg Thr Gly Ser Phe His Ile
   385                 390                 395                 400
   Arg Arg Gly Glu Val Gly Ile Tyr Gln Val Gln Leu Arg Ala Leu Glu
                   405                 410                 415
   His Val Asp Gly Thr His Val Cys Gln Leu Pro Glu Asp Gln Lys Gly
               420                 425                 430
   Asn Ile His Leu Lys Pro Ser Phe Ser Asp Gly Leu Lys Met Asp Ala
   435 440 445
   Gly Ile Ile Cys Asp Val Cys Thr Cys Glu Leu Gln Lys Glu Val Arg
   450 455 460
   Ser Ala Arg Cys Ser Phe Asn Gly Asp Phe Val Cys Gly Gln Cys Val
   465 470 475 480
   Cys Ser Glu Gly Trp Ser Gly Gln Thr Cys Asn Cys Ser Thr Gly Ser
   485 490 495
   Leu Ser Asp Ile Gln Pro Cys Leu Arg Glu Gly Glu Asp Lys Pro Cys
   500 505 510
   Ser Gly Arg Gly Glu Cys Gln Cys Gly His Cys Val Cys Tyr Gly Glu
   515 520 525
   Gly Arg Tyr Glu Gly Gln Phe Cys Glu Tyr Asp Asn Phe Gln Cys Pro
   530 535 540
   Arg Thr Ser Gly Phe Leu Cys Asn Asp Arg Gly Arg Cys Ser Met Gly
   545 550 555 560
   Gln Cys Val Cys Glu Pro Gly Trp Thr Gly Pro Ser Cys Asp Cys Pro
   565 570 575
   Leu Ser Asn Ala Thr Cys Ile Asp Ser Asn Gly Gly Ile Cys Asn Gly
   580 585 590
   Arg Gly His Cys Glu Cys Gly Arg Cys His Cys His Gln Gln Ser Leu
   595 600 605
   Tyr Thr Asp Thr Ile Cys Glu Ile Asn Tyr Ser Ala Ile His Pro Gly
   610 615 620
   Leu Cys Glu Asp Leu Arg Ser Cys Val Gln Cys Gln Ala Trp Gly Thr
   625 630 635 640
   Gly Glu Lys Lys Gly Arg Thr Cys Glu Glu Cys Asn Phe Lys Val Lys
   645 650 655
   Met Val Asp Glu Leu Lys Arg Ala Glu Glu Val Val Val Arg Cys Ser
   660 665 670
   Phe Arg Asp Glu Asp Asp Asp Cys Thr Tyr Ser Tyr Thr Met Glu Gly
   675 680 685
   Asp Gly Ala Pro Gly Pro Asn Ser Thr Val Leu Val His Lys Lys Lys
   690 695 700
   Asp Cys Pro Pro Gly Ser Phe Trp Trp Leu Ile Pro Leu Leu Leu Leu
   705 710 715 720
   Leu Leu Pro Leu Leu Ala Leu Leu Leu Leu Leu Cys Trp Lys Tyr Cys
   725 730 735
   Ala Cys Cys Lys Ala Cys Leu Ala Leu Leu Pro Cys Cys Asn Arg Gly
   740 745 750
   His Met Val Gly Phe Lys Glu Asp His Tyr Met Leu Arg Glu Asn Leu
   755 760 765
   Met Ala Ser Asp His Leu Asp Thr Pro Met Leu Arg Ser Gly Asn Leu
   770 775 780
   Lys Gly Arg Asp Val Val Arg Trp Lys Val Thr Asn Asn Met Gln Arg
   785 790 795 800
   Pro Gly Phe Ala Thr His Ala Ala Ser Ile Asn Pro Thr Glu Leu Val
   805 810 815
   Pro Tyr Gly Leu Ser Leu Arg Leu Ala Arg Leu Cys Thr Glu Asn Leu
   820 825 830
   Leu Lys Pro Asp Thr Arg Glu Cys Ala Gln Leu Arg Gln Glu Val Glu
   835 840 845
   Glu Asn Leu Asn Glu Val Tyr Arg Gln Ile Ser Gly Val His Lys Leu
   850 855 860
   Gln Gln Thr Lys Phe Arg Gln Gln Pro Asn Ala Gly Lys Lys Gln Asp
   865 870 875 880
   His Thr Ile Val Asp Thr Val Leu Met Ala Pro Arg Ser Ala Lys Pro
   885 890 895
   Ala Leu Leu Lys Leu Thr Glu Lys Gln Val Glu Gln Arg Ala Phe His
   900 905 910
   Asp Leu Lys Val Ala Pro Gly Tyr Tyr Thr Leu Thr Ala Asp Gln Asp
   915 920 925
   Ala Arg Gly Met Val Glu Phe Gln Glu Gly Val Glu Leu Val Asp Val
   930 935 940
   Arg Val Pro Leu Phe Ile Arg Pro Glu Asp Asp Asp Glu Lys Gln Leu
   945 950 955 960
   Leu Val Glu Ala Ile Asp Val Pro Ala Gly Thr Ala Thr Leu Gly Arg
   965 970 975
   Arg Leu Val Asn Ile Thr Ile Ile Lys Glu Gln Ala Arg Asp Val Val
   980 985 990
   Ser Phe Glu Gln Pro Glu Phe Ser Val Ser Arg Gly Asp Gln Val Ala
   995 1000 1005
   Arg Ile Pro Val Ile Arg Arg Val Leu Asp Gly Gly Lys Ser Gln
   1010 1015 1020
   Val Ser Tyr Arg Thr Gln Asp Gly Thr Ala Gln Gly Asn Arg Asp
   1025 1030 1035
   Tyr Ile Pro Val Glu Gly Glu Leu Leu Phe Gln Pro Gly Glu Ala
   1040 1045 1050
   Trp Lys Glu Leu Gln Val Lys Leu Leu Glu Leu Gln Glu Val Asp
   1055 1060 1065
   Ser Leu Leu Arg Gly Arg Gln Val Arg Arg Phe His Val Gln Leu
   1070 1075 1080
   Ser Asn Pro Lys Phe Gly Ala His Leu Gly Gln Pro His Ser Thr
   1085 1090 1095
   Thr Ile Ile Ile Arg Asp Pro Asp Glu Leu Asp Arg Ser Phe Thr
   1100 1105 1110
   Ser Gln Met Leu Ser Ser Gln Pro Pro Pro His Gly Asp Leu Gly
   1115 1120 1125
   Ala Pro Gln Asn Pro Asn Ala Lys Ala Ala Gly Ser Arg Lys Ile
   1130 1135 1140
   His Phe Asn Trp Leu Pro Pro Ser Gly Lys Pro Met Gly Tyr Arg
   1145 1150 1155
   Val Lys Tyr Trp Ile Gln Gly Asp Ser Glu Ser Glu Ala His Leu
   1160 1165 1170
   Leu Asp Ser Lys Val Pro Ser Val Glu Leu Thr Asn Leu Tyr Pro
   1175 1180 1185
   Tyr Cys Asp Tyr Glu Met Lys Val Cys Ala Tyr Gly Ala Gln Gly
   1190 1195 1200
   Glu Gly Pro Tyr Ser Ser Leu Val Ser Cys Arg Thr His Gln Glu
   1205 1210 1215
   Val Pro Ser Glu Pro Gly Arg Leu Ala Phe Asn Val Val Ser Ser
   1220 1225 1230
   Thr Val Thr Gln Leu Ser Trp Ala Glu Pro Ala Glu Thr Asn Gly
   1235 1240 1245
   Glu Ile Thr Ala Tyr Glu Val Cys Tyr Gly Leu Val Asn Asp Asp
   1250 1255 1260
   Asn Arg Pro Ile Gly Pro Met Lys Lys Val Leu Val Asp Asn Pro
   1265 1270 1275
   Lys Asn Arg Met Leu Leu Ile Glu Asn Leu Arg Glu Ser Gln Pro
   1280 1285 1290
   Tyr Arg Tyr Thr Val Lys Ala Arg Asn Gly Ala Gly Trp Gly Pro
   1295 1300 1305
   Glu Arg Glu Ala Ile Ile Asn Leu Ala Thr Gln Pro Lys Arg Pro
   1310 1315 1320
   Met Ser Ile Pro Ile Ile Pro Asp Ile Pro Ile Val Asp Ala Gln
   1325 1330 1335
   Ser Gly Glu Asp Tyr Asp Ser Phe Leu Met Tyr Ser Asp Asp Val
   1340 1345 1350
   Leu Arg Ser Pro Ser Gly Ser Gln Arg Pro Ser Val Ser Asp Asp
   1355 1360 1365
   Thr Glu His Leu Val Asn Gly Arg Met Asp Phe Ala Phe Pro Gly
   1370 1375 1380
   Ser Thr Asn Ser Leu His Arg Met Thr Thr Thr Ser Ala Ala Ala
   1385 1390 1395
   Tyr Gly Thr His Leu Ser Pro His Val Pro His Arg Val Leu Ser
   1400 1405 1410
   Thr Ser Ser Thr Leu Thr Arg Asp Tyr Asn Ser Leu Thr Arg Ser
   1415 1420 1425
   Glu His Ser His Ser Thr Thr Leu Pro Arg Asp Tyr Ser Thr Leu
   1430 1435 1440
   Thr Ser Val Ser Ser His Gly Leu Pro Pro Ile Trp Glu His Gly
   1445 1450 1455
   Arg Ser Arg Leu Pro Leu Ser Trp Ala Leu Gly Ser Arg Ser Arg
   1460 1465 1470
   Ala Gln Met Lys Gly Phe Pro Pro Ser Arg Gly Pro Arg Asp Ser
   1475 1480 1485
   Ile Ile Leu Ala Gly Arg Pro Ala Ala Pro Ser Trp Gly Pro Asp
   1490 1495 1500
   Ser Arg Leu Thr Ala Gly Val Pro Asp Thr Pro Thr Arg Leu Val
   1505 1510 1515
   Phe Ser Ala Leu Gly Pro Thr Ser Leu Arg Val Ser Trp Gln Glu
   1520 1525 1530
   Pro Arg Cys Glu Arg Pro Leu Gln Gly Tyr Ser Val Glu Tyr Gln
   1535 1540 1545
   Leu Leu Asn Gly Gly Glu Leu His Arg Leu Asn Ile Pro Asn Pro
   1550 1555 1560
   Ala Gln Thr Ser Val Val Val Glu Asp Leu Leu Pro Asn His Ser
   1565 1570 1575
   Tyr Val Phe Arg Val Arg Ala Gln Ser Gln Glu Gly Trp Gly Arg
   1580 1585 1590
   Glu Arg Glu Gly Val Ile Thr Ile Glu Ser Gln Val His Pro Gln
   1595 1600 1605
   Ser Pro Leu Cys Pro Leu Pro Gly Ser Ala Phe Thr Leu Ser Thr
   1610 1615 1620
   Pro Ser Ala Pro Gly Pro Leu Val Phe Thr Ala Leu Ser Pro Asp
   1625 1630 1635
   Ser Leu Gln Leu Ser Trp Glu Arg Pro Arg Arg Pro Asn Gly Asp
   1640 1645 1650
   Ile Val Gly Tyr Leu Val Thr Cys Glu Met Ala Gln Gly Gly Gly
   1655 1660 1665
   Pro Ala Thr Ala Phe Arg Val Asp Gly Asp Ser Pro Glu Ser Arg
   1670 1675 1680
   Leu Thr Val Pro Gly Leu Ser Glu Asn Val Pro Tyr Lys Phe Lys
   1685 1690 1695
   Val Gln Ala Arg Thr Thr Glu Gly Phe Gly Pro Glu Arg Glu Gly
   1700 1705 1710
   Ile Ile Thr Ile Glu Ser Gln Asp Gly Gly Pro Phe Pro Gln Leu
   1715 1720 1725
   Gly Ser Arg Ala Gly Leu Phe Gln His Pro Leu Gln Ser Glu Tyr
   1730 1735 1740
   Ser Ser Ile Thr Thr Thr His Thr Ser Ala Thr Glu Pro Phe Leu
   1745 1750 1755
   Val Asp Gly Pro Thr Leu Gly Ala Gln His Leu Glu Ala Gly Gly
   1760 1765 1770
   Ser Leu Thr Arg His Val Thr Gln Glu Phe Val Ser Arg Thr Leu
   1775 1780 1785
   Thr Thr Ser Gly Thr Leu Ser Thr His Met Asp Gln Gln Phe Phe
   1790 1795 1800
   Gln Thr
   1805
<210> 4
   <211> 1745
   <212> PRT
   <213> Homo sapiens
<400> 4
   Met Ala Gly Pro Arg Pro Ser Pro Trp Ala Arg Leu Leu Leu Ala Ala
   1 5 10 15
   Leu Ile Ser Val Ser Leu Ser Gly Thr Leu Ala Asn Arg Cys Lys Lys
   20 25 30
   Ala Pro Val Lys Ser Cys Thr Glu Cys Val Arg Val Asp Lys Asp Cys
   35 40 45
   Ala Tyr Cys Thr Asp Glu Met Phe Arg Asp Arg Arg Cys Asn Thr Gln
       50                  55                  60
   Ala Glu Leu Leu Ala Ala Gly Cys Gln Arg Glu Ser Ile Val Val Met
   65                  70                  75                  80
   Glu Ser Ser Phe Gln Ile Thr Glu Glu Thr Gln Ile Asp Thr Thr Leu
                   85                  90                  95
   Arg Arg Ser Gln Met Ser Pro Gln Gly Leu Arg Val Arg Leu Arg Pro
               100                 105                 110
   Gly Glu Glu Arg His Phe Glu Leu Glu Val Phe Glu Pro Leu Glu Ser
           115                 120                 125
   Pro Val Asp Leu Tyr Ile Leu Met Asp Phe Ser Asn Ser Met Ser Asp
       130                 135                 140
   Asp Leu Asp Asn Leu Lys Lys Met Gly Gln Asn Leu Ala Arg Val Leu
   145                 150                 155                 160
   Ser Gln Leu Thr Ser Asp Tyr Thr Ile Gly Phe Gly Lys Phe Val Asp
                   165                 170                 175
   Lys Val Ser Val Pro Gln Thr Asp Met Arg Pro Glu Lys Leu Lys Glu
               180                 185                 190
   Pro Trp Pro Asn Ser Asp Pro Pro Phe Ser Phe Lys Asn Val Ile Ser
           195                 200                 205
   Leu Thr Glu Asp Val Asp Glu Phe Arg Asn Lys Leu Gln Gly Glu Arg
       210                 215                 220
   Ile Ser Gly Asn Leu Asp Ala Pro Glu Gly Gly Phe Asp Ala Ile Leu
   225                 230                 235                 240
   Gln Thr Ala Val Cys Thr Arg Asp Ile Gly Trp Arg Pro Asp Ser Thr
                   245                 250                 255
   His Leu Leu Val Phe Ser Thr Glu Ser Ala Phe His Tyr Glu Ala Asp
               260                 265                 270
   Gly Ala Asn Val Leu Ala Gly Ile Met Ser Arg Asn Asp Glu Arg Cys
           275                 280                 285
   His Leu Asp Thr Thr Gly Thr Tyr Thr Gln Tyr Arg Thr Gln Asp Tyr
           290                 295                 300
   Pro Ser Val Pro Thr Leu Val Arg Leu Leu Ala Lys His Asn Ile Ile
   305                 310                 315                 320
   Pro Ile Phe Ala Val Thr Asn Tyr Ser Tyr Ser Tyr Tyr Glu Lys Leu
                   325                 330                 335
   His Thr Tyr Phe Pro Val Ser Ser Leu Gly Val Leu Gln Glu Asp Ser
               340                 345                 350
   Ser Asn Ile Val Glu Leu Leu Glu Glu Ala Phe Asn Arg Ile Arg Ser
           355                 360                 365
   Asn Leu Asp Ile Arg Ala Leu Asp Ser Pro Arg Gly Leu Arg Thr Glu
       370                 375                 380
   Val Thr Ser Lys Met Phe Gln Lys Thr Arg Thr Gly Ser Phe His Ile
   385                 390                 395                 400
   Arg Arg Gly Glu Val Gly Ile Tyr Gln Val Gln Leu Arg Ala Leu Glu
                   405                 410                 415
   His Val Asp Gly Thr His Val Cys Gln Leu Pro Glu Asp Gln Lys Gly
               420                 425                 430
   Asn Ile His Leu Lys Pro Ser Phe Ser Asp Gly Leu Lys Met Asp Ala
           435                 440                 445
   Gly Ile Ile Cys Asp Val Cys Thr Cys Glu Leu Gln Lys Glu Val Arg
       450                 455                 460
   Ser Ala Arg Cys Ser Phe Asn Gly Asp Phe Val Cys Gly Gln Cys Val
   465                 470                 475                 480
   Cys Ser Glu Gly Trp Ser Gly Gln Thr Cys Asn Cys Ser Thr Gly Ser
                   485                 490                 495
   Leu Ser Asp Ile Gln Pro Cys Leu Arg Glu Gly Glu Asp Lys Pro Cys
               500                 505                 510
   Ser Gly Arg Gly Glu Cys Gln Cys Gly His Cys Val Cys Tyr Gly Glu
           515                 520                 525
   Gly Arg Tyr Glu Gly Gln Phe Cys Glu Tyr Asp Asn Phe Gln Cys Pro
       530                 535                 540
   Arg Thr Ser Gly Phe Leu Cys Asn Asp Arg Gly Arg Cys Ser Met Gly
   545                 550                 555                 560
   Gln Cys Val Cys Glu Pro Gly Trp Thr Gly Pro Ser Cys Asp Cys Pro
                   565                 570                 575
   Leu Ser Asn Ala Thr Cys Ile Asp Ser Asn Gly Gly Ile Cys Asn Gly
               580                 585                 590
   Arg Gly His Cys Glu Cys Gly Arg Cys His Cys His Gln Gln Ser Leu
           595                 600                 605
   Tyr Thr Asp Thr Ile Cys Glu Ile Asn Tyr Ser Ala Ile His Pro Gly
       610                 615                 620
   Leu Cys Glu Asp Leu Arg Ser Cys Val Gln Cys Gln Ala Trp Gly Thr
   625                 630                 635                 640
   Gly Glu Lys Lys Gly Arg Thr Cys Glu Glu Cys Asn Phe Lys Val Lys
                   645                 650                 655
   Met Val Asp Glu Leu Lys Arg Ala Glu Glu Val Val Val Arg Cys Ser
               660                 665                 670
   Phe Arg Asp Glu Asp Asp Asp Cys Thr Tyr Ser Tyr Thr Met Glu Gly
           675                 680                 685
   Asp Gly Ala Pro Gly Pro Asn Ser Thr Val Leu Val His Lys Lys Lys
       690                 695                 700
   Asp Cys Pro Pro Gly Ser Phe Trp Trp Leu Ile Pro Leu Leu Leu Leu
   705                 710                 715                 720
   Leu Leu Pro Leu Leu Ala Leu Leu Leu Leu Leu Cys Trp Lys Tyr Cys
                   725                 730                 735
   Ala Cys Cys Lys Ala Cys Leu Ala Leu Leu Pro Cys Cys Asn Arg Gly
               740                 745                 750
   His Met Val Gly Phe Lys Glu Asp His Tyr Met Leu Arg Glu Asn Leu
           755                 760                 765
   Met Ala Ser Asp His Leu Asp Thr Pro Met Leu Arg Ser Gly Asn Leu
       770                 775                 780
   Lys Gly Arg Asp Val Val Arg Trp Lys Val Thr Asn Asn Met Gln Arg
   785                 790                 795                 800
   Pro Gly Phe Ala Thr His Ala Ala Ser Ile Asn Pro Thr Glu Leu Val
                   805                 810                 815
   Pro Tyr Gly Leu Ser Leu Arg Leu Ala Arg Leu Cys Thr Glu Asn Leu
               820                 825                 830
   Leu Lys Pro Asp Thr Arg Glu Cys Ala Gln Leu Arg Gln Glu Val Glu
           835                 840                 845
   Glu Asn Leu Asn Glu Val Tyr Arg Gln Ile Ser Gly Val His Lys Leu
       850                 855                 860
   Gln Gln Thr Lys Phe Arg Gln Gln Pro Asn Ala Gly Lys Lys Gln Asp
   865                 870                 875                 880
   His Thr Ile Val Asp Thr Val Leu Met Ala Pro Arg Ser Ala Lys Pro
                   885                 890                 895
   Ala Leu Leu Lys Leu Thr Glu Lys Gln Val Glu Gln Arg Ala Phe His
               900                 905                 910
   Asp Leu Lys Val Ala Pro Gly Tyr Tyr Thr Leu Thr Ala Asp Gln Asp
           915                 920                 925
   Ala Arg Gly Met Val Glu Phe Gln Glu Gly Val Glu Leu Val Asp Val
       930                 935                 940
   Arg Val Pro Leu Phe Ile Arg Pro Glu Asp Asp Asp Glu Lys Gln Leu
   945                 950                 955                 960
   Leu Val Glu Ala Ile Asp Val Pro Ala Gly Thr Ala Thr Leu Gly Arg
                   965                 970                 975
   Arg Leu Val Asn Ile Thr Ile Ile Lys Glu Gln Ala Arg Asp Val Val
               980                 985                 990
   Ser Phe Glu Gln Pro Glu Phe Ser  Val Ser Arg Gly Asp  Gln Val Ala
           995                 1000                 1005
   Arg Ile  Pro Val Ile Arg Arg  Val Leu Asp Gly Gly  Lys Ser Gln
       1010                 1015                 1020
   Val Ser  Tyr Arg Thr Gln Asp  Gly Thr Ala Gln Gly  Asn Arg Asp
       1025                 1030                 1035
   Tyr Ile  Pro Val Glu Gly Glu  Leu Leu Phe Gln Pro  Gly Glu Ala
       1040                 1045                 1050
   Trp Lys Glu Leu Gln Val Lys Leu Leu Glu Leu Gln Glu Val Asp
   1055 1060 1065
   Ser Leu Leu Arg Gly Arg Gln Val Arg Arg Phe His Val Gln Leu
   1070 1075 1080
   Ser Asn Pro Lys Phe Gly Ala His Leu Gly Gln Pro His Ser Thr
   1085 1090 1095
   Thr Ile Ile Ile Arg Asp Pro Asp Glu Leu Asp Arg Ser Phe Thr
   1100 1105 1110
   Ser Gln Met Leu Ser Ser Gln Pro Pro Pro His Gly Asp Leu Gly
   1115 1120 1125
   Ala Pro Gln Asn Pro Asn Ala Lys Ala Ala Gly Ser Arg Lys Ile
   1130 1135 1140
   His Phe Asn Trp Leu Pro Pro Ser Gly Lys Pro Met Gly Tyr Arg
   1145 1150 1155
   Val Lys Tyr Trp Ile Gln Gly Asp Ser Glu Ser Glu Ala His Leu
   1160 1165 1170
   Leu Asp Ser Lys Val Pro Ser Val Glu Leu Thr Asn Leu Tyr Pro
   1175 1180 1185
   Tyr Cys Asp Tyr Glu Met Lys Val Cys Ala Tyr Gly Ala Gln Gly
   1190 1195 1200
   Glu Gly Pro Tyr Ser Ser Leu Val Ser Cys Arg Thr His Gln Glu
   1205 1210 1215
   Val Pro Ser Glu Pro Gly Arg Leu Ala Phe Asn Val Val Ser Ser
   1220 1225 1230
   Thr Val Thr Gln Leu Ser Trp Ala Glu Pro Ala Glu Thr Asn Gly
   1235 1240 1245
   Glu Ile Thr Ala Tyr Glu Val Cys Tyr Gly Leu Val Asn Asp Asp
   1250 1255 1260
   Asn Arg Pro Ile Gly Pro Met Lys Lys Val Leu Val Asp Asn Pro
   1265 1270 1275
   Lys Asn Arg Met Leu Leu Ile Glu Asn Leu Arg Glu Ser Gln Pro
   1280 1285 1290
   Tyr Arg Tyr Thr Val Lys Ala Arg Asn Gly Ala Gly Trp Gly Pro
   1295 1300 1305
   Glu Arg Glu Ala Ile Ile Asn Leu Ala Thr Gln Pro Lys Arg Pro
   1310 1315 1320
   Met Ser Ile Pro Ile Ile Pro Asp Ile Pro Ile Val Asp Ala Gln
   1325 1330 1335
   Ser Gly Glu Asp Tyr Asp Ser Phe Leu Met Tyr Ser Asp Asp Val
   1340 1345 1350
   Leu Arg Ser Pro Ser Gly Ser Gln Arg Pro Ser Val Ser Asp Asp
   1355 1360 1365
   Thr Glu His Leu Val Asn Gly Arg Met Asp Phe Ala Phe Pro Gly
   1370 1375 1380
   Ser Thr Asn Ser Leu His Arg Met Thr Thr Thr Ser Ala Ala Ala
   1385 1390 1395
   Tyr Gly Thr His Leu Ser Pro His Val Pro His Arg Val Leu Ser
   1400 1405 1410
   Thr Ser Ser Thr Leu Thr Arg Asp Tyr Asn Ser Leu Thr Arg Ser
   1415 1420 1425
   Glu His Ser His Ser Thr Thr Leu Pro Arg Asp Tyr Ser Thr Leu
   1430 1435 1440
   Thr Ser Val Ser Ser His Asp Ser Arg Leu Thr Ala Gly Val Pro
   1445 1450 1455
   Asp Thr Pro Thr Arg Leu Val Phe Ser Ala Leu Gly Pro Thr Ser
   1460 1465 1470
   Leu Arg Val Ser Trp Gln Glu Pro Arg Cys Glu Arg Pro Leu Gln
   1475 1480 1485
   Gly Tyr Ser Val Glu Tyr Gln Leu Leu Asn Gly Gly Glu Leu His
   1490 1495 1500
   Arg Leu Asn Ile Pro Asn Pro Ala Gln Thr Ser Val Val Val Glu
   1505 1510 1515
   Asp Leu Leu Pro Asn His Ser Tyr Val Phe Arg Val Arg Ala Gln
   1520 1525 1530
   Ser Gln Glu Gly Trp Gly Arg Glu Arg Glu Gly Val Ile Thr Ile
   1535 1540 1545
   Glu Ser Gln Val His Pro Gln Ser Pro Leu Cys Pro Leu Pro Gly
   1550 1555 1560
   Ser Ala Phe Thr Leu Ser Thr Pro Ser Ala Pro Gly Pro Leu Val
   1565 1570 1575
   Phe Thr Ala Leu Ser Pro Asp Ser Leu Gln Leu Ser Trp Glu Arg
   1580 1585 1590
   Pro Arg Arg Pro Asn Gly Asp Ile Val Gly Tyr Leu Val Thr Trp
   1595 1600 1605
   Pro Ala Thr Ala Phe Arg Val Asp Gly Asp Ser Pro Glu Ser Arg
   1610 1615 1620
   Leu Thr Val Pro Gly Leu Ser Glu Asn Val Pro Tyr Lys Phe Lys
   1625 1630 1635
   Val Gln Ala Arg Thr Thr Glu Gly Phe Gly Pro Glu Arg Glu Gly
   1640 1645 1650
   Ile Ile Thr Ile Glu Ser Gln Asp Gly Gly Pro Phe Pro Gln Leu
   1655 1660 1665
   Gly Ser Arg Ala Gly Leu Phe Gln His Pro Leu Gln Ser Glu Tyr
   1670 1675 1680
   Ser Ser Ile Thr Thr Thr His Thr Ser Ala Thr Glu Pro Phe Leu
   1685 1690 1695
   Val Asp Gly Pro Thr Leu Gly Ala Gln His Leu Glu Ala Gly Gly
   1700 1705 1710
   Ser Leu Thr Arg His Val Thr Gln Glu Phe Val Ser Arg Thr Leu
   1715 1720 1725
   Thr Thr Ser Gly Thr Leu Ser Thr His Met Asp Gln Gln Phe Phe
   1730 1735 1740
   Gln Thr
   1745
<210> 5
   <211> 964
   <212> PRT
   <213> Homo sapiens
<400> 5
   Met Ala Gly Pro Arg Pro Ser Pro Trp Ala Arg Leu Leu Leu Ala Ala
   1               5                   10                  15
   Leu Ile Ser Val Ser Leu Ser Gly Thr Leu Ala Asn Arg Cys Lys Lys
               20                  25                  30
   Ala Pro Val Lys Ser Cys Thr Glu Cys Val Arg Val Asp Lys Asp Cys
           35                  40                  45
   Ala Tyr Cys Thr Asp Glu Met Phe Arg Asp Arg Arg Cys Asn Thr Gln
       50                  55                  60
   Ala Glu Leu Leu Ala Ala Gly Cys Gln Arg Glu Ser Ile Val Val Met
   65                  70                  75                  80
   Glu Ser Ser Phe Gln Ile Thr Glu Glu Thr Gln Ile Asp Thr Thr Leu
                   85                  90                  95
   Arg Arg Ser Gln Met Ser Pro Gln Gly Leu Arg Val Arg Leu Arg Pro
               100                 105                 110
   Gly Glu Glu Arg His Phe Glu Leu Glu Val Phe Glu Pro Leu Glu Ser
           115                 120                 125
   Pro Val Asp Leu Tyr Ile Leu Met Asp Phe Ser Asn Ser Met Ser Asp
       130                 135                 140
   Asp Leu Asp Asn Leu Lys Lys Met Gly Gln Asn Leu Ala Arg Val Leu
   145                 150                 155                 160
   Ser Gln Leu Thr Ser Asp Tyr Thr Ile Gly Phe Gly Lys Phe Val Asp
                   165                 170                 175
   Lys Val Ser Val Pro Gln Thr Asp Met Arg Pro Glu Lys Leu Lys Glu
               180                 185                 190
   Pro Trp Pro Asn Ser Asp Pro Pro Phe Ser Phe Lys Asn Val Ile Ser
           195                 200                 205
   Leu Thr Glu Asp Val Asp Glu Phe Arg Asn Lys Leu Gln Gly Glu Arg
       210                 215                 220
   Ile Ser Gly Asn Leu Asp Ala Pro Glu Gly Gly Phe Asp Ala Ile Leu
   225 230 235 240
   Gln Thr Ala Val Cys Thr Arg Asp Ile Gly Trp Arg Pro Asp Ser Thr
   245 250 255
   His Leu Leu Val Phe Ser Thr Glu Ser Ala Phe His Tyr Glu Ala Asp
   260 265 270
   Gly Ala Asn Val Leu Ala Gly Ile Met Ser Arg Asn Asp Glu Arg Cys
   275 280 285
   His Leu Asp Thr Thr Gly Thr Tyr Thr Gln Tyr Arg Thr Gln Asp Tyr
   290 295 300
   Pro Ser Val Pro Thr Leu Val Arg Leu Leu Ala Lys His Asn Ile Ile
   305 310 315 320
   Pro Ile Phe Ala Val Thr Asn Tyr Ser Tyr Ser Tyr Tyr Glu Lys Leu
   325 330 335
   His Thr Tyr Phe Pro Val Ser Ser Leu Gly Val Leu Gln Glu Asp Ser
   340 345 350
   Ser Asn Ile Val Glu Leu Leu Glu Glu Ala Phe Asn Arg Ile Arg Ser
   355 360 365
   Asn Leu Asp Ile Arg Ala Leu Asp Ser Pro Arg Gly Leu Arg Thr Glu
   370 375 380
   Val Thr Ser Lys Met Phe Gln Lys Thr Arg Thr Gly Ser Phe His Ile
   385 390 395 400
   Arg Arg Gly Glu Val Gly Ile Tyr Gln Val Gln Leu Arg Ala Leu Glu
   405 410 415
   His Val Asp Gly Thr His Val Cys Gln Leu Pro Glu Asp Gln Lys Gly
   420 425 430
   Asn Ile His Leu Lys Pro Ser Phe Ser Asp Gly Leu Lys Met Asp Ala
   435 440 445
   Gly Ile Ile Cys Asp Val Cys Thr Cys Glu Leu Gln Lys Glu Val Arg
   450 455 460
   Ser Ala Arg Cys Ser Phe Asn Gly Asp Phe Val Cys Gly Gln Cys Val
   465 470 475 480
   Cys Ser Glu Gly Trp Ser Gly Gln Thr Cys Asn Cys Ser Thr Gly Ser
                   485                 490                 495
   Leu Ser Asp Ile Gln Pro Cys Leu Arg Glu Gly Glu Asp Lys Pro Cys
               500                 505                 510
   Ser Gly Arg Gly Glu Cys Gln Cys Gly His Cys Val Cys Tyr Gly Glu
           515                 520                 525
   Gly Arg Tyr Glu Gly Gln Phe Cys Glu Tyr Asp Asn Phe Gln Cys Pro
       530                 535                 540
   Arg Thr Ser Gly Phe Leu Cys Asn Asp Arg Gly Arg Cys Ser Met Gly
   545                 550                 555                 560
   Gln Cys Val Cys Glu Pro Gly Trp Thr Gly Pro Ser Cys Asp Cys Pro
                   565                 570                 575
   Leu Ser Asn Ala Thr Cys Ile Asp Ser Asn Gly Gly Ile Cys Asn Gly
               580                 585                 590
   Arg Gly His Cys Glu Cys Gly Arg Cys His Cys His Gln Gln Ser Leu
           595                 600                 605
   Tyr Thr Asp Thr Ile Cys Glu Ile Asn Tyr Ser Ala Ile His Pro Gly
       610                 615                 620
   Leu Cys Glu Asp Leu Arg Ser Cys Val Gln Cys Gln Ala Trp Gly Thr
   625                 630                 635                 640
   Gly Glu Lys Lys Gly Arg Thr Cys Glu Glu Cys Asn Phe Lys Val Lys
                   645                 650                 655
   Met Val Asp Glu Leu Lys Arg Ala Glu Glu Val Val Val Arg Cys Ser
               660                 665                 670
   Phe Arg Asp Glu Asp Asp Asp Cys Thr Tyr Ser Tyr Thr Met Glu Gly
           675                 680                 685
   Asp Gly Ala Pro Gly Pro Asn Ser Thr Val Leu Val His Lys Lys Lys
       690                 695                 700
   Asp Cys Pro Pro Gly Ser Phe Trp Trp Leu Ile Pro Leu Leu Leu Leu
   705                 710                 715                 720
   Leu Leu Pro Leu Leu Ala Leu Leu Leu Leu Leu Cys Trp Lys Tyr Cys
   725 730 735
   Ala Cys Cys Lys Ala Cys Leu Ala Leu Leu Pro Cys Cys Asn Arg Gly
   740 745 750
   His Met Val Gly Phe Lys Glu Asp His Tyr Met Leu Arg Glu Asn Leu
   755 760 765
   Met Ala Ser Asp His Leu Asp Thr Pro Met Leu Arg Ser Gly Asn Leu
   770 775 780
   Lys Gly Arg Asp Val Val Arg Trp Lys Val Thr Asn Asn Met Gln Arg
   785 790 795 800
   Pro Gly Phe Ala Thr His Ala Ala Ser Ile Asn Pro Thr Glu Leu Val
   805 810 815
   Pro Tyr Gly Leu Ser Leu Arg Leu Ala Arg Leu Cys Thr Glu Asn Leu
   820 825 830
   Leu Lys Pro Asp Thr Arg Glu Cys Ala Gln Leu Arg Gln Glu Val Glu
   835 840 845
   Glu Asn Val Arg Thr Gln Glu Leu Gly Leu Ala Gly Asp Val Ala Glu
   850 855 860
   Arg Gly Leu Gln Ala Asp Leu Arg Cys Thr Gln Ala Pro Ala Asp Gln
   865 870 875 880
   Val Pro Ala Ala Ala Gln Cys Arg Glu Lys Ala Arg Pro His His Cys
   885 890 895
   Gly His Ser Ala Asp Gly Ala Pro Leu Gly Gln Ala Gly Pro Ala Glu
   900 905 910
   Ala Tyr Arg Glu Ala Gly Gly Thr Glu Gly Leu Pro Arg Pro Gln Gly
   915 920 925
   Gly Pro Arg Leu Leu His Pro His Cys Arg Pro Gly Arg Pro Gly His
   930 935 940
   Gly Gly Val Pro Gly Gly Arg Gly Ala Gly Gly Arg Thr Gly Ala Pro
   945 950 955 960
   Leu Tyr Pro Ala
<210> 6
   <211> 11
   <212> PRT
   <213> Homo sapiens
<400> 6
   Ala Cys Leu Ala Leu Leu Pro Cys Cys Asn Arg
   1 5 10
<210> 7
   <211> 7
   <212> PRT
   <213> Homo sapiens
<400> 7
   Ala Glu Glu Val Val Val Arg
   1 5
<210> 8
   <211> 6
   <212> PRT
   <213> Homo sapiens
<400> 8
   Ala Phe His Asp Leu Lys
   1 5
<210> 9
   <211> 18
   <212> PRT
   <213> Homo sapiens
<400> 9
   Ala Leu Glu His Val Asp Gly Thr His Val Cys Gln Leu Pro Glu Asp
   1 5 10 15
   Gln Lys
<210> 10
   <211> 9
   <212> PRT
   <213> Homo sapiens
<400> 10
   Ala Gln Ser Gln Glu Gly Trp Gly Arg
   1 5
<210> 11
   <211> 22
   <212> PRT
   <213> Homo sapiens
<400> 11
   Cys Glu Arg Pro Leu Gln Gly Tyr Ser Val Glu Tyr Gln Leu Leu Asn
   1               5                   10                  15
   Gly Gly Glu Leu His Arg
               20
<210> 12
   <211> 7
   <212> PRT
   <213> Homo sapiens
<400> 12
   Asp Pro Asp Glu Leu Asp Arg
   1               5
<210> 13
   <211> 14
   <212> PRT
   <213> Homo sapiens
<400> 13
   Asp Val Val Ser Phe Glu Gln Pro Glu Phe Ser Val Ser Arg
   1               5                   10
<210> 14
   <211> 18
   <212> PRT
   <213> Homo sapiens
<400> 14
   Asp Tyr Ile Pro Val Glu Gly Glu Leu Leu Phe Gln Pro Gly Glu Ala
   1               5                   10                  15
   Trp Lys
<210> 15
   <211> 7
   <212> PRT
   <213> Homo sapiens
<400> 15
   Asp Tyr Asn Ser Leu Thr Arg
   1               5
<210> 16
   <211> 14
   <212> PRT
   <213> Homo sapiens
<400> 16
   Asp Tyr Ser Thr Leu Thr Ser Val Ser Ser His Asp Ser Arg
   1 5 10
<210> 17
   <211> 11
   <212> PRT
   <213> Homo sapiens
<400> 17
   Glu Ala Ile Ile Asn Leu Ala Thr Gln Pro Lys
   1 5 10
<210> 18
   <211> 6
   <212> PRT
   <213> Homo sapiens
<400> 18
   Glu Cys Ala Gln Leu Arg
   1 5
<210> 19
   <211> 7
   <212> PRT
   <213> Homo sapiens
<400> 19
   Glu Asp His Tyr Met Leu Arg
   1 5
<210> 20
   <211> 10
   <212> PRT
   <213> Homo sapiens
<400> 20
   Glu Gly Glu Asp Lys Pro Cys Ser Gly Arg
   1 5 10
<210> 21
   <211> 20
   <212> PRT
   <213> Homo sapiens
<400> 21
   Glu Gly Ile Ile Thr Ile Glu Ser Gln Asp Gly Gly Pro Phe Pro Gln
   1 5 10 15
   Leu Gly Ser Arg
   20
<210> 22
   <211> 15
   <212> PRT
   <213> Homo sapiens
<400> 22
   Glu Asn Leu Met Ala Ser Asp His Leu Asp Thr Pro Met Leu Arg
   1 5 10 15
<210> 23
   <211> 12
   <212> PRT
   <213> Homo sapiens
<400> 23
   Phe Glu Pro Leu Leu Gly Glu Glu Leu Asp Leu Arg
   1 5 10
<210> 24
   <211> 13
   <212> PRT
   <213> Homo sapiens
<400> 24
   Phe Glu Pro Leu Leu Gly Glu Glu Leu Asp Leu Arg Arg
   1 5 10
<210> 25
   <211> 9
   <212> PRT
   <213> Homo sapiens
<400> 25
   Phe His Val Gln Leu Ser Asn Pro Lys
   1 5
<210> 26
   <211> 11
   <212> PRT
   <213> Homo sapiens
<400> 26
   Gly Glu Val Gly Ile Tyr Gln Val Gln Leu Arg
   1 5 10
<210> 27
   <211> 15
   <212> PRT
   <213> Homo sapiens
<400> 27
   Gly Met Val Glu Phe Gln Glu Gly Val Glu Leu Val Asp Val Arg
   1 5 10 15
<210> 28
   <211> 14
   <212> PRT
   <213> Homo sapiens
<400> 28
   Gly Asn Ile His Leu Lys Pro Ser Phe Ser Asp Gly Leu Lys
   1 5 10
<210> 29
   <211> 19
   <212> PRT
   <213> Homo sapiens
<400> 29
   His Asn Ile Ile Pro Ile Phe Ala Val Thr Asn Tyr Ser Tyr Ser Tyr
   1 5 10 15
   Tyr Glu Lys
<210> 30
   <211> 9
   <212> PRT
   <213> Homo sapiens
<400> 30
   His Val Thr Gln Glu Phe Val Ser Arg
   1 5
<210> 31
   <211> 16
   <212> PRT
   <213> Homo sapiens
<400> 31
   Ile His Phe Asn Trp Leu Pro Pro Ser Gly Lys Pro Met Gly Tyr Arg
   1 5 10 15
<210> 32
   <211> 23
   <212> PRT
   <213> Homo sapiens
<400> 32
   Ile Ser Gly Asn Leu Asp Ala Pro Glu Gly Gly Phe Asp Ala Ile Leu
   1 5 10 15
   Gln Thr Ala Val Cys Thr Arg
   20
<210> 33
   <211> 17
   <212> PRT
   <213> Homo sapiens
<400> 33
   Lys Ile His Phe Asn Trp Leu Pro Pro Ser Gly Lys Pro Met Gly Tyr
   1 5 10 15
   Arg
<210> 34
   <211> 12
   <212> PRT
   <213> Homo sapiens
<400> 34
   Leu Leu Glu Leu Gln Glu Val Asp Ser Leu Leu Arg
   1 5 10
<210> 35
   <211> 25
   <212> PRT
   <213> Homo sapiens
<400> 35
   Leu Asn Ile Pro Asn Pro Ala Gln Thr Ser Val Val Val Glu Asp Leu
   1 5 10 15
   Leu Pro Asn His Ser Tyr Val Phe Arg
   20 25
<210> 36
   <211> 11
   <212> PRT
   <213> Homo sapiens
<400> 36
   Leu Thr Ala Gly Val Pro Asp Thr Pro Thr Arg
   1 5 10
<210> 37
   <211> 13
   <212> PRT
   <213> Homo sapiens
<400> 37
   Leu Thr Val Pro Gly Leu Ser Glu Asn Val Pro Tyr Lys
   1 5 10
<210> 38
   <211> 12
   <212> PRT
   <213> Homo sapiens
<400> 38
   Leu Val Phe Ser Ala Leu Gly Pro Thr Ser Leu Arg
   1               5                   10
<210> 39
   <211> 11
   <212> PRT
   <213> Homo sapiens
<400> 39
   Met Ala Gly Pro Arg Pro Ser Pro Trp Ala Arg
   1               5                   10
<210> 40
   <211> 16
   <212> PRT
   <213> Homo sapiens
<400> 40
   Met Asp Ala Gly Ile Ile Cys Asp Val Cys Thr Cys Glu Leu Gln Lys
   1               5                   10                  15
<210> 41
   <211> 14
   <212> PRT
   <213> Homo sapiens
<400> 41
   Met Asp Phe Ala Phe Pro Gly Ser Thr Asn Ser Leu His Arg
   1               5                   10
<210> 42
   <211> 7
   <212> PRT
   <213> Homo sapiens
<400> 42
   Met Gly Gln Asn Leu Ala Arg
   1               5
<210> 43
   <211> 8
   <212> PRT
   <213> Homo sapiens
<400> 43
   Met Leu Leu Ile Glu Asn Leu Arg
   1               5
<210> 44
   <211> 20
   <212> PRT
   <213> Homo sapiens
<400> 44
   Met Thr Thr Thr Ser Ala Ala Ala Tyr Gly Thr His Leu Ser Pro His
   1               5                   10                  15
   Val Pro His Arg
               20
<210> 45
   <211> 6
   <212> PRT
   <213> Homo sapiens
<400> 45
   Met Val Asp Glu Leu Lys
   1               5
<210> 46
   <211> 9
   <212> PRT
   <213> Homo sapiens
<400> 46
   Asn Gly Ala Gly Trp Gly Pro Glu Arg
   1               5
<210> 47
   <211> 13
   <212> PRT
   <213> Homo sapiens
<400> 47
   Asn Val Ile Ser Leu Thr Glu Asp Val Asp Glu Phe Arg
   1               5                   10
<210> 48
   <211> 14
   <212> PRT
   <213> Homo sapiens
<400> 48
   Pro Ser Val Ser Asp Asp Thr Glu His Leu Val Asn Gly Arg
   1               5                   10
<210> 49
   <211> 14
   <212> PRT
   <213> Homo sapiens
<400> 49
   Gln Asp His Thr Ile Val Asp Thr Val Leu Met Ala Pro Arg
   1 5 10
<210> 50
   <211> 12
   <212> PRT
   <213> Homo sapiens
<400> 50
   Gln Glu Val Glu Glu Asn Leu Asn Glu Val Tyr Arg
   1 5 10
<210> 51
   <211> 7
   <212> PRT
   <213> Homo sapiens
<400> 51
   Gln Ile Ser Gly Val His Lys
   1 5
<210> 52
   <211> 18
   <212> PRT
   <213> Homo sapiens
<400> 52
   Gln Leu Leu Val Glu Ala Ile Asp Val Pro Ala Gly Thr Ala Thr Leu
   1 5 10 15
   Gly Arg
<210> 53
   <211> 7
   <212> PRT
   <213> Homo sapiens
<400> 53
   Gln Gln Pro Asn Ala Gly Lys
   1 5
<210> 54
   <211> 8
   <212> PRT
   <213> Homo sapiens
<400> 54
   Arg Ala Glu Glu Val Val Val Arg
   1 5
<210> 55
   <211> 15
   <212> PRT
   <213> Homo sapiens
<400> 55
   Arg Cys Asn Thr Gln Ala Glu Leu Leu Ala Ala Gly Cys Gln Arg
   1 5 10 15
<210> 56
   <211> 10
   <212> PRT
   <213> Homo sapiens
<400> 56
   Arg Phe His Val Gln Leu Ser Asn Pro Lys
   1 5 10
<210> 57
   <211> 12
   <212> PRT
   <213> Homo sapiens
<400> 57
   Arg Gly Glu Val Gly Ile Tyr Gln Val Gln Leu Arg
   1 5 10
<210> 58
   <211> 26
   <212> PRT
   <213> Homo sapiens
<400> 58
   Arg Pro Asn Gly Asp Ile Val Gly Tyr Leu Val Thr Cys Glu Met Ala
   1 5 10 15
   Gln Gly Gly Gly Pro Ala Thr Ala Phe Arg
   20 25
<210> 59
   <211> 10
   <212> PRT
   <213> Homo sapiens
<400> 59
   Arg Ser Gln Met Ser Pro Gln Gly Leu Arg
   1 5 10
<210> 60
   <211> 5
   <212> PRT
   <213> Homo sapiens
<400> 60
   Arg Val Thr Trp Arg
   1 5
<210> 61
   <211> 15
   <212> PRT
   <213> Homo sapiens
<400> 61
   Ser Ala Thr Pro Gly Pro Pro Gly Glu His Leu Val Asn Gly Arg
   1 5 10 15
<210> 62
   <211> 13
   <212> PRT
   <213> Homo sapiens
<400> 62
   Ser Cys Val Gln Cys Gln Ala Trp Gly Thr Gly Glu Lys
   1 5 10
<210> 63
   <211> 11
   <212> PRT
   <213> Homo sapiens
<400> 63
   Ser Glu His Ser His Ser Thr Thr Leu Pro Arg
   1 5 10
<210> 64
   <211> 26
   <212> PRT
   <213> Homo sapiens
<400> 64
   Ser Phe Thr Ser Gln Met Leu Ser Ser Gln Pro Pro Pro His Gly Asp
   1 5 10 15
   Leu Gly Ala Pro Gln Asn Pro Asn Ala Lys
   20 25
<210> 65
   <211> 6
   <212> PRT
   <213> Homo sapiens
<400> 65
   Ser Asn Leu Asp Ile Arg
   1 5
<210> 66
   <211> 9
   <212> PRT
   <213> Homo sapiens
<400> 66
   Ser Gln Met Ser Pro Gln Gly Leu Arg
   1 5
<210> 67
   <211> 6
   <212> PRT
   <213> Homo sapiens
<400> 67
   Ser Gln Val Ser Tyr Arg
   1 5
<210> 68
   <211> 19
   <212> PRT
   <213> Homo sapiens
<400> 68
   Ser Ser Asp Ala Glu Ala Pro His Gly Pro Pro Asp Asp Gly Gly Ala
   1 5 10 15
   Gly Gly Lys
<210> 69
   <211> 7
   <212> PRT
   <213> Homo sapiens
<400> 69
   Thr Gly Ser Phe His Ile Arg
   1 5
<210> 70
   <211> 8
   <212> PRT
   <213> Homo sapiens
<400> 70
   Thr Gly Ser Phe His Ile Arg Arg
   1 5
<210> 71
   <211> 11
   <212> PRT
   <213> Homo sapiens
<400> 71
   Thr His Gln Glu Val Pro Ser Glu Pro Gly Arg
   1 5 10
<210> 72
   <211> 19
   <212> PRT
   <213> Homo sapiens
<400> 72
   Thr Leu Thr Thr Ser Gly Thr Leu Ser Thr His Met Asp Gln Gln Phe
   1 5 10 15
   Phe Gln Thr
<210> 73
   <211> 12
   <212> PRT
   <213> Homo sapiens
<400> 73
   Thr Gln Asp Tyr Pro Ser Val Pro Thr Leu Val Arg
   1 5 10
<210> 74
   <211> 9
   <212> PRT
   <213> Homo sapiens
<400> 74
   Thr Thr Glu Gly Phe Gly Pro Glu Arg
   1 5
<210> 75
   <211> 15
   <212> PRT
   <213> Homo sapiens
<400> 75
   Val Ala Pro Gly Tyr Tyr Thr Leu Thr Ala Asp Gln Asp Ala Arg
   1 5 10 15
<210> 76
   <211> 19
   <212> PRT
   <213> Homo sapiens
<400> 76
   Val Cys Ala Tyr Gly Ala Gln Gly Glu Gly Pro Tyr Ser Ser Leu Val
   1 5 10 15
   Ser Cys Arg
<210> 77
   <211> 9
   <212> PRT
   <213> Homo sapiens
<400> 77
   Val Asp Gly Asp Ser Pro Glu Ser Arg
   1 5
<210> 78
   <211> 10
   <212> PRT
   <213> Homo sapiens
<400> 78
   Val Leu Ser Thr Ser Ser Thr Leu Thr Arg
   1 5 10
<210> 79
   <211> 7
   <212> PRT
   <213> Homo sapiens
<400> 79
   Val Leu Val Asp Asn Pro Lys
   1 5
<210> 80
   <211> 13
   <212> PRT
   <213> Homo sapiens
<400> 80
   Val Pro Leu Phe Ile Arg Pro Glu Asp Asp Asp Glu Lys
   1 5 10
<210> 81
   <211> 18
   <212> PRT
   <213> Homo sapiens
<400> 81
   Val Pro Ser Val Glu Leu Thr Asn Leu Tyr Pro Tyr Cys Asp Tyr Glu
   1 5 10 15
   Met Lys
<210> 82
   <211> 12
   <212> PRT
   <213> Homo sapiens
<400> 82
   Val Ser Val Pro Gln Thr Asp Met Arg Pro Glu Lys
   1 5 10
<210> 83
   <211> 7
   <212> PRT
   <213> Homo sapiens
<400> 83
   Val Ser Trp Gln Glu Pro Arg
   1 5
<210> 84
   <211> 17
   <212> PRT
   <213> Homo sapiens
<400> 84
   Tyr Trp Ile Gln Gly Asp Ser Glu Ser Glu Ala His Leu Leu Asp Ser
   1 5 10 15
   Lys
<210> 85
   <211> 12
   <212> PRT
   <213> Homo sapiens
<400> 85
   Leu Cys Thr Glu Asn Leu Leu Lys Pro Asp Thr Arg
   1 5 10